# EUROPEAN PATENT SPECIFICATION

(11) **EP 2 035 573 B1**
(45) Date of publication and mention of the grant of the patent: **18.08.2010**
(21) Application number: 07733386.2
(22) Date of filing: 27.06.2007
(51) Int. Cl.: C12Q 1/26, C12Q 1/48, G01N 33/88, A61P 11/06, A61P 29/00, A61K 31/202

(54) **METHODS FOR IDENTIFYING MODULATORS OF EOXIN FORMATION**
VERFAHREN ZUR IDENTIFIZIERUNG VON MODULATOREN DER EOXIN-BILDUNG
MÉTHODES POUR L'IDENTIFICATION DE MODULATEURS DE FORMATION D'EOXIN

(30) Priority: 27.06.2006 SE 0601394
(43) Date of publication of application: 18.03.2009
(73) Proprietor: Biolipox AB, 171 65 Solna (SE)
(72) Inventor: CLAESSON, Hans-Erik, SE-113 61 Stockholm (SE); BJÖRKHOLM, Magnus, S-183 50 Täby (SE)
(74) Representative: Smith, Stephen Edward
(86) International application number: PCT/GB2007/002394
(87) International publication number: WO 2008/001079

(56) References cited:
- WO-A-00/58295
- WO-A-95/32280
- WO-A-96/30761
- WO-A-2004/080999
- US-A- 6 093 741
- SALA ANGELO ET AL: "14,15-dehydroleukotriene A4: A specific substrate for leukotriene C4 synthase" BIOCHEMICAL JOURNAL, vol. 328, no. 1, 15 November 1997 (1997-11-15), pages 225-229, XP001051022 ISSN: 0264-6021
- CHUNG, K.F.: EUROPEAN RESPIRATORY JOURNAL, vol. 8, no. 7, 1995, pages 1203-1213, XP001051489 ISSN: 0903-1936
- KROEGEL C. ET AL.: ALLERGOLOGIE, vol. 22, no. 3, March 1999 (1999-03), pages 161-170, XP001051499 ISSN: 0344-5062
- TAKANO T ET AL: JOURNAL OF CLINICAL INVESTIGATION, NEW YORK, NY, US, vol. 101, no. 4, 1998, pages 819-826, XP000923075 ISSN: 0021-9738

## Description

### Field of the Invention

The present invention relates to methods for identifying pharmaceutically useful compounds. The invention further relates to methods for identifying compounds that are useful in the treatment of inflammatory diseases and of inflammation generally. The invention also relates to a method of making an anti-inflammatory composition and methods of using pharmaceutically useful compounds.

### Background of the invention

There are many diseases/disorders that are inflammatory in their nature. One of the major problems associated with existing treatments of inflammatory conditions is a lack of efficacy and/or the prevalence of side effects (real or perceived).

Asthma is a chronic inflammatory disease affecting of 6% to 8% of the adult population of the industrialized world. In children, the incidence is even higher, being close to 10% in most countries. Asthma is the most common cause of hospitalization for children under the age of fifteen.

Treatment regimens for asthma are based on the severity of the condition. Mild cases are either untreated or are only treated with inhaled β-agonists. Patients with more severe asthma are typically treated with anti-inflammatory compounds on a regular basis.

There is a considerable under-treatment of asthma, which is due at least in part to perceived risks with existing maintenance therapy (mainly inhaled corticosteroids). These include risks of growth retardation in children and loss of bone mineral density, resulting in unnecessary morbidity and mortality. As an alternative to steroids, leukotriene receptor antagonists (LTRas) have been developed. These drugs may be given orally, but are considerably less efficacious than inhaled steroids and usually do not control airway inflammation satisfactorily.

This combination of factors has led to at least 50% of all asthma patients being inadequately treated.

A similar pattern of under-treatment exists in relation to allergic disorders, where drugs are available to treat a number of common conditions but are underused in view of apparent side effects. Rhinitis, conjunctivitis and dermatitis may have an allergic component, but may also arise in the absence of underlying allergy. Indeed, non-allergic conditions of this class are in many cases more difficult to treat.

Chronic obstructive pulmonary disease (COPD) is a common disease affecting 6% to 8% of the world population. The disease is potentially lethal, and the morbidity and mortality from the condition is considerable. At present, there is no known pharmacological treatment capable of changing the course of COPD.

Other inflammatory disorders which may be mentioned include:
(a) idiopathic pulmonary fibrosis (this is less common than COPD, but is a serious disorder with a very bad prognosis. No curative treatment exists);
(b) inflammatory bowel disease (a group of disorders with a high morbidity rate. Today only symptomatic treatment of such disorders is available); and
(c) rheumatoid arthritis and osteoarthritis (common disabling inflammatory disorders of the joints. There are currently no curative, and only moderately effective symptomatic, treatments available for the management of such conditions).

Inflammation is also a common cause of pain. Inflammatory pain may arise for numerous reasons, such as infection, surgery or other trauma. Moreover, several malignancies are known to have inflammatory components adding to the symptomatology of the patients.

Thus, a new and/or alternative anti-inflammatory treatment would be of benefit to all of the above-mentioned patient groups. In particular, there is a real and substantial unmet clinical need for an effective anti-inflammatory drug capable of treating inflammatory disorders, such as asthma, with no real or perceived side effects.

The mammalian lipoxygenases are a family of structurally related enzymes, which catalyse the oxygenation of arachidonic acid leading to the formation of various pro-inflammatory mediators. Three types of human lipoxygenases are known which catalyse the insertion of molecular oxygen into arachidonic acid at carbon positions 5, 12 and 15¹. The enzymes are hence named 5-, 12- and 15-lipoxygenase, respectively.

Arachidonic acid metabolites that are formed following the action of lipoxygenases are known to have pronounced pathophysiological activity including pro-inflammatory effects.

The key enzyme in leukotriene biosynthesis is 5-lipoxygenase which in a two step-reaction converts arachidonic acid to leukotriene (LT)A₄. This metabolite can be hydrolysed enzymatically to leukotriene B₄ (LTB₄) or conjugated with glutathione, by leukotriene C₄ (LTC₄) synthase, to form LTC₄, which in turn can be cleaved by sequential action of γ-glutamyl-transpeptidase and dipeptidase to yield leukotriene D₄ (LTD₄) and leukotriene E₄ (LTE₄), respectively. LTC₄ and its metabolites are pro-inflammatory agents and potent bronchoconstrictors².

Huge efforts have been devoted towards the development of drugs that inhibit the action of these metabolites as well as the biological processes that form them. Drugs that have been developed to this end include 5-lipoxygenase inhibitors and, as mentioned previously, leukotriene receptor antagonists (LTRas). Inhibitors of FLAP (Five Lipoxygenase Activating Protein) have also been investigated.

Another class of enzymes that metabolize arachidonic acid are the cyclooxygenases. Arachidonic acid metabolites that are produced by this process include prostaglandins, thromboxanes and prostacyclin, all of which possess physiological or pathophysiological activity. In particular, the prostaglandin PGE₂ is a strong pro-inflammatory mediator, which also induces fever and pain. Consequently, a number of drugs have been developed to inhibit the formation of PGE₂, including "NSAIDs" (non-steroidal antiinflammatory drugs) and "coxibs" (selective cyclooxygenase-2 inhibitors). These classes of compounds act predominantly by way of inhibition of one or several cyclooxygenases. In addition receptor antagonists are being investigated.

Thus, in general, agents that are capable of blocking the formation of arachidonic acid metabolites are likely to be of benefit in the treatment of inflammation.

Pro-inflammatory agents may also be useful, for example in immunosuppressed patients, for example patients undergoing chemotherapy, treatment to prevent graft rejection or patients with AIDS.

In addition to 5-lipoxygenase 12- and 15-lipoxygenase are also expressed in the human body.
12-lipoxygenase (12-LO) is expressed to high extent in human platelets but very little is known about the biological role of this enzyme ¹.

15-lipoxygenase (15-LO) type 1 (human 15-LO:EC 1.13.11.33) is a 75-kDa protein containing a non-heme iron. 15-LO introduces molecular oxygen at C-15 of arachidonic acid (20:4) or at C-13 of linoleic acid (18:2) to form 15S-hydroperoxy-5,8,11-cis-13-trans-eicosatetraenoic acid (15-HPETE) or 13S-hydroperoxy-9-cis-13-trans-octadecadienoic acid (13-HPODE), respectively. A small amount of 12S-hydroperoxy-5,8,14-cis-10-trans-eicosatetraenoic acid (12-HPETE) is also formed from arachidonic acid by the 15-LO enzyme. Briefly, the catalytic reaction involved hydrogen abstraction at C-3 of the cis-cis-1,4-pentadiene structure of the unsaturated fatty acid (20:4 or 18:2), leading to a carbon radical and reduction of the iron from Fe³⁺ to Fe²⁺ (see scheme 1 below).

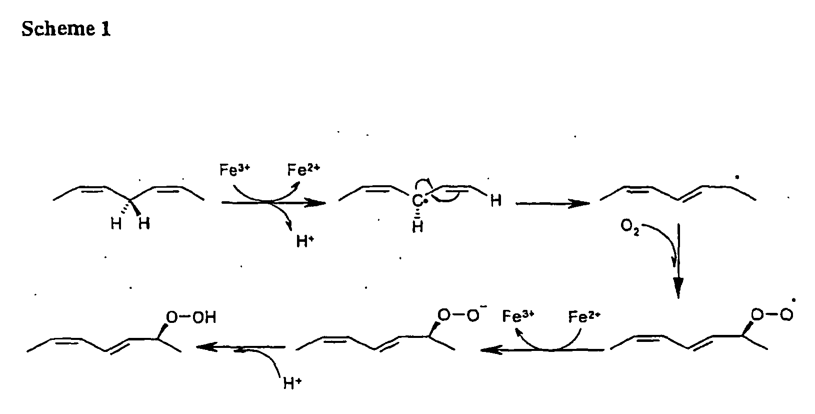

The radical migrates to C-5 where it reacts with molecular oxygen to form a peroxy radical. Oxidation of the iron to Fe³⁺ by the peroxy radical yields a peroxy-anion that is protonated to give the final lipidhydroperoxide.

The rabbit enzyme 15-LO has been cloned and crystallised and the substrate-binding site was found to be a deep hydrophobic pocket²², which is defined at its base by the side chains of Phe353, Met419, Ile418 and Ile593. The fatty acid substrate is tethered and positioned by Arg403 (on the surface of the enzyme). Mutagenic studies have demonstrated that variation of the size of the hydrophobic pocket affects the positional specificity of the enzyme. Thus, enlargement of the pocket of 15-LO increased the amount of 12-lipoxygenation products (12-HPETE). The iron in 15-LO is liganded to histidines and to the carboxy group of a C-terminal isoleucine.

In addition to undergoing reduction to 15(S)-HETE, 15 (S)-HPETE may also undergo dehydration to form 14,15-oxido-5,8,10,12 (Z,Z,E,E)-eicosatetraenoic acid (14,15-LTA₄). This allylic epoxide can be hydrolysed to the dihydroxy acids 8 (R,S), 15(S)-LTB4 or 14 (R,S), 15(S)-LTB₄¹¹⁻¹². In addition, with 15-HPETE as a substrate, 15-LO can also catalyse the formation of the double dioxygenation products 5(S), 15 (S)-DHETE, 8 (S,R), 15 (S)-DHETE and 14 (R,S), 15 (S)-DHETE. 15 (S)-lipoxygenase (15-LO) does not only metabolise free fatty acids but can also attack polyenoic fatty acids located in the membrane. The enzyme has been predicted to play an important role in reticulocyte maturation through breakdown of mitochondria membranes³. However, the enzyme may play a more general role in the differentiation of cells, such as maturation of keratinocytes and the eye lens⁴. 15-LO is predominantly expressed in reticulocytes, eosinophils, skin, monocytes/macrophages and airway epithelium⁵. Interleukin-4 (IL-4) and interleukin-13 (IL-13) induce the expression of 15-LO in cultivated monocytes and epithelial cells⁶, but also damage to certain cells has been reported to increase the activity of 15-LO (Green FA. Transformation of 5-HETE by activated keratinocyte 15-lipoxygenase and the activation mechanism. Lipids 25, 618-623 (1990)).

In contrast to 5-lipoxygenase and 12-lipoxygenase, 15-LO appears to be expressed only in rabbit and human cells¹. The murine ortholog of the human 15-LO appears to be leukocyte-type 12-LO¹. However, the regulation of the two enzymes activities seems to be quite different⁷.

15-LO was discovered more than two decades ago and the first 15-LO to be purified to homogeneity was derived from rabbit reticulocytes⁸. The human form of the enzyme has been cloned from reticulocytes and epithelial cells, and immunological studies indicate that the enzyme is identical in reticulocytes, eosinophils and airway epithelium⁹. A second form of 15-LO (type 2) has been cloned from a cDNA library from human hair roots and subsequently expression of the enzyme and amino acid analysis demonstrated that this 15-LO type 2 enzyme was approximately 40% identical to the earlier described 15-LO type 1¹⁰.

15-LO is thought to be involved in the formation of lipoxins, which mainly seem to have anti-inflammatory effects ¹³ , but a 15-LO derived product, 5-oxo-15-hydroxy-6,8,1,13-eicosatetraenoic acid, has been reported to be a potent chemotactic agent for human eosinophils ¹⁴. The physiological and pathophysiological roles of the 15-LO pathway in humans have not been fully characterised.

Based on arachidonic acid metabolites which are known to occur in biological systems for example leukotrienes, a number of different analogues have been synthesised. Synthetic analogues of LTC₄, such as 14.15-LTC₄ did not induce bronchoconstriction. Drazen, J.M, Lewis, R A, Austin, K.P., Toda, M, Brion, F., Marfat, A. and Corey, EJ.: Contractile activities of structural analogs of leukotrienes C and D: necessity of a hydrophobic region. Proc. Natl. Acad. Sci. 78,3195-3198 (1981).

Sala et al (1997) Biochem. J. 328: 225-229 describes a study into the metabolism of 14,15-dehydro-leukotriene A₄ (14,15-dehydro-LTA₄) by human platelet leukotriene C₄ (LTC₄) synthase and polymorphonuclear leukocyte leukotriene A₄ (LTA₄) hydrolase.

US 6,093,741 discusses methods of treating herpesviridae infection with an exogenous eicosanoid other than a prostaglandin that deficits leukotriene B₄ (LTB₄)-like activity.

WO 95/32280 describes an isolated nucleotide sequence encoding human leukotriene C₄ synthase.

WO 00/58295 describes compounds that have both leukotriene inhibition properties as well as antihistaminergic properties comprising 1,4 substituted piperazines, and 1-substituted,4-alkylidenyl piperadines.

### Summary of the Invention

We have identified the generation in biological systems of arachidonic acid metabolites with pro-inflammatory effects. The metabolites can be formed *via* the action of 15-lipoxygenase and via the action of soluble glutathione S transferases and/or leukotriene C₄ synthase. The metabolites are termed Eoxin C₄, Eoxin D₄ and Eoxin B₄. We provide screens for identifying anti-inflammatcry compounds and compounds that inhibit Eoxin C₄, D₄ and E₄ formation.

We show that the Hodgkin cell line L1236¹⁵ possesses 15-lipoxygease (15-LO) type 1 activity and converts arachidonic acid to Eoxins via the 15-LO pathway. Having revealed the presence and formation of Eoxin C₄, D₄ and E₄ in L1236, we also show that these Eoxins are formed in other human cells and tissues, in particular eosinophils, but also by nasal polyp tissue and dendritic cells (Example 2). We also show that a broad variety of cell types express 15-LO. Interestingly, cells in patients suffering from an inflammatory disease or a disease involving inflammatory processes, such as Reed-Sternberg cells in lymph-nodes from patients with Hodgkin's lymphoma, epitheloid cells in lung biopsies from sarcoidosis patients, synoviocytes from rheumatoid arthritis and neurones from patients suffering from Alzheimer's Disease (Example 3) display an increased expression of 15-LO, compared to control cells.

In addition to demonstrating the biosynthesis of these new metabolites, and identifying enzymes involved, biological effects of Eoxins are also described. For example, in Example 6a, it is indicated that Eoxins C₄, D₄ and E₄ are about 100 times more potent that histamine in causing permeabilisation of endothelial cells, (an in vitro model of inflammation), showing comparable potency to the sister metabolite, LTC₄, formed through the 5-LO pathway. For example, Eoxins C₄, D₄ and E₄ induce significant plasma leakage at 10⁻⁷ and 10⁻⁸ M in a model system with confluent bovine or human endothelial cells. In another model of inflammation, EoxC₄ and EoxD₄ induced mainly oxygen free radical production by GM-CSF primed neutrophils, while EoxE₄ induced mainly oxygen free radical production by IL-5 primed eosinophils. We also demonstrate compounds that block the formation of Eoxins C₄, D₄ and E₄.

We identify three 15-lipoxygenase derived metabolites from arachidonic acid, termed Eoxins: 14,15-LTC₄, 14,15-LTD₄, and 14,15-LTE₄, named Eoxin C₄ (EoxC₄), Eoxin D₄ (EoxD₄) and Eoxin E₄ (EoxE₄), respectively.

Formation of the 15-LO derived cysteinyl-containing products Eoxin C₄, D₄ and E₄ from arachidonic acid have never before been described in eukaryotic or prokaryotic cells.

The metabolites were isolated using RP-HPLC and the structures of the metabolites were determined by negative-ion electrospray tandem mass spectrometry. The Eoxins have a conjugated triene structure demonstrating an UV absorbance maximum at 282 nm (Example 1). We consider that these metabolites are formed by 15-LO catalysed conversion of arachidonic acid to 14,15-oxido-5,8,10,12 (Z,Z,E,E)-eicosatetraenoic acid (14,15-LTA₄; Eoxin A₄), which is conjugated with glutathione to form 14 (R)-glutathionyl-15 (S)-hydroxy-5,8,10,12 (Z,Z,E,E)-eicosatetraenoic acid (14,15-LTC₄; Eoxin C₄), which is subsequently metabolised to 14 (R)-cysteinyl-glycyl-15 (S)-hydroxy-5,8,10,12 (Z,Z,E,E)-eicosatetraenoic acid (14,15-LTD₄; Eoxin D₄) and 14 (R)-cysteinyl-15 (S)-hydroxy-5,8,10,12 (Z,Z,E,E)-eicosatetrasnoic acid (14,15-LTE₄; Eoxin E₄).

The enzymes involved in the formation of 14,15-LTC₄ (Eoxin C₄) from 14,15-LTA₄ (Eoxin A₄) are considered to be LTC₄-synthase and/or the soluble glutathione S-transferases (GST), in particular OSIM1, and GSTP1, as discussed in Example 4.

The suggested pathway is shown below:

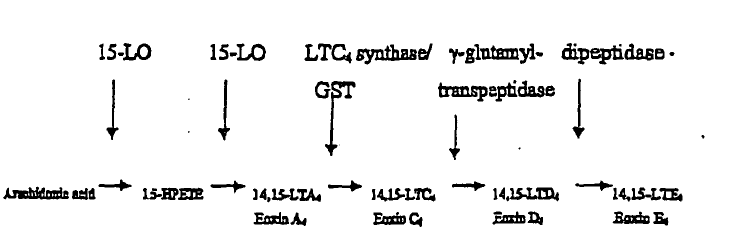

### Detailed description of the invention

A first aspect of the invention provides a method for identifying a compound for modulating the formation of Eoxin C₄, Eoxin D₄ or Eoxin E₄ in a biological system other than a human being or human embryonic cells, the method comprising testing the compound for an affect on 15-LO activity or GSTM1-1, GSTP1-1 and/or leukotriene C₄ synthase activity.

A second aspect of the invention provides a method for identifying a compound with an inflammation-modulating effect or bone loss-modulating (typically bone-loss-reducing) effect, the method comprising testing the compound for an effect on formation of Eoxin C₄, Eoxin D₄ or Eoxin E₄ in a biological system other than a human being or human embryonic cells, and/or testing the compound for an effect on activity of EoxC₄. EoxD₄ or EoxE₄ in a biological system other than a human being or human embryonic cells wherein the activity of the compound is tested by testing for an inhibitory effect on Eoxin-induced permeabilisation of endothelial cells In an *in vitro* model of inflammation: or for an inhibitory effect on Eoxin-Induced oxygen free radical production by leukocytes.

By "modulating the formation" is included inhibiting or promoting the formation and/or activity of Eoxin C₄, D₄ or E₄. Typically, the method may be used for identifying a compound for inhibiting the formation and/or activity of EoxC₄, EoxD₄ or EoxE₄ Such a compound is considered to be an anti-inflammatory compound. Such a compound is also considered m be a bone-loss-reducing compound. The compound may typically inhibit the formation and/or activity of EoxC₄, EoxD₄ and EoxE₄.

The method may alternatively be used for identifying a compound for promoting the formation and/or activity of EoxC₄, EoxD₄ or EoxE₄. Such a compound is considered to be pro-inflammatory. Such a compound may typically promote the formation of EoxC₄, EoxD₄ and EoxE₄.

The method of the first aspect of the invention may further comprise the step of testing the compound for effect on formation of EoxC₄, EoxD₄ or EoxE₄ in a biological system other than a human being or human embryonic cells, or testing the compound for an effect on activity of EoxC₄, EoxD₄ or EoxE₄ in a biological system wherein the activity of the compound is tested by testing for an inhibitory effect on Eoxin-induced permeabilisation of endothelial cells in an *in vitro* model of inflammation; or for an inhibitory effect on Eoxin-induced oxygen free radical production by leukocytes.

The biological system typically comprises 15-LO, glutathione S transferase (typically a soluble glutathione S transferase, for example GSTM1-1 or GSTP1-1) and/or leukotriene C4 synthase activity.

The compound may be tested in a system other than a human being or human embryonic cells comprising a cell fraction or isolated enzyme or enzymes, as discussed further below. Alternatively, the method of the first or second aspect of the invention may be performed by testing the effect of the test compound on a whole cell other than human embryonic cells. For example, the method may be performed using ex vivo human cells or cell lines other than human embryonic cells. For example, human eosinophils or mast cells (for example cord blood derived mast cells, for Example as described in Example 8) or L1236 cell line cells may be used, but human nasal polyp tissue and/or dendritic, cells or any other cell or cell-line expressing the necessary enzymes (either endogenous or recombinant); for example 15-LO and/or GST could also be used. However, it is not necessary to have co-location of the enzymes involved in this type of metabolic pathway. A well-known phenomenon in the formation of various arachidonic acid metabolites is transcellular biosynthesis, i.e. the first enzymatic step is catalysed in one cell type and the intermediate is exported to another cell type in which the second enzymatic step is performed. For example, biosynthesis of Eoxin A₄ may occur in one cell type, whilst conversion of Eoxin A₄ to Eoxin C₄ (and optionally also conversion of Eoxin C₄ to Eoxin D₄ and Eoxin E₄) may occur in a second cell type. Thus, the test could also be carried out in co-incubations of two cell types with complementing enzymatic capacities for the biosynthesis of Eoxins.

As will be apparent to the skilled person, it may be necessary to stimulate the cell before or as part of the assay procedure; or provide arachidonic acid in the assay, for example as described in the Examples. Eosinophils can be stimulated to produce EoxC₄ by LTC₄, Prostaglandin D₂ or IL-5, as discussed in Example 8. It may be desirable to prime mast cells, for example using IL-4, in order to induce the expression of 15-LO-1; and then provide arachidonic acid in the assay, as discussed in Example 8. Other cells may not require stimulation in order to be able to release Eoxins. For example, human nasal polyp cells are considered to be able spontaneously to release Eoxins, as discussed in Example 8.

As will be apparent to the skilled person, if effects on the formation of Eoxin C₄ are being tested for, then it may not be necessary to have present enzymes involved in the biosynthesis of metabolites of Eoxin C₄, (for example γ-glutamyl-transpeptidase or dipeptidase). Similarly, if effects on 15-LO activity are being tested for, then it may not be necessary to have present enzymes involved in the biosynthesis of metabolites of the 15-LO product (Eoxin A₄), for example LTC₄ synthase or a soluble glutathione S transferase, for example GSTM1-1 or GSTP1-1. As will be apparent to those skilled in the art, it may be desirable to test compounds both in systems in which one or a limited number of enzymes are present; and in systems in which more enzymes (for example all of the enzymes involved in Eoxin synthesis from arachidonic acid, for example 15-LO; LTC₄ synthase or a soluble GST such as GSTM1-1 or GSTP1-1; γ-glutamyl-transpeptidase and dipeptidase) are present. Tests in single or limited enzyme systems may be useful in establishing structure-function relationships, as well known to those skilled in the art; whilst tests in more complex systems may be useful in establishing whether a compound is likely to be useful *in vivo.*

Suitable ways to test if a compound is capable of inhibiting the formation of Eoxin C₄, Eoxin D₄ or Eoxin E₄, as described above, are illustrated in Example 7. However, other cell lines other than human embryonic cells may alternatively be used, if they have the enzymatic machinery to generate the Eoxin, the modification to the procedure depending on the cell type being evident to the person skilled in the art.

The eoxins can be detected and quantified by e.g. HPLC, gas chromatography, mass spectrometry, UV spectroscopy and different immunological methods such as EIA and RIA.

Presently, two forms of the 15-LO enzyme have been cloned, as noted above. Type 2 15-LO is approximately 40% identical to the Type 1 15-LO. In contrast to 15-LO type 1, linoleic acid is a poor substrate for 15-LO type 2. With present knowledge, the preferred enzyme for inhibition is thus 15-LO type 1, but 15-LO type 2 cannot be excluded as an enzyme in the metabolic pathway leading to the formation of Eoxins C₄, D₄ and E₄.

The catalytic activity of 15-LO can e.g. be measured as described⁹. The enzyme can be assayed by assessing conversion of arachidonic acid (substrate) into 15-(s)-HETE or linoleic acid (substrate) to 13-hydroxyoctadecadienoic acid (13-HODE), in the presence or absence of the test compound in question. A decrease in the formation of 15-(S)-HETE or 13-HODE when the compound is present illustrates a decrease in enzymatic activity. Such a compound will block the first enzymatic step in the formation of Eoxins C₄, D₄ and E₄. Such a compound is considered to have anti-inflammatory actions *in vivo.*

In one aspect of the first aspect of the invention, an assay for testing the catalytic activity of human 15-LO is envisioned as described in Example 5. The assay illustrated herein takes advantage of the ability of lipidhydroperoxides to oxidize diphenyl-1-pyrenylphosphone (DPPP) to a phosphine oxide. Diphenyl-1-pyrenylphosphine is a non-fluorescent compound until oxidized. Oxidation of DPPP yields a fluorescent compound, which is not sensitive to temperature and is stable for at least 60 minutes.

In another aspect of the first aspect of the invention, the assay is performed in a cell lysate to determine the conversion of arachidonic acid to 15-(S)-HETE or linoleic acid (substrate) to 13-hydroxyoctadecadienoic acid (13-HODE), in the presence or absence of the test compound in question.

Another of the key enzymes in the formation of said novel 15-lipoxygenase catalysed arachidonic acid metabolite is soluble GST. As illustrated in Example 4, GSTM1-1, GSTP1-1 and/or LTC₄ synthase can participate in the formation of Eoxins C₄, D₄ and E₄. Thus, in an embodiment of the first aspect of the invention, the effect of a test compound on the catalytic activity of GSTM1-1, GSTP1-1 and/or LTC₄ synthase is assessed. An assay for the catalytic activity of GSTM1-1 or GSTP1-1 can be performed essentially as described in Example 4.

In an embodiment of the second aspect of the invention, the test compound is assessed for its ability to modulate Eoxin C₄, D₄ or E₄ induced permeabilisation of endothelial cells in an in vitro model of inflammation, such as the one described in Example 6a. Such a test may also be performed in conjunction with a method of the first aspect of the invention.

The compound may be tested in a non-human animal or ex vivo model of inflammation in which 15-LO is present, for example in a rabbit model of inflammation, or in a transgenic non-human animal (for example a mouse or rat) which expresses 15-LO. The compound may be tested in a human ex vivo model of inflammation other than human embryonic cells, for example on human peripheral blood or human umbilical cord blood, or on cells isolated from human peripheral blood or human umbilical cord blood, for example on leukocytes, for example neutrophils, eosinophils, dendritic cells or mast cells. Examples of such tests are described in the Examples, for example Example 6b. The compound may also be tested for ability to differentiate stem cells other than human embryonic cells or other bone-forming cell precursors into bone cells, for example as described in US 2003/0175680.

The compounds to be tested may be selected on the basis of structure-based screening methods, for example in which compound structures are compared with or designed or modified on the basis of the substrate binding site of 15-LO, as discussed above. A starting compound may initially be selected by screening for an effect on 15-LO enzyme activity (for example using an assay as described in Example 5); then compared with the structure; used as the basis for designing further compounds which may then be tested by further modelling and/or synthesis and assessment, for example for an effect on Eoxin C₄, D₄ or E₄ formation.

Assessment of effects on 15-LO activity using DPPP (for example as described in Example 5) means that a spectrophotometric assay can be used, is straightforward to perform and can be used in, for example, a microtitre plate format which is very suitable for high throughput testing. Assessment using arachidonic acid or 15-HPETE or other arachidonic acid metabolite as the substrate, may be useful in confirming the ability of a compound to modulate 15-LO activity *in vivo.*

Compounds may also be subjected to other tests, for example toxicology or metabolism tests, as is well known to those skilled in the art.

It will be appreciated that the invention provides screening assays for use in trying to identify drugs which may be useful in modulating, for example either enhancing or inhibiting, the formation or activity of one or more of Eox C₄, Eox D₄ and Eox E₄. Compounds identified in the methods may themselves be useful as a drug or they may represent lead compounds for the design and synthesis of more efficacious compounds.

The compound may be a drug-like compound or lead compound for the development of a drug-like compound for each of the above methods of identifying a compound. It will be appreciated that the said methods may be useful as screening assays in the development of pharmaceutical compounds or drugs, as well known to those skilled in the art.

The term "drug-like compound" is well known to those skilled in the art, and may include the meaning of a compound that has characteristics that may make it suitable for use in medicine, for example as the active ingredient in a medicament. Thus, for example, a drug-like compound may be a molecule that may be synthesised by the techniques of organic chemistry, less preferably by techniques of molecular biology or biochemistry, and is preferably a small molecule, which may be of less than 5000 daltons. A drug-like compound may additionally exhibit features of selective interaction with a particular protein or proteins and be bioavailable and/or able to penetrate cellular membranes, but it will be appreciated that these features are not essential.

The term "lead compound" is similarly well known to those skilled in the art, and may include the meaning that the compound, whilst not itself suitable for use as a drug (for example because it is only weakly potent against its intended target, non-selective in its action, unstable, difficult to synthesise or has poor bioavailability) may provide a starting-point for the design of other compounds that may have more desirable characteristics.

It is appreciated that screening assays which are capable of high throughput operation are particularly preferred. Enzyme assays using DPPP may be used, as discussed above.

It will be understood that it will be desirable to identify compounds that may modulate, as appropriate, inflammation or the formation or activity of Eoxins C₄, D₄ or E₄ in vivo. Thus it will be understood that reagents and conditions used in the methods of the invention may be chosen such that the interactions between, for example, the 15-LO and the substrate, are similar to those between the human 15-LO and a naturally occurring substrate (for example arachidonic acid). As well known to those skilled in the art, different assay systems may be used to assess a compound, in some of which the convenience of the assay or the specificity for an effect on a particular enzyme involved in Eoxin formation may be optimised (for example using a single enzyme system), whilst in others the *in vivo* relevance may be optimised, for example by assessing the effect of the compound in a whole cell.

The screening assays of the invention are useful in the search for compounds which may be useful in the treatment of inflammatory disease or in a disease involving inflammatory processes. A compound selected on the basis of in vitro and/or in vivo screens may be further tested for an anti-inflammatory effect in an inflammatory disease or in a disease involving inflammatory processes. Similarly, the screening assays of the invention are useful in the search for compounds which may be useful in treating or preventing bone loss/enhancing bone formation. A compound selected on the basis of in vitro and/or in vivo screens may be further tested for a bone-loss-reducing/bone-formation-enhancing effect in a patient with or at risk of bone loss or in a disease involving inflammatory processes.

The term "inflammation" will be understood by those skilled in the art to include any condition characterised by a localised or a systemic protective response, which may be elicited by physical trauma, infection, chronic diseases, such as those mentioned hereinbefore, and/or chemical and/or physiological reactions to external stimuli (e.g. as part of an allergic response). Any such response, which may serve to destroy, dilute or sequester both the injurious agent and the injured tissue, may be manifest by, for example, heat, swelling, pain, redness, dilation of blood vessels and/or increased blood flow, invasion of the affected area by white blood cells, loss of function and/or any other symptoms known to be associated with inflammatory conditions.

The term "inflammation" will thus also be understood to include any inflammatory disease, disorder or condition *per se,* any condition that has an inflammatory component associated with it, and/or any condition characterised by inflammation as a symptom, including *inter alia* acute, chronic, ulcerative, specific, allergic and necrotic inflammation, and other forms of inflammation known to those skilled in the art. The term thus also includes, for the purposes of this invention, inflammatory pain and/or fever.

It is considered that a compound that decreases formation or activity of Eoxins C₄, D₄ or E₄ will be useful in treating an inflammatory disease or a disease involving inflammatory processes. Accordingly, the screening methods of the invention are useful in identifying compounds which may be useful in the treatment of asthma, chronic obstructive pulmonary disease (COPD), pulmonary fibrosis, allergic disorders, rhinitis, inflammatory bowel disease, ulcers, inflammatory pain, fever, atherosclerosis, coronary artery disease, vasculitis, pancreatitis, arthritis, osteoarthritis, rheumatoid arthritis, conjunctivitis, iritis, scleritis, uveitis, wound healing, dermatitis, eczema, psoriasis, stroke, diabetes, autoimmune diseases, Alzheimer's disease, multiple sclerosis, sarcoidosis, atherosclerosis, hypertension (see, for example Anning PB et al (March 2005) Elevated endothelial nitric oxide bioactivity and resistance to angiotensin-dependent hypertension in 12/15-lipoxygenase knockout mice. Am J Pathol. 166(3):653-62), Hodgkin's lymphoma and other malignancies, or any other disease with an inflammatory component.

The screening methods of the invention are also considered to be useful in identifying compounds which may be useful in the treatment or prevention of bone loss, for example associated with osteoporosis, osteoarthritis, Paget's disease or periodontal diseases, as discussed in US 2003/0175680. The screening methods of the invention may thus also be useful in identifying compounds which may be useful in increasing bone mineral density, as well as the reduction in incidence and/or healing of fractures, in subjects.

The compounds may be useful both in the therapeutic and/or prophylactic treatment of the above-mentioned conditions.

It is considered that a compound that increases formation or activity of Eoxins C₄, D₄ or E₄ will be useful in treating a patient in which promotion of an inflammatory response may be beneficial, for example in an immunosuppressed patient, for example a patient undergoing chemotherapy or treatment to reduce rejection of a transplant. Eox C₄, Eox D₄ or Eox E₄, for example, are considered to be useful in treating such a patient in which promotion of an inflammatory response may be beneficial.

A further aspect of the invention provides Eox C₄, Eox D₄ or Eox E₄ for use in treating a patient in need of promotion of an inflammatory response.

As a result of performing a screening method of the invention a compound is identified, which may typically be a peptide or a small molecule, for example as shown by Example 7. The compound can be formulated for pharmaceutical use, for example for use in *in vivo* trials in patients, for example animals (typically mammals, far example experimental mammals such as mice, rats, rabbits, dogs or monkeys, preferably rabbits or other animals (eg transgenic animals) which express 15-LO) or humans.

We provide a method of making an anti-inflammatory composition or bone-loss-reducing composition comprising
(i) identifying respectively an anti-inflammatory or bone-loss-reducing compound by a method of the second aspect of the invention;
(ii) combining the compound with a pharmaceutically acceptable excipient or carrier.
   We provide a method of making an Eoxin formation-modulating composition comprising
   (i) identifying an Eoxin formation-modulating compound by a method of the first aspect of the invention;
   (ii) combining the compound with a pharmaceutically acceptable excipient or carrier.

The composition is typically intended to supply an effective amount of the compound to a patient. The term "effective amount" refers to an amount of a compound, which confers a therapeutic effect on the treated patient. The effect may be objective (i.e. measurable by some test or marker) or subjective (i.e. the subject gives an indication of or feels an effect).

The composition may be intended for or suitable for administering orally, intravenously, subcutaneously, buccally, rectally, dermally, nasally, tracheally, bronchially, sublingually, by any other parenteral route or *via* inhalation, in a pharmaceutically acceptable dosage form. The composition may be, for example, a tablet, capsule or elixir for oral administration, suppository for rectal administration, sterile solution or suspension for parenteral or intramuscular administration, and the like. It may be a spray for inhalation or a liquid for injection. Such compositions may be prepared in accordance with standard and/or accepted pharmaceutical practice.

The composition may also contain another therapeutic agent that is useful in the treatment of inflammation as defined herein (e.g. selected from NSAIDs, coxibs, corticosteroids, analgesics, inhibitors of 5-lipoxygenase, inhibitors of FLAP (5-lipoxygenase activating protein), and leukotriene receptor antagonists (LTRas), and/or other therapeutic agents that are useful in the treatment of inflammation).

Alternatively, the composition may also contain another therapeutic agent that is useful in reducing bone loss or increasing bone formation, for example a biosphosphonate, estrogen, selective estrogen receptor modulator (SERM), calcinonin or an anabolic agent. Examples of such therapeutic agents are discussed in, for example, US 2003/0175680, for example in paragraph [0065].

Such combination products provide for the administration of the identified compound in conjunction with the other therapeutic agent, and may thus be presented either as separate formulations, wherein at least one of those formulations comprises the identified, and at least one comprises the other therapeutic agent, or may be presented (i.e. formulated) as a combined preparation (i.e. presented as a single formulation including the identified compound and the other therapeutic agent).

Compounds may be administered at varying doses. Oral, pulmonary and topical dosages may range from between about 0.01 mg/kg of body weight per day (mg/kg/day) to about 100 mg/kg/day, preferably about 0.01 to about 10 mg/kg/day, and more preferably about 0.1 to about 5.0 mg/kg/day. For e.g. oral administration, the compositions typically contain between about 0.01 mg to about 500 mg, and preferably between about 1 mg to about 100 mg, of the active ingredient. Intravenously, the most preferred doses will range from about 0.001 to about 10 mg/kg/hour during constant rate infusion. Advantageously, compounds may be administered in a single daily dose, or the total daily dosage may be administered in divided doses of two, three or four times daily.

In any event, the physician, or the skilled person, will be able to determine the actual dosage which will be most suitable for an individual patient, which is likely to vary with the route of administration, the type and severity of the condition that is to be treated, as well as the species, age, weight, sex, renal function, hepatic function and response of the particular patient to be treated. The above-mentioned dosages are exemplary of the average case; there can, of course, be individual instances where higher or lower dosage ranges are merited . Example 7, for example, shows a method by which compounds that are capable of inhibiting the formation of Eoxins are identified.

The invention will now be described further by reference to the following, nonlimiting, Figures and Examples.
Figure 1: RP-HPLC chromatogram of products with UV absorbance at 236 nm formed by L1236 cells after incubation with arachidonic acid (40 µM) for 10 min. Inserted: UV-spectrum of the materials, which coeluted with 15 (S)-HETE and 12 (S)-HETE, respectively.
Figure 2a: RP-HPLC chromatogram of products with UV absorbance at 280 nm formed by L1236 cells after incubation with arachidonic acid (40 µM) for 10 min. The numbers 1-5 correspond to retention times of synthetic standards: 1) 8 (R), 15 (S)-LTB₄; 2) 8 (S), 15 (S)-DHETE; 3) 8 (S), 15 (S)-LTB₄, 4) 8 (R), 15 (S)-DHETE and 5) 14 (R), 15 (S)-DHETE.
Figure 2b: UV-spectra of metabolites eluting under peaks I-IV.
Figure 3: 400 eV (Elab) CID spectrum of [M-H] -ions of the material in peak II. Upper panel: LTC₄; middle panel: peak II; lower panel: synthetic 14,15-LTC4.
Figure 4: 400 eV (Elab) CID spectrum of [M-H]-ions of the material in peak IV. Upper panel: LTD₄; middle panel: peak IV; lower panel: synthetic 14,15-LTD₄.
Figure 5: RP-HPLC chromatogram of the products generated by L1236 cells after incubation with 14,15-LTA₄ (2 µM) for 30 min. The numbers 1, 3 and 5 correspond to retention times of synthetic standards for 8 (R), 15 (S)-LTB₄; 8 (s), 15 (S)-LTB₄, and 14 (R), 15 (S)-LTB₄, respectively. Peaks I-IV had identical retention times and UV spectra as the peaks I-IV described in Fig. 2.
   Insert: UV-spectrum of the material eluting under peak V.
Figure 6: 400 eV (Elab) CID spectrum of [M-H] - ions of the material in peak V. Upper panel: LTE₄; middle panel: peak V; lower panel: synthetic 14, 15-LTE₄.
Figure 7: Overview of the 15-LO pathway and suggested pathway for the biosynthesis of 14,15-leukotrienes (Eoxins).
Figure 8: Incubation of L1236 cells with arachidonic acid.
   a) Time course of the formation of 14,15-LTC₄ and 14,15-LTD₄ after incubation with 40 µM arachidonic acid.
   b) Dose-response curve of the formation of 14,15-LTC₄ and 14,15-LTD₄ after 5 min incubation with various concentrations of arachidonic acid as indicated.
Figure 9: RP-HPLC chromatogram of the products generated by human eosinophil cells after incubation with arachidonic acid (20 µM) for 5 min.
   a) HPLC chromatogram of the products formed. Co-elution with synthetic standards for Eoxin C₄, LTC₄, 8,15-DHETEs (see fig 2) and 14 (R), 15 (S)-DHETE, is indicated.
   b) UV spectrum of the material under the peak co-eluting with synthetic Eoxin C₄.
Figure 10: Positive ion MS/MS spectra of a) the material coeluting with Eoxin C₄ b) synthetic Eoxin C₄ and c) synthetic LTC₄.
Figure 11: Eosinophils were incubated with 5, 10, 20 and 40 µM arachidonic acid for a) 2 minutes, b) 5 minutes and c) 10 minutes. Mean of two separate experiments.
Figure 12A: Formation of Eoxin C₄ (14,15-LTC₄) and a series of 8,15-DHETE and 14,15-DHETE metabolites after incubation of chopped human nasal polyps with 40 µM arachidonic acid for 5 minutes.
Figure 12B: the UV spectrum of the material under the peak co-eluting with synthetic Eoxin C₄ (14,15-LTC₄).
Figure 13A: Formation of Eoxin C₄ (14,15-LTC₄) and a series of 8,15-DHETE and 14,15-DHETE metabolites after incubation of human dendritic cells with 40 µM arachidonic acid for 5 minutes.
Figure 13B: the UV spectrum of the material under the peak co-eluting with synthetic Eoxin C4 (14,15-LTC₄).
Figure 14: Immunostaining. Expression of 15-LO in a lymph node from a patient with Hodgkin's lymphoma. A polyclonal 15-lipoxygenase antiserum, raised against a specific peptide sequence of human 15-LO, was utilised followed by a secondary peroxidase-conjugated antibodies. A strong staining in Reed-Stembergs cells was observed (the multinucleated cell in the central part of the figure).
Figure 15: Immunostaining. Expression of 15-LO in a lung biopsy from a patient with sarcoidosis. A polyclonal 15-lipoxygenase antiserum, raised against a specific peptide sequence of human 15-LO, was utilised followed by a secondary peroxidase-conugated antibodies. A strong staining in epitheloid cells (granuloma) was observed.
Figure 16: Immunostaining. Expression of 15-LO in specimen from A) synoviocytes and B) condrocytes from a rheumatoid arthritis patient C) pyramidal cells from brain cortex of an Alzheimer patient, and D) astrocytes from an astrocytoma specimen.
Figure 17: Metabolism of A) Eoxin A₄ (14,15-LTA₄) to Eoxin C₄ (14,15-LTC₄) and B) LTA₄ to LTC₄ by purified GSTM1-1.
Figure 18: Metabolism of Eoxin A₄ (14,15-LTA₄) to Eoxin C₄ (14,15-LTC₄) by various soluble glutathione S-transferases.
Figure 19: HPLC chromatogram of products formed after incubation of subcellular fractions of eosinophils with EoxA₄ (40 µM) for 5 minutes.
   A) supernatant and B) membrane fraction.
Figure 20: HPLC chromatogram of products formed in human eosinophils after incubation with EoxA₄ (10 µM) (upper panel) LTA₄ (10 µM) (middle panel) or both (lower panel) for 5 minutes at 37°C.
Figure 21: Effect of a 5-lipoxygenase inhibitor (BW70C) and an LTC₄ synthase inhibitor (MK886) on formation of Eoxin C₄ and LTC₄. Eosinophils were preincubated in absence or presence of inhibitor for 2 minutes prior to stimulation with A23187 (1 µM) and arachidonic acid (10 µM) for 5 minutes in 37°C.
Figure 22: Effect of Eoxin C₄ (14,15-LTC₄), Eoxin D₄ (14,15-LTD4), Eoxin E₄ (14,15 LTE₄) and LTC₄ on plasma leakage measured as reduced resistance in a model system with confluent bovine endothelium cells (maximal effect 70%).
Figure 23: Eoxin-induced oxidative metabolism by GM-CSF-primed neutrophils. Kinetic chemiluminescence curves illustrating the measured parameters.
   a: Purified blood neutrophils stimulated (filled symbols) or GM-CSF treated and subsequently stimulated (open symbols) to produce oxygen free radicals. "BLX-26" is used to indicate BLX-926.
   b: EoxD₄ (BLX-926) induced oxygen free radical by GM-CSF-primed neutrophils. Results show total radical production and are presented as % of Control (primed cells) and as mean +/- se, n = 13
   c: Scatter plot showing EoxD₄ (BLX-926) induced oxygen free radical by GM-CSF-primed neutrophils. Results show total radical production measured as count per minute (CPM x10⁶), n = 13. The line shows the reference line (k=1), indicating that all observations above the line are responders to the stimuli.

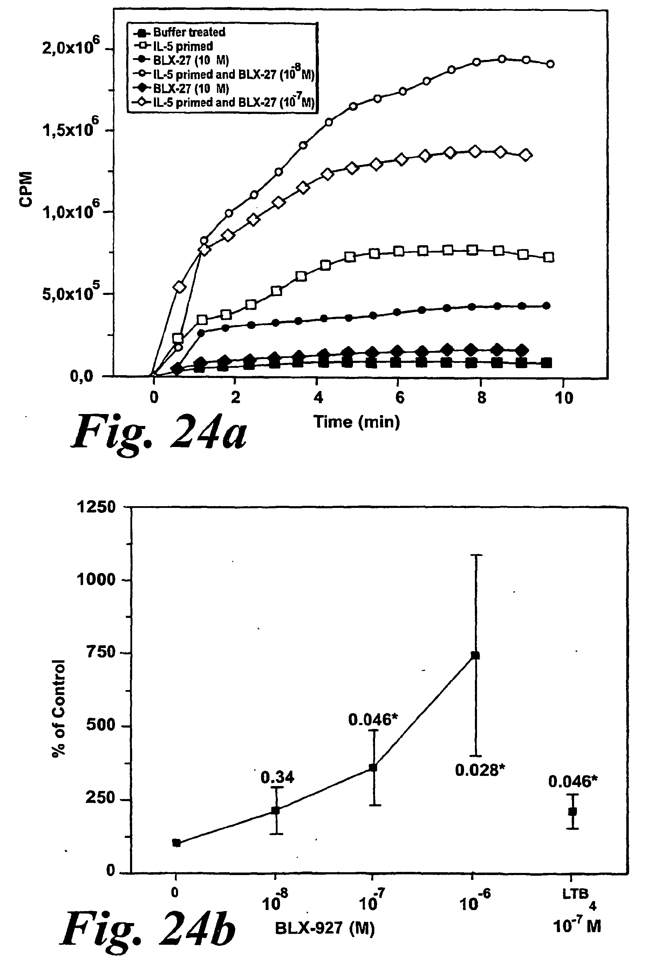

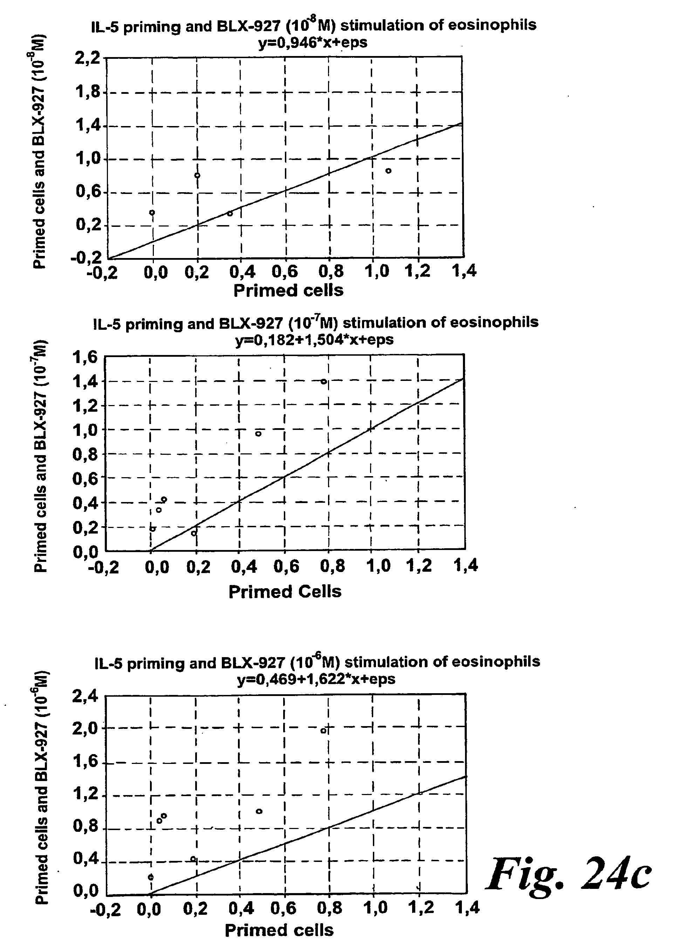

Figure 24: EoxE₄ (BLX-927). Kinetic chemiluminescence curves illustrating the measured parameters.
   a: Purified blood eosinophils stimulated (filled symbols) or IL-5 treated and subsequently stimulated (open symbols) to produce oxygen free radicals. "BLX-27" is used to indicate BLX-927.
   b: Stimulation and EoxE₄ (BLX-927) induced oxygen free radical by IL-5 primed eosinophils. Results show total radical production and are presented as % of Control (primed cells) and as mean +/- se, n = 6
   c: Scatter plot showing EoxE₄ (BLX-927) induced oxygen free radical by IL-5 primed eosinophils. Results show total radical production measured as count per minute (CPM x10⁶), n =6. The line shows the reference line (k=1), indicating that all observations above the line are responders to the stimuli.

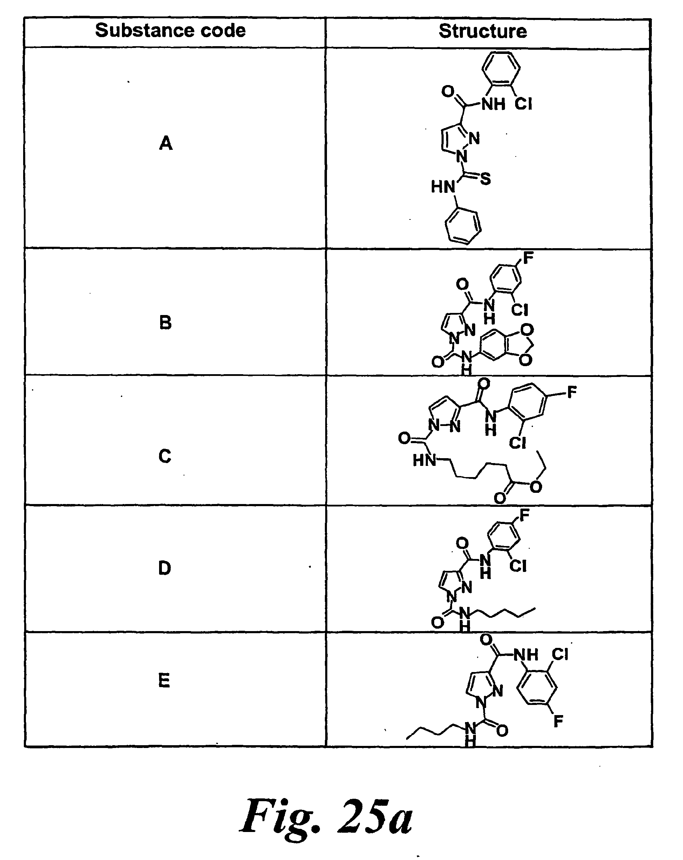

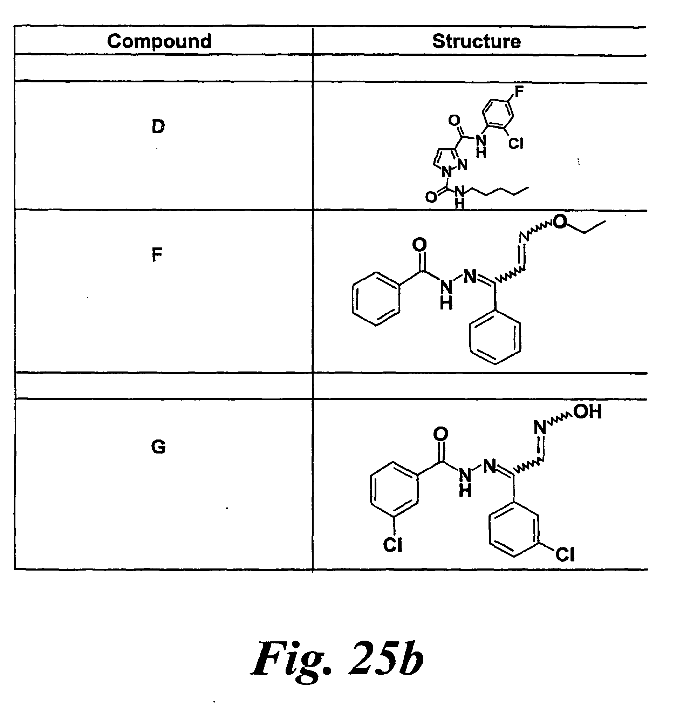

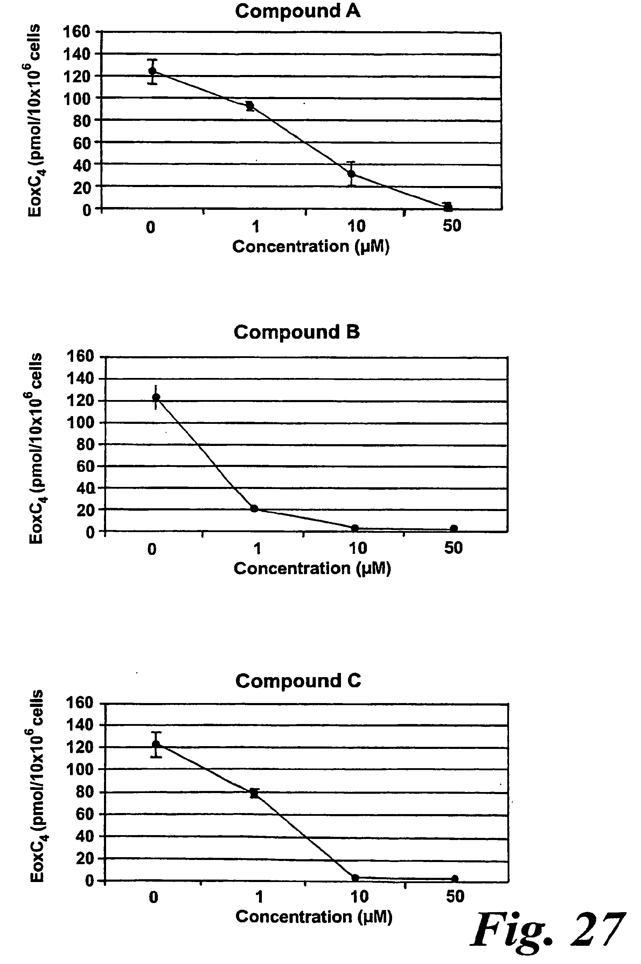

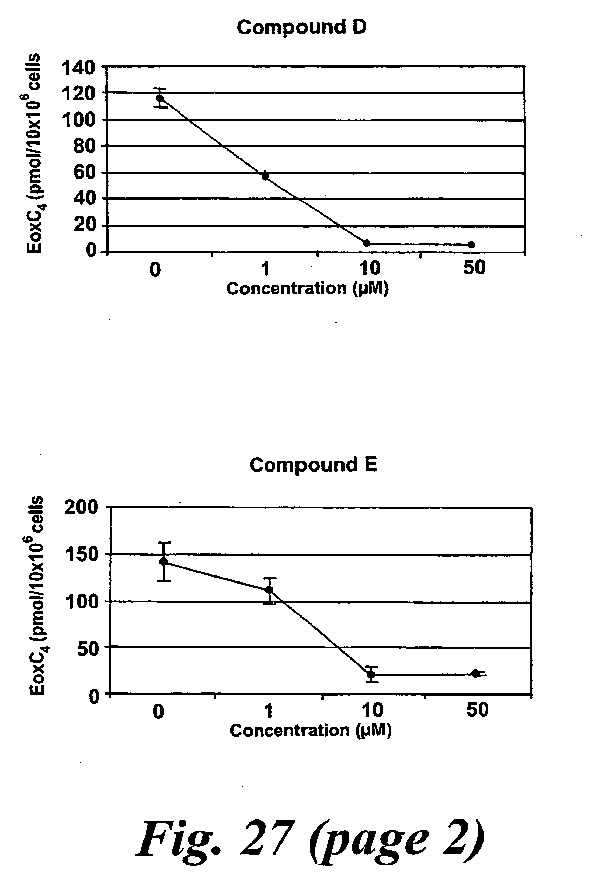

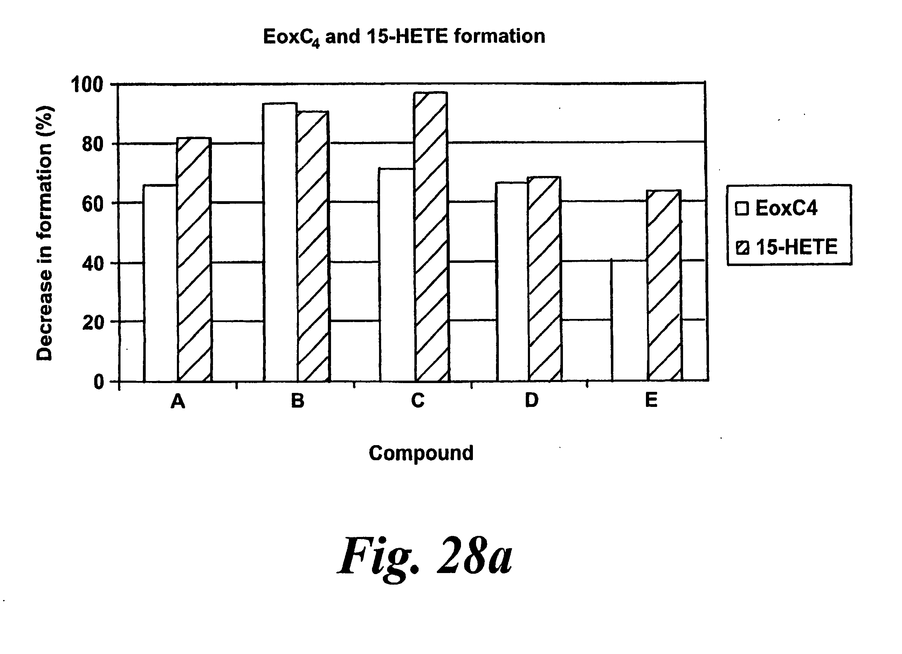

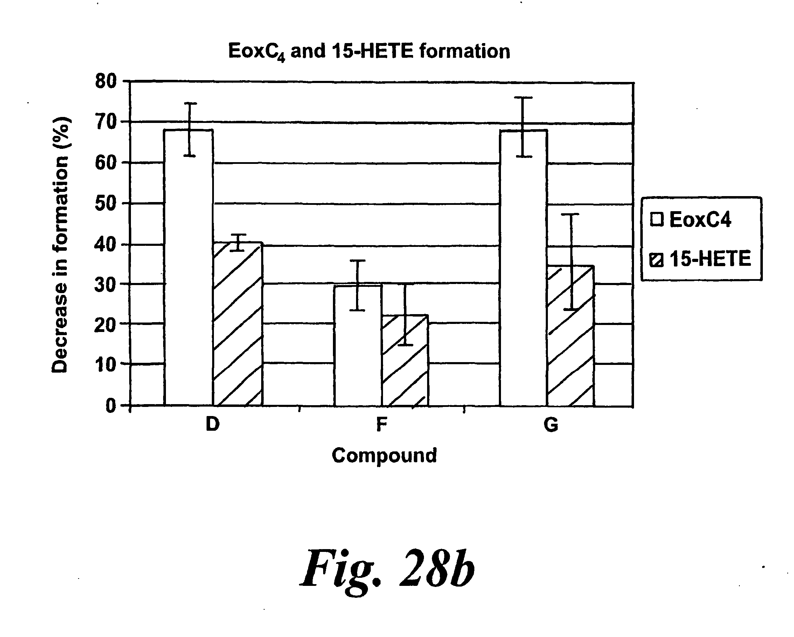

Figure 25: Structures of compounds tested in Example 7 and Figures 27 and 28.

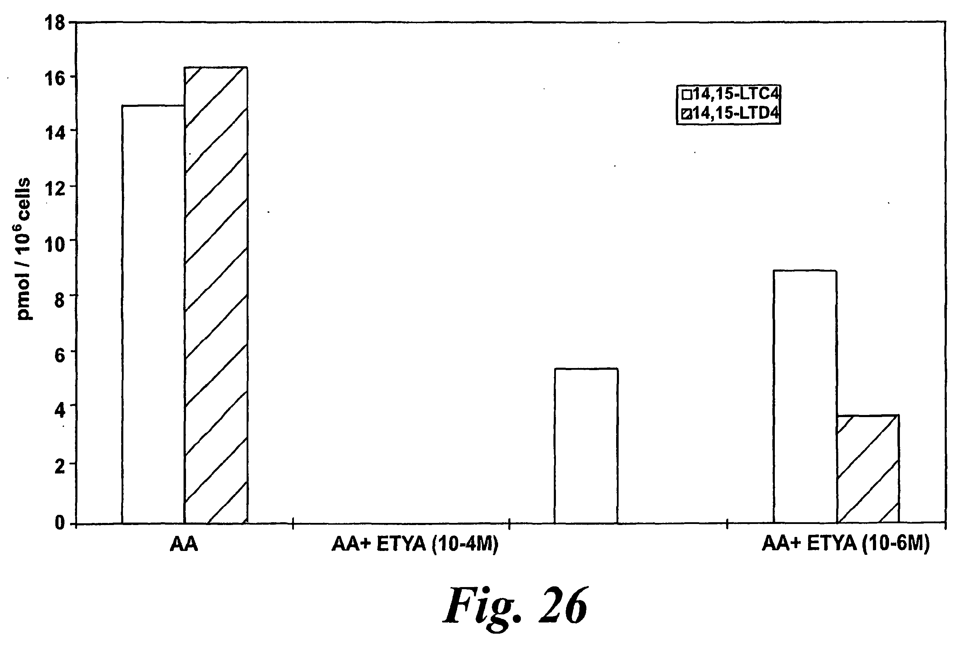

Figure 26: Effect of the leukotriene inhibitor ETYA on formation of Eoxin C₄ (14,15-LTC₄) and Eoxin D₄ (14,15-LTD₄).
Figure 27: Effect of test compounds on formation of Eoxin C₄.
Figure 28: Comparison of the effects of the tested compounds on Eoxin C₄ and 15-HETE formation. a) compounds A, B, C, D and E; b) compounds D, F and G.

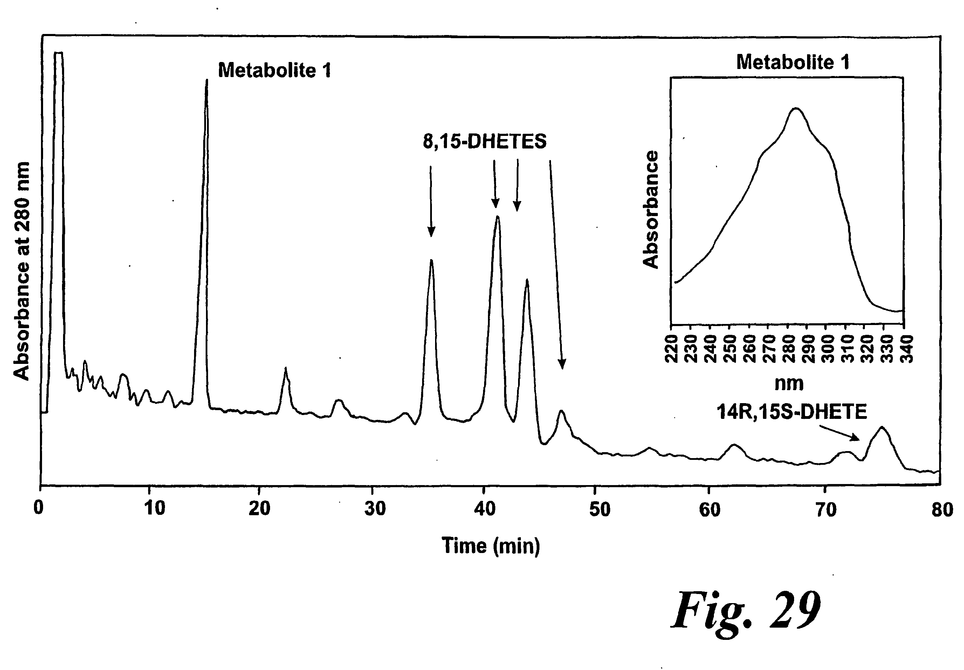

Figure 29: Formation of novel 15-lipoxygenase products in isolated human eosinophils. Isolated eosinophils (10x106 cells) were resuspended in phosphate buffered saline (1 ml) and pre-incubated for 2 minutes prior to stimulation with arachidonic acid (10 µM) for 5 minutes in 37 °C. The reaction was terminated by addition of three volumes of methanol. After evaporation, the sample was resuspended in mobile phase and subjected to analysis in a RP-HPLC-UV system using AcN:MeOH:H20:HAc (29:19:72:0,8 by volume) pH 5,6 as mobile phase.

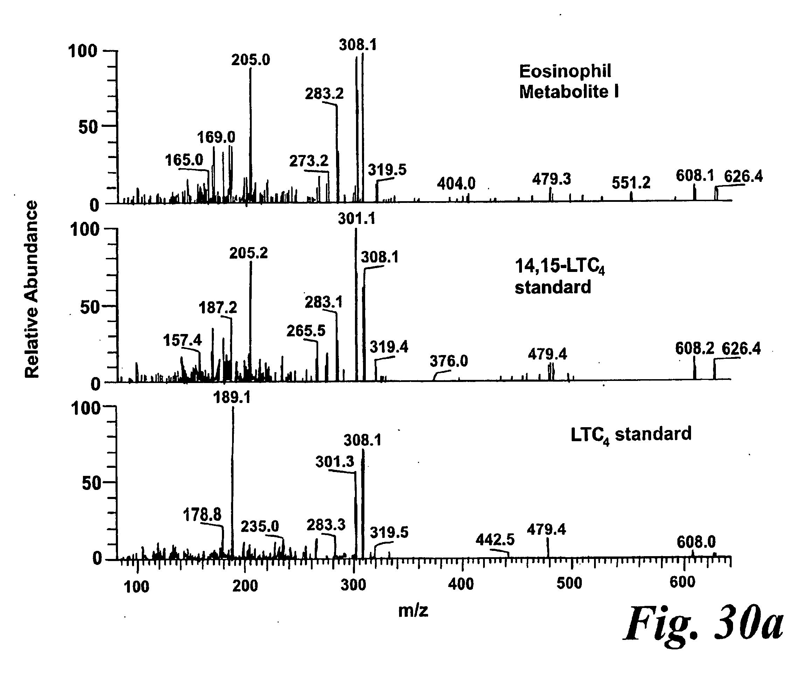

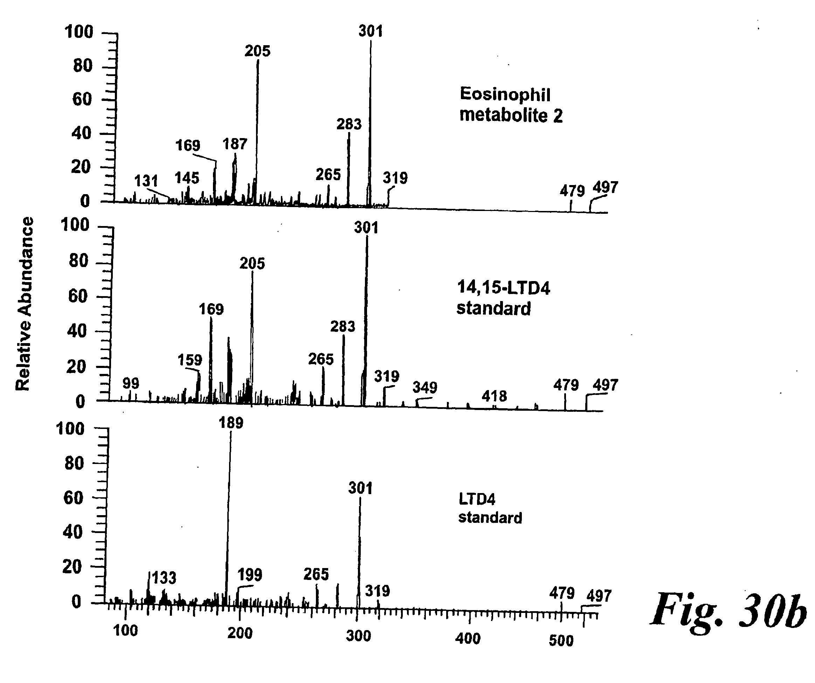

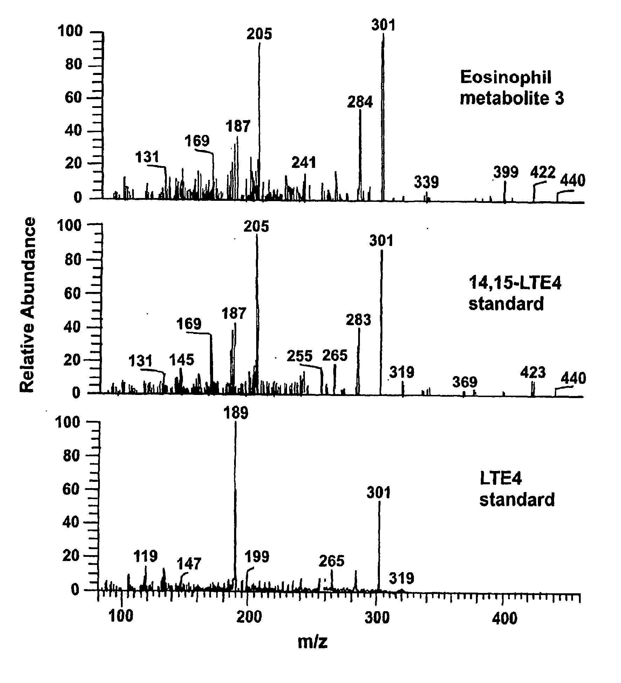

Figure 30: Liquid Chromatography-Mass Spectrometry analysis of novel 15-lipoxygenase products. Panel A: LC-MS/MS spectra of metabolite I formed by eosinophils compared to synthetic 14,15-LTC4 and LTC4 standards. Upper panel shows the spectrum of a product ion scan of 626.3 [M+H]+ of a metabolite formed in eosinophils after challenge with arachdonic acid (10 µM) for 5 min in 37 °C.
   The middle panel is the spectrum of synthetic 14,15-LTC4 standard and the lower panel is the spectrum of synthetic LTC4 standard. Panel B: LC-MS/MS spectra of metabolite II formed by eosinophils compared to 14,15-LTD4 and LTD4 synthetic standards. Upper panel shows the spectrum of a product ion scan of 497.2 [M+H]+ of a metabolite formed from 14,15-LTC4 (10 µM) incubated with eosinophils for 60 min in 37 °C. The middle panel is the spectrum of synthetic 14,15-LTD4 standard and the lower panel is the spectrum of synthetic LTD4 standard. Panel C: LC-MS/MS spectra of metabolite III formed by eosinophils compared to 14,15-LTE4 and LTE4 synthetic standards. Upper panel shows the spectrum of a product ion scan of 440.2 [M+H]+ of a metabolite formed from 14,15-LTD4 (10 µM) incubated with eosinophils for 60 min in 37 °C. The middle panel is the spectrum of synthetic 14,15-LTE4 standard and the lower panel is the spectrum of synthetic LTE4 standard.

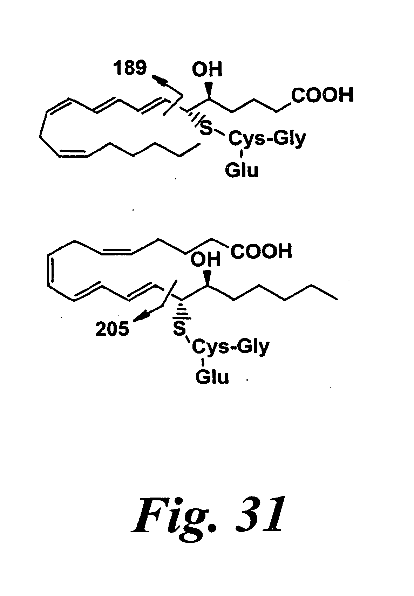

Figure 31: Collision induced dissociation fragments distinguishing 14,15-leukotrienes from cysteinyl leukotrienes. A prominent difference of the cysteinyl leukotrienes and 14,15-leukotrienes in the positive ion mode MS/MS product ion spectra are m/z 189 and 205. The probable origins of these fragment ions are indicated.

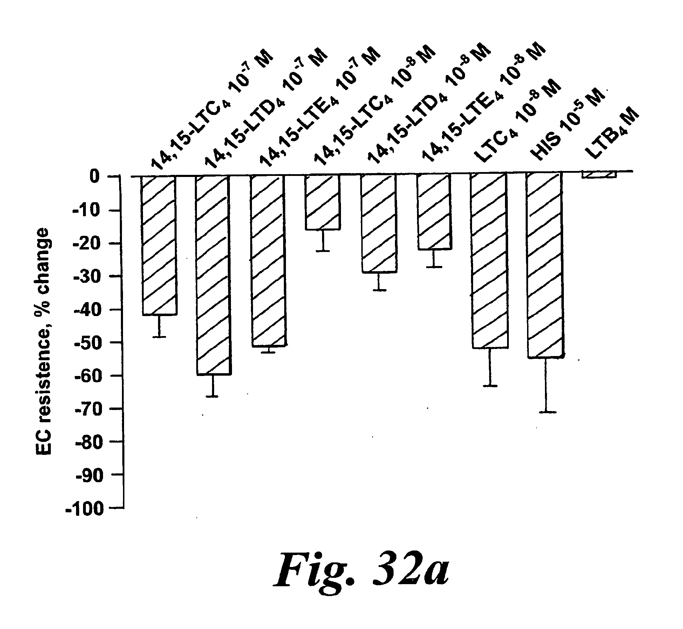

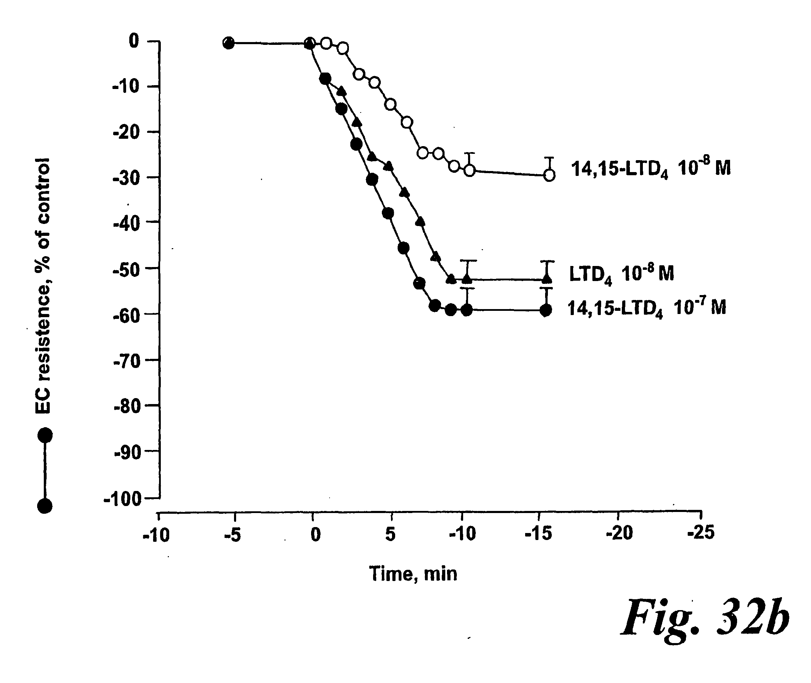

Figure 32: Alterations in vascular permeability induced by 14,15-leukotrienes. Panel A: Analysis of rapid changes in endothelial cell barrier function in response to 14,15-LTC4, 14,15-LTD4 and 14,15-LTE4. LTC4 (10⁻⁸ M) and histamine (10⁻⁵ M). Analyses were accomplished by continuous registration of transendothelial electrical resistance (TEER) with electrodes on each side of the endothelial cell monolayer. Maximal effect in this model is -70% change in resistance. Two typical experiments out of five is demonstrated. Each value is mean value of triplicate determinations. Panel B: Time course for changes in endothelial cell barrier function caused by 14,15-LTD4 (10⁻⁸M and 10⁻⁷M, respectively) in comparison to LTD4 (10⁻⁸M).

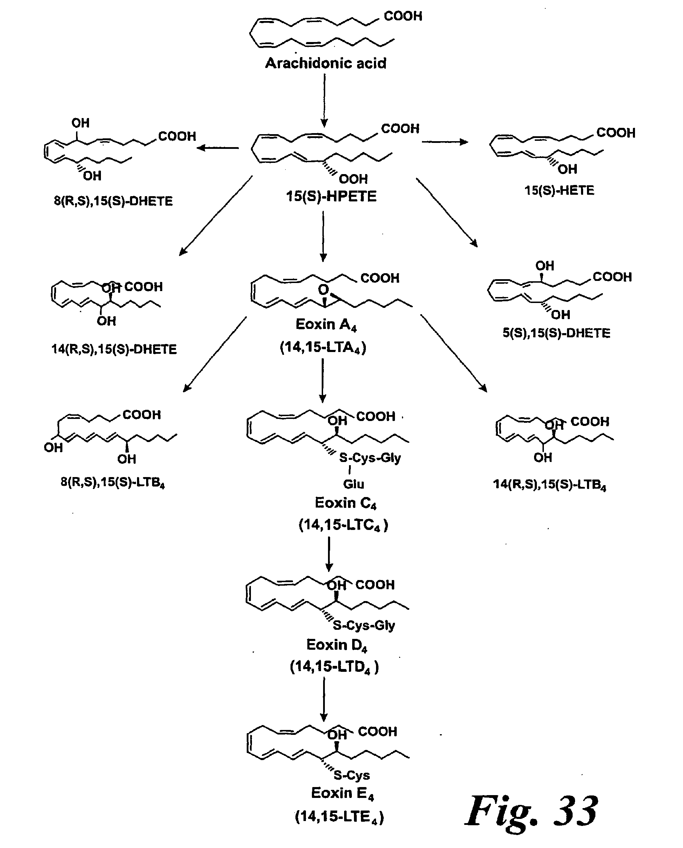

Figure 33: Proposed metabolic pathway for the formation of 14,15-leukotrienes (Eoxins).

### Example 1: Demonstration of the biosynthesis of 14,15-LTC₄, 14,15-LTD₄, and 14,15-LTE₄ (i.e. Eoxin C₄, Eoxin D₄ and Eoxin E₄).

### Materials and methods

Synthetic 14,15-oxido-5,8,10,12 (Z,Z,E,E)-eicosatetraenoic acid (14,15-LTA₄), 14 (R)-glutathionyl-15 (S)-hydroxy-5,8,10,10 (Z,Z,E,E)-eicosatetraenoic acid (14,15-LTC₄), 14 (R)-cysteinyl-glycyl-15 (S)-hydroxy-5,8,10,12 (Z,Z,E,E)-eicosatetraenoic acid (14,15-LTD₄), 14 (R)-cysteinyl-15 (S)-dydroxy-5,8,10,12 (Z,Z,E,E)-eicosatetraenoic acid (14,15-LTE₄), 8 (R), 15 (S)-dihydroxy-5,9,11,13 (Z,E,E,E)-eicosatetraenoic acid (8 (R), 15 (S)-LTB₄), 8 (S), 15 (S)-dihydroxy-5,9,11,13 (Z,E,Z,E)-eicosatetraenoic acid (8 (S), 15(S)-DHETE), 8 (S), 15 (S)-dihydroxy-5,9,11,13 (Z,E,E,E)-eicosatetraenoic acid (8 (S), 15 (S)-LTB₄), 8' (R), 15 (S)-dihydroxy-5,9,11,13 (Z,E,Z,E)-eicosatetraenoic acid (8 (R), 15 (S)-DHETE), 14 (R), 15 (S)-dihydroxy-5,8,10,12 (Z,Z,E,E)-eicosatetraenoic acid (14 (R), 15 (S)-DHETE or 14 (R), 15 (S)-LTB₄), 15 (S)-hydroxy-5,8,11,13 (Z,Z,Z,E)-eicosatetraenoic acid (15-HETE), 12 (S)-hydroxy-5,8,10.14 eicosatetraenoic acid (Z,Z,E,Z) (12 (S)-HETE) and 13 (S)-hydroxyoctadeca-9,11 (Z,E)-dienoic acid (13 (S)-HODE) were from Biomole Inc. (Plymouth Meeting, USA). Cell culture medium RPMI 1640, foetal calf serum, penicillin, streptomycin and glutamine were obtained from Gibco, BRL. HPLC solvents were obtained from Rathburn Chemicals (Walkerburn, United Kingdom).

### L1236 cell line

The cell line L1236 was originally generated from the peripheral blood of a patient suffering from Hodgkin's lymphoma (HL) of mixed cellularity subtype¹⁵. The cells were grown in suspension at 37°C in an atmosphere of 5% CO₂. Culture medium was RPMI 1640 containing 100 U/ml penicillin and 100 µg/ml streptomycin supplemented with 10% foetal calf serum and 2 mM glutamine.

### Experimental conditions and HPLC analysis

The cell suspensions were centrifuged at 200 x g for 5 min and the pellet was suspended in PBS. After washing twice in PBS, the cells were solved in PBS at a concentration of 10-20 x 10⁶ cells/ml. The sample was prewarmed for 2 minutes at 37°C prior to the addition of arachidonic acid, 14,15-LTA₄ or other indicated metabolite and subsequently incubated for the period indicated. The incubations were terminated by addition of 3 vol. ice-cold methanol. Precipitated proteins and cell fragments were removed by centrifugation and excess water and ethanol were evaporated under reduced pressure. The residues were solved in methanol and transferred to a test tube and evaporated to dryness under a stream of nitrogen. Subsequently, the residues were reconstituted in the chromatographic solvent described below. The samples were applied to an octadecyl reverse-phase column (Chromabond, C₁₈, 100 mm, Macherey-Nagal, Duren, Germany), eluted at a flow rate of 0.4 ml/min. The mobile phase was acetronitriletmethanol/water/acetic acid (28:18:72:0.8, by vol., pH adjusted to 5.6 with 30% NaOH) or methanol/water/acetic acid (72.5:27.5:0.01, by vol) for analysis of cysteinyl-containing arachidonic acid metabolites and dihydroxy acids or monohydroxy acids, respectively. Eluted metabolites were detected and quantified utilising a programmable Waters 991 diode array spectrophotometer.

### Negative-ion electrospray tandem mass spectrometry.

Spectra were recorded on a hybrid magnetic sector/time-of flight tandem instrument at laboratory frame collision energy of 400 eV with Xe as the collision gas as described¹⁶.

### Results

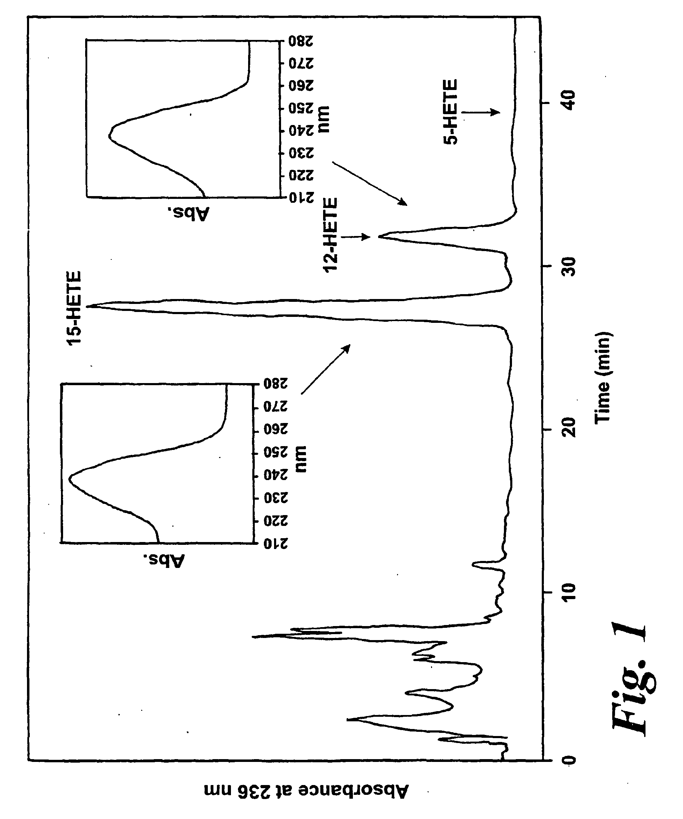

Incubation of L1236 cells with arachidonic acid for 10 min led to the formation of a major product, which co-chromatographed with synthetic 15 (S)-HETE (Figure 1). In addition, a minor peak, with UV absorbance maximum at 236 nm, which coeluted with synthetic 12 (S)-HETE, was observed. The UV-spectra of the materials in these peaks are in agreement with the reported spectrum for 15-HETE and 12-HETE, respectively. The ratio between 15-HETE vs 12 HETE was about 9:1, which is in agreement with 15-LO type 1 catalysed formation of these products⁵. These results demonstrate that the cells possessed 15-LO type 1 activity but no 5-lipoxygenase activity. Linoleic acid was an excellent substrate and L1236 cells efficiently metabolised linoleic acid to 13-HODE (data not shown), which is in agreement with the expression of 15-LO type 1 in these cells. When sonicated cells were analysed by SDS/PAGE followed by Western blotting with an anti-15-LO rabbit polyclonal antibody, an immunoreactive band appeared that comigrated with reticulocyte 15-LO (data not shown).

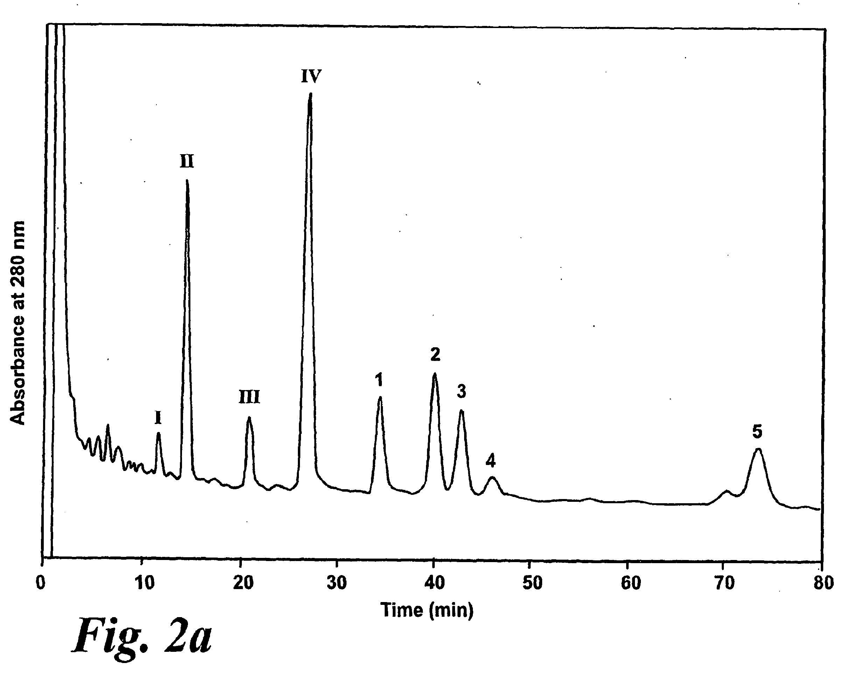

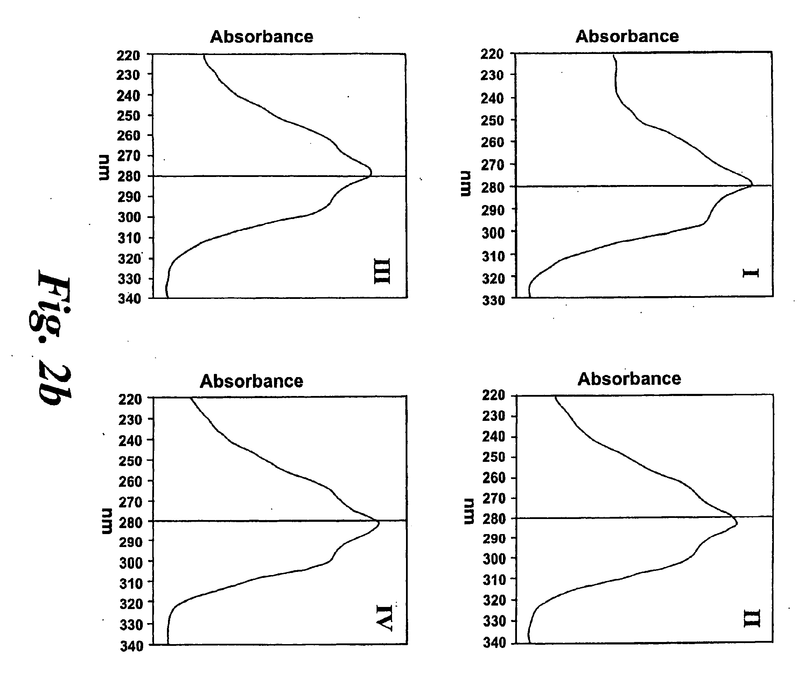

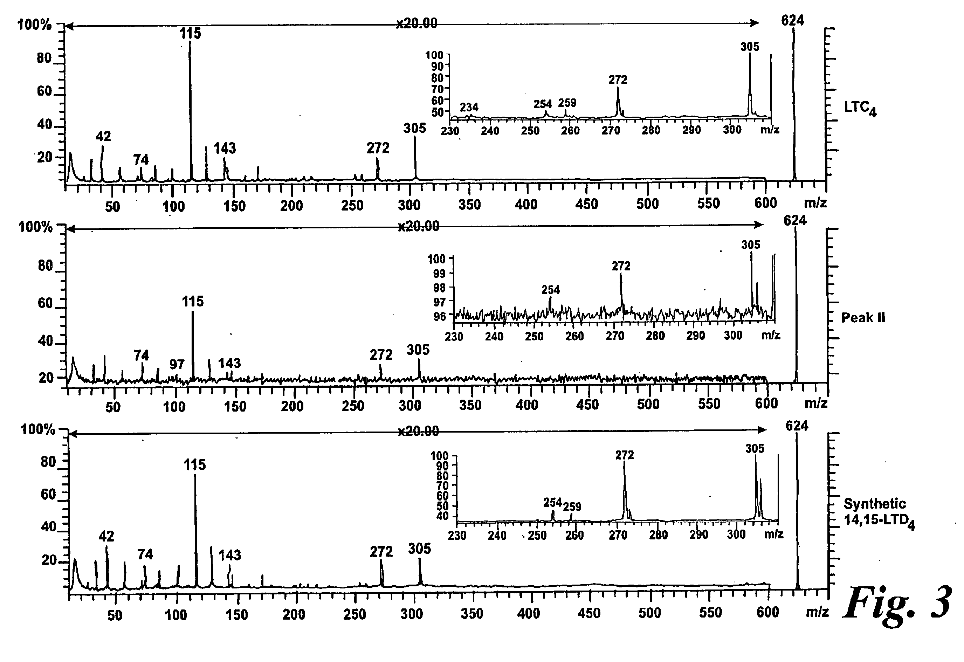

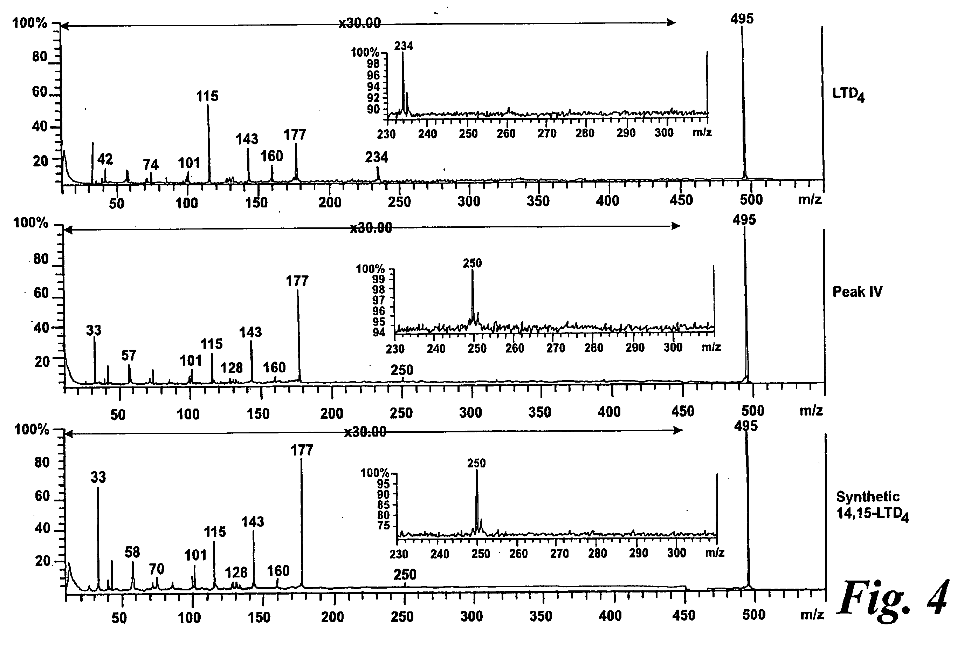

In addition to the peaks absorbing UV at 236 nm a number of peaks with UV absorbance maximum around 270 nm and retention times between 5-7 min were observed in the chromatogram. An acetonitrile based mobile phase was used to get separation of these products. Figure 2a shows a typical RP-HPLC chromatogram of the products formed after 10 min incubation with arachidonic acid. Five peaks (1-5) containing a conjugated triene spectrum and UV absorbance maximum at 272 nm was observed. These peaks had elution times corresponding to synthetic standards of 8 (R), 15 (S)-LTB₄; 8 (S), 15 (S)-DHETE; 8 (S), 15 (S)-LTB₄; 8 (R), 15 (S)-DHETE and 14 (R), 15 (S)-DHETE, respectively. The pattern of these products was almost identical to that reported for human airway epithelium incubated with arachidonic acid¹⁷. However, the two additional and major peaks in the chromatogram also possessed a conjugated triene spectrum but had UV absorbance maximum at 282 nm (II and IV) (Figure 2b). The eluted material was collected and analysed by negative-ion electrospray tandem mass spectrometry. Figure 3 shows the collision-induced dissociation (CID) spectrum of [M-H] ions of the material from peak II (middle panel). The mass spectrum was almost identical to LTC₄ (upper panel), with molecular ion at 624, except the presence of the ion m/z 306 which was not found in LTC₄ (Figure 3). In light of the observed 15-LO activity in these cells, the mass spectrum indicated that the metabolite was 14 (R)-glutathionyl-15 (S)-hydroxy-5,8,10,12-eicosateraenoic acid (14,15-LTC₄). To confirm that the product was indeed 14,15-LTC₄, synthetic 14 (R)-glutathionyl-15 (S)-hydroxy-5,8,10,12 (Z,Z,E,E)-eicosatetraenoic acid was prepared. The mass spectrum of this synthetic metabolite (Figure 3, lower panel) was identical to the spectrum of the material in peak II. The corresponding CID spectrum of [M-H]- ions of the other major metabolite, peak IV, is shown in Figure 4. This mass spectrum is very similar to the spectrum for LTD₄ with the same molecular ion m/z 495, except the presence of the ions 250 and 251 which was not found in LTD₄, indicating that the metabolite is 14 (R)-cysteinyl-glycyl-15 (S)-hydroxy-5,8,10,12-eicosateraenoic acid (14,15-LTD4). The corresponding CID spectrum of [M-H]- ions of synthetic 14 (R)-cysteinyl-glycyl-15 (S)-hydroxy-5,8,10,12 (Z,Z,E,E)-eicosatetraenoic acid was also identical to the spectrum of metabolite IV (Figure 4, lower panel). Furthermore, synthetic 14, 15-LTC₄ and 14, 15-LTD₄ coeluted exactly with metabolite II and IV, respectively, on RP-HPLC. Incubation of the cells with synthetic 14, 15-LTC₄ led to the formation of 14, 15-LTD₄ (data not shown). In addition, two other minor peaks with conjugated triene spectrum and UV absorbance maximum at 278 nm were observed (Figures 2a and b, peaks I and III). The material in peak III was analysed by negative-ion electrospray tandem mass spectrometry. The CID spectrum of this metabolite was identical to the CID spectrum for 14, 15-LTD₄ (data not shown). The exact structure of this metabolite has not been established. However, the CID spectrum and UV profile of this metabolite are consistent with an all trans triene structure since the spectrum is shifted 4 nm hypochromically compared to the spectrum of 14, 15-LTD₄. The material in peak III is therefore probably 8-trans-14, 15-LTD₄. The material in peak I was insufficient to obtain a mass spectrum, but in line with the formation of 8-trans-14, 15-LTD_{4,} it is very likely that the metabolite is 8-trans-14, 15-LTC₄. Furthermore, incubation of the cells with synthetic 14, 15-LTC₄ or 14, 15-LTD₄ led to the formation of metabolite I and III, respectively, demonstrating that the postulated all trans trienes were formed from the parent 14, 15-LTC₄ and 14, 15-LTD₄ (data not shown).

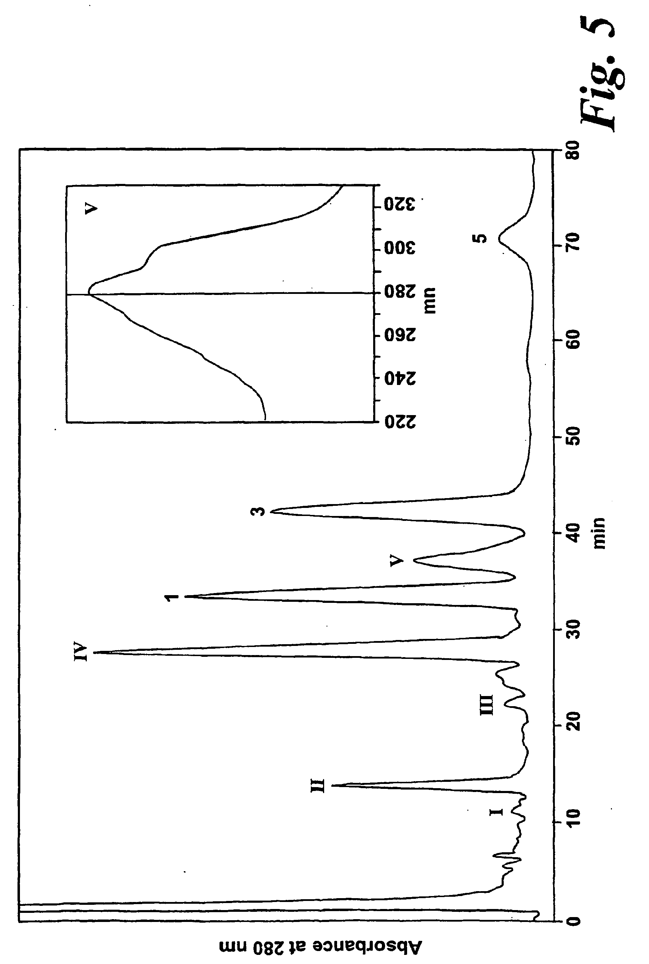

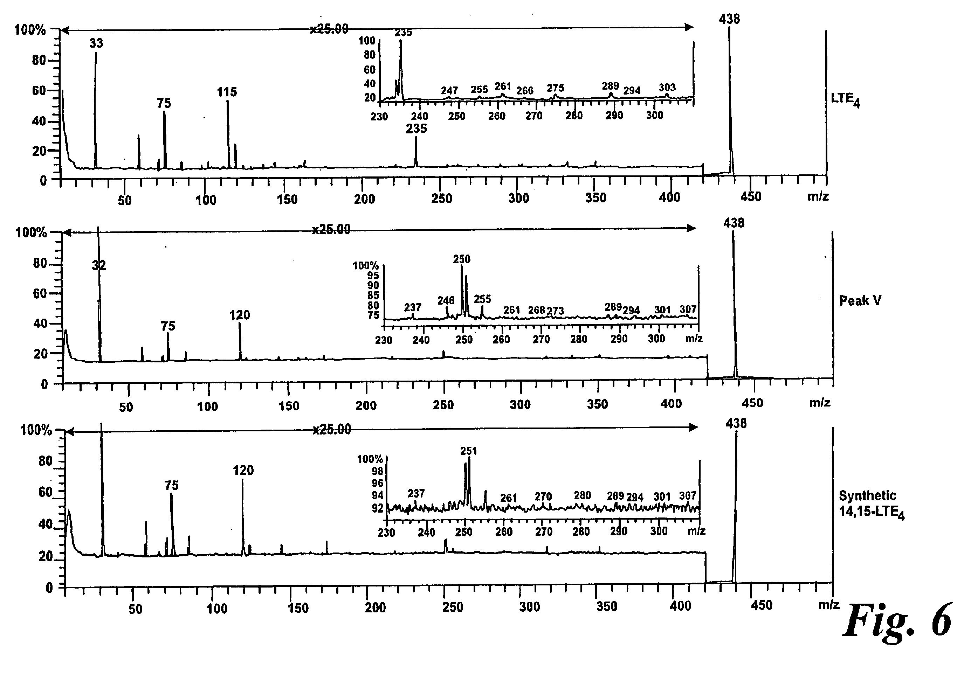

In order to elucidate the mechanism of formation and double bond structure of 14, 15-LTC₄ and 14, 15-LTD₄, the cells were incubated with 2 µM synthetic 14, 15-oxido-5,8,10,12 (Z,Z,EE)-eicosatetraenoic acid (14, 15-LTA₄) for 30 min. Figure 5 shows the HPLC chromatogram of the products generated by the cells. Three peaks with UV absorbance maximum at 272 nm and conjugated triene spectrum were observed (peaks 1, 3 and 5). These products were also formed when the cells were incubated with arachidonic acid (Figure 2a) and the products co-chromatographed with synthetic 8 (R), 15 (S)-LTB₄; 8 (S), 15 (S)-LTB₄ and 14 (R), 15 (S)-LTB₄ respectively. Three peaks were observed which had a conjugated triene spectrum and UV absorbance maximum at 282 nm (peaks II, IV and V). Metabolites II and IV had identical retention times with synthetic 14, 15-LTC₄ and 14, 15-LTD₄ respectively, on HPLC, demonstrating that these metabolites were 14, 15-LTC₄ and 14, 15-LTD₄. The material in peak V contained a conjugated triene and had UV absorbance maximum at 282 nm. This metabolite was not detected when the cells were incubated with arachidonic acid (Figure 2a) due to co-elution on HPLC with the double dioxygenation product 8,15-DHETE. Since this latter product is not formed from 14, 15-LTA₄, metabolite V was now visible on the HPLC chromatogram and not longer hidden behind the double dioxygenation product. Metabolite V was also formed when the cells were incubated with synthetic 14, 15-LTD₄ (data not shown). The material in peak V, formed after incubation of the cells with synthetic 14, 15-LTD₄, was analysed by negative-ion electrospray tandem mass spectrometry. Figure 6 shows that the mass spectrum of the material in peak V was identical to the spectrum of synthetic 14 (R)-cysteinyl-15 (S)-hydroxy-5,8,10,12-eicosateraenoic acid (14,15-LTE₄). Metabolite V also had identical retention time with synthetic 14,15-LTE₄ on HPLC. In addition, smaller amounts of 8-trans- 14, 15-LTC₄ (peak I) and 8-trans-14, 15-LTD₄ (peak III) were produced by the cells after incubation with 14, 15-LTA₄.

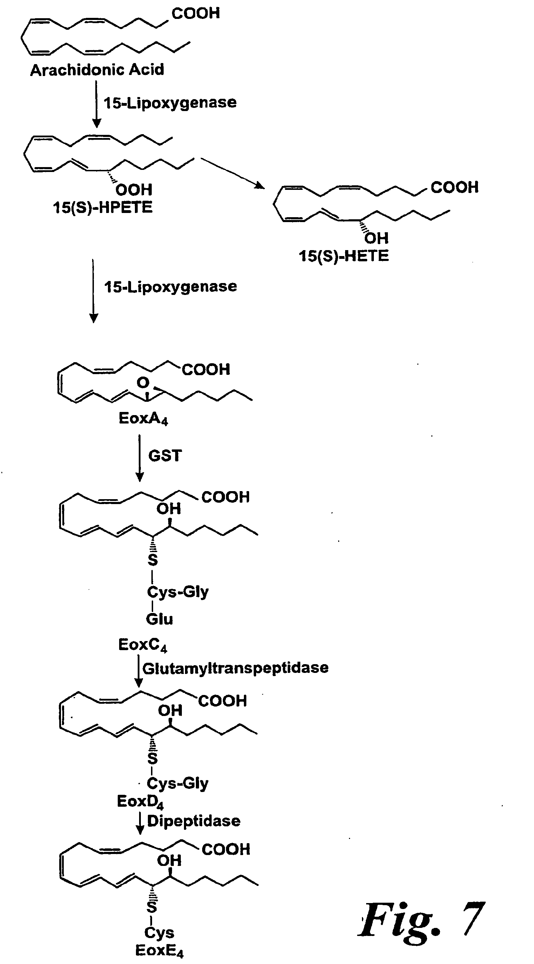

An overview of the 15-LO pathway and the suggested pathway for the biosynthesis of the 14, 15-LTC₄, 14, 15-LTD₄ and 14, 15-LTE₄ is shown in Figure 7.

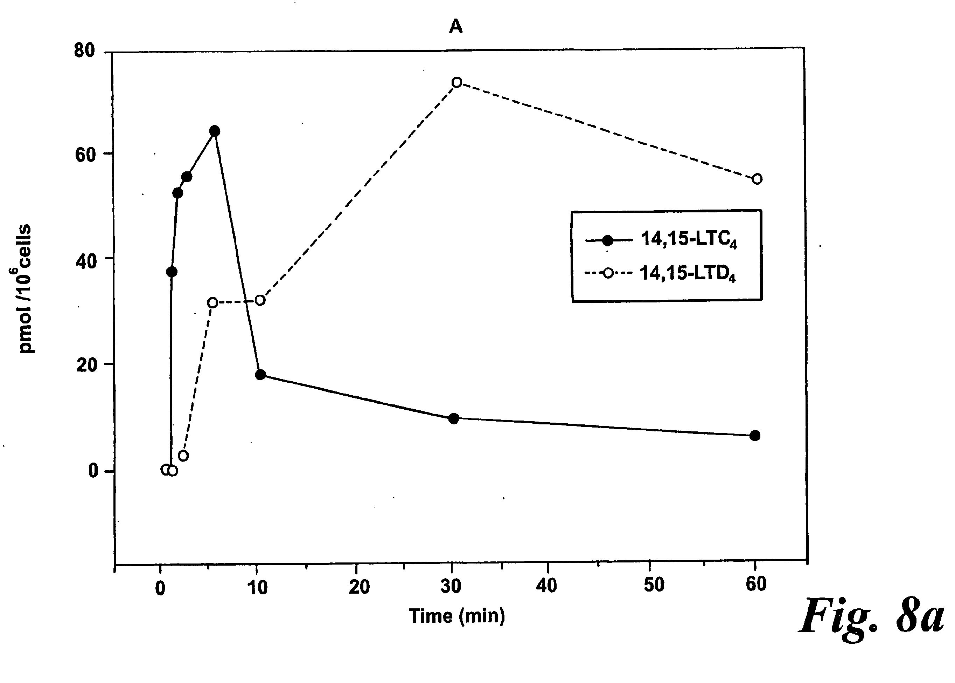

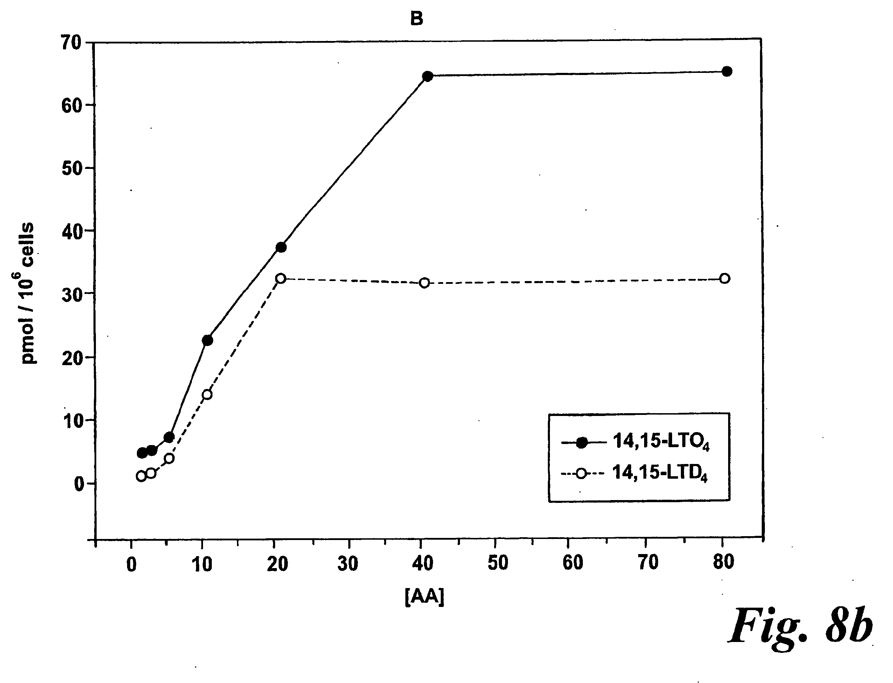

The time-courses of the formation of 14, 15-LTC₄ and 14, 15-LTD₄ after incubation with arachidonic acid (40 µM) are shown in Figure 8a. Substantial amount of 14, 15-LTC₄, 37 pmol/10⁶ cells, was produced by 10 × 10⁶ cells already after 30 sec of incubation with arachidonic acid. The maximal level of 14, 15-LTC₄ was observed after 5 min incubation and thereafter the level of 14, 15-LTC₄ declined with time. Measurable amount of 14, 15-LTD₄ was observed after 2 min of incubation with arachidonic acid and the levels increased with time and reached maximal level after 30 min. It was not possible to measure the level of 14, 15- . LTE₄ in this experiment, since 8, 15-DHETE co-chromatographed with 14, 15-LTE₄ in this HPLC system. The dose-response curves on the formation of 14,15-leukotrienes from arachidonic acid show that significant amounts of 14, 15-LTC₄ and 14, 15-LTD₄ were formed already at a concentration of 5 µM arachidonic acid ( 5 min incubation time; Figure 8b). The levels of these metabolites increased with the concentration of arachidonic acid and a plateau was reached at 40 µM arachidonic acid.
14, 15-LTC₄; 14, 15-LTD₄ and 14, 15-LTE₄ have been named, Eoxin C₄, Eoxin D₄ and Eoxin E₄, respectively.

### Example 2: Demonstration of the biosynthesis of Eoxin C₄ (14, 15-LTC₄) in other cells and tissues.

### Material and methods

### Isolation of human eosinophils

Venous blood (100ml) was drawn from healthy volunteers or from patients with eosinophilia, including hypereosinophilic syndrome (HES), chronic lymphocytic leukaemia (CLL) and certain inflammatory disorders. An initial centrifugation at 400 x G for 15 minutes was performed. The upper phase was discarded and the lower phase was collected and subjected to Dextran ™ sedimentation for 30 minutes in room temperature. After sedimentation, the upper phase containing white blood cells was centrifuged once at 400 x G for 10 minutes. The pellet was suspended in Lysis buffer and incubated for 30 minutes in room temperature to get rid of contaminating erythrocytes. Following incubation, density gradient centrifugation, using Lymphoprep ™ was performed. The polymorphonuclear (PMN) fraction, containing neutrophils and eosinophils, was collected and subjected to Magnetic Cell Sorting (MACS). Eosinophils were isolated by negative selection using CD16 antibodies conjugated to magnetic microbeads. Purity was confirmed by morphological examination and by staining with May Grunewald-Giemsa solution. Purity was always found to be greater that 95%.

### Incubation of cells

Isolated eosinophils (5-10 x 10⁶ / sample) were resuspended in phosphate buffered saline (1 ml / sample) and preincubated for 2 minutes in 37 °C prior to stimulation with ionophore A23187 (1 µM), arachidonic acid (5, 10, 20 or 40 µM) or both for 2, 5 or 10 minutes in 37 °C. The reactions were terminated by addition of three volumes of methanol.

### Analysis of 15-lipoxygenase products

In order to get rid of cellular debris and proteins, centrifugation was performed. Supernatants were evaporated under reduced pressure and the residues resolved in methanol and transferred to a test tube. Samples were thereafter evaporated to dryness under a steam of nitrogen, and finally resuspended in 200 µl mobile phase (acetonitril: methanol: H₂O: acetic acid, 29: 19: 72:0.8, pH adjusted to 5.6 with NH₃). The extracted samples were subjected to analysis in a reverse phase HPLC system, as described. Briefly, samples were applied to an octadecyl reverse phase column (Chromabond C₁₈, Macherey - Nagel, Duren, Germany) and elution rate was 1.2 ml/minute. Eluted compounds were detected and quantified utilising Waters 996 photo diode array spectrophotometer connected to Waters 1996 HPLC system

### Mass spectrometry

Mass spectrometry was performed as described in Example 1.

### Results

### Biosynthesis of Eoxin C₄ in human eosinophils

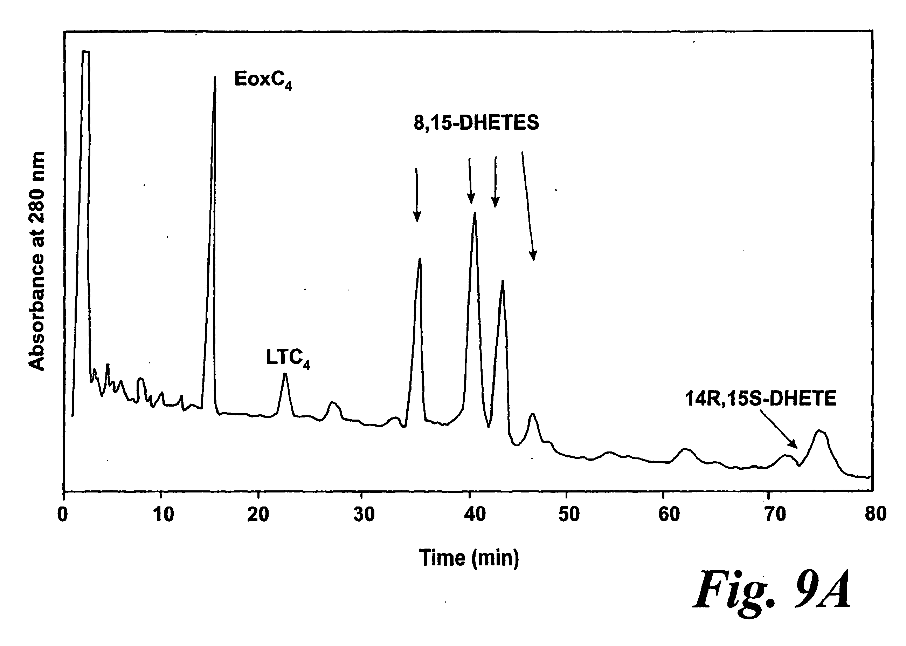

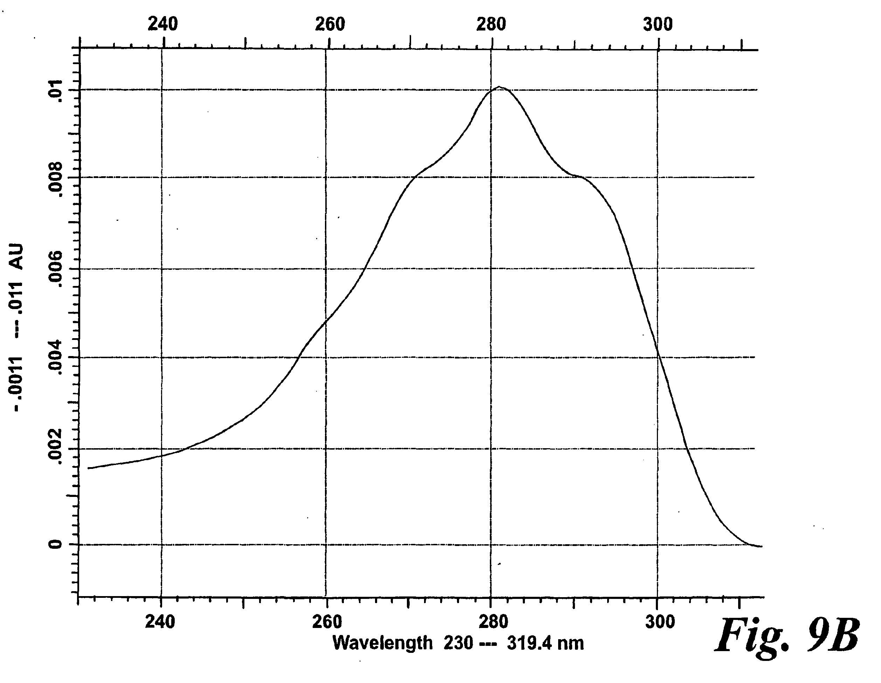

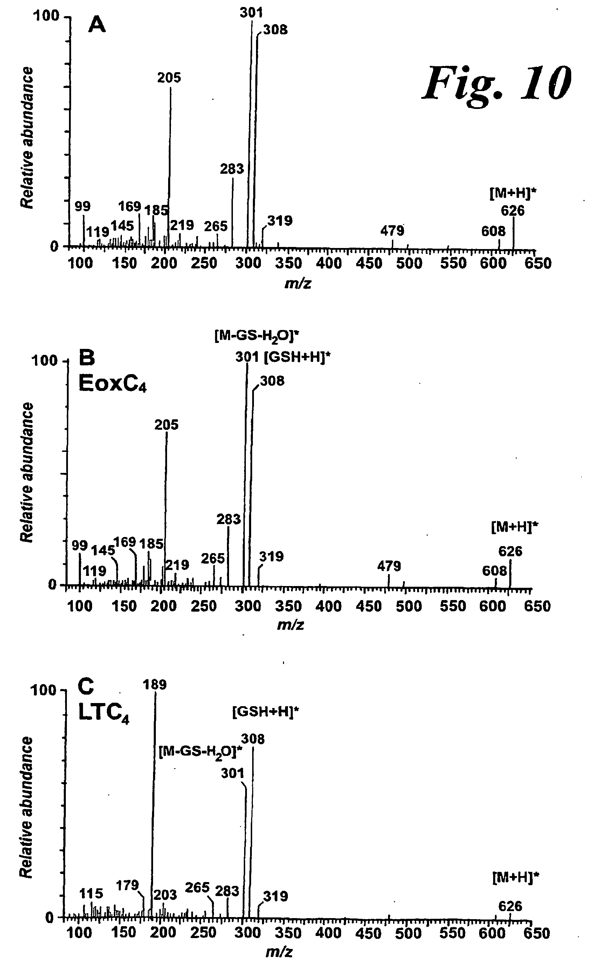

When analysed by HPLC, one peak with a conjugated triene spectrum (abs. maximum 282 nm) was observed after incubation with arachidonic acid (Figure 9a and b). The product co-chromatograped with synthetic 14 (R)-glutathionyl-15(S)= hydroxy-5, 8, 10, 12(Z, Z, E, E)- eicosatetraenoic acid (EoxC₄). To secure the identity of obtained product, tandem mass spectrometry was performed. The MS/MS spectra of the metabolite was essentially identical to that of EoxC₄ (Figure 10). In contrast to the production profile shown for the Hodgkin cell line L1236, eosinophils did not readily convert EoxC₄ to EoxD₄ and EoxE₄.

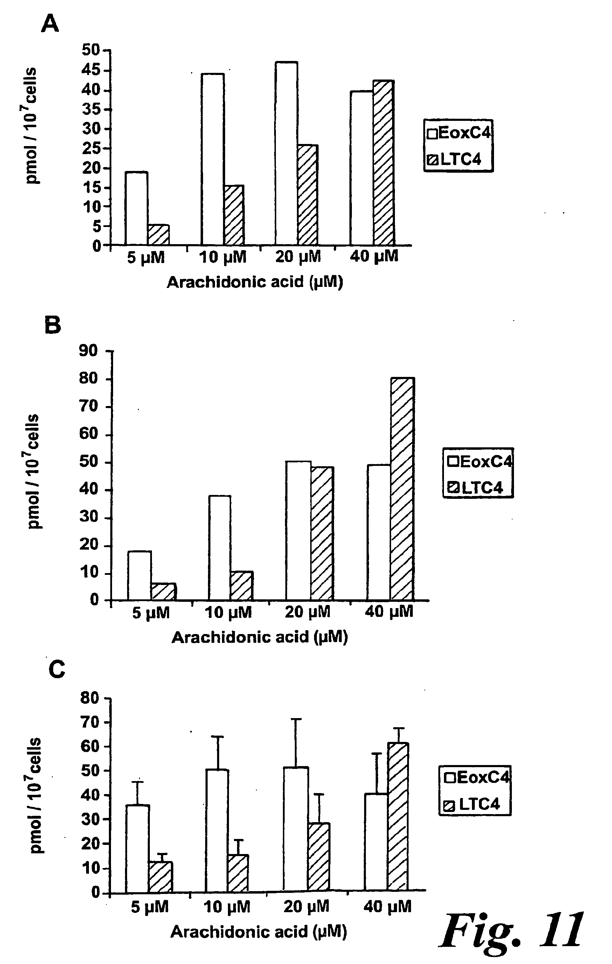

Besides expression of 15-lipoxygenase, eosinophils also express 5-lipoxygenase, which generates leukotrienes. Dose-response experiments were carried out in order to optimise the experimental conditions for generation of EoxC₄. Also the time course for formation of EoxC₄ and LTC₄ was investigated (Figure 11). However, neither Eoxin D₄ nor Eoxin E₄ was detected after 5 min incubation.

Taken together the results demonstrate that the eosinophils readily convert exogenous arachidonic acid to EoxC₄.

### Biosynthesis of Eoxin C₄ (14, 15-LTC₄) in human nasal polyp tissue

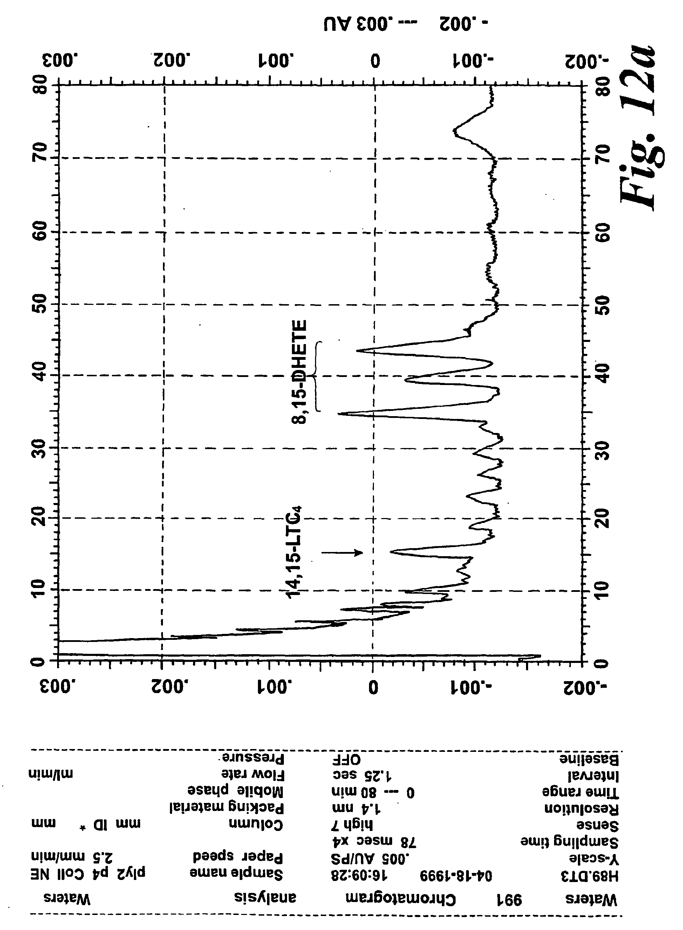

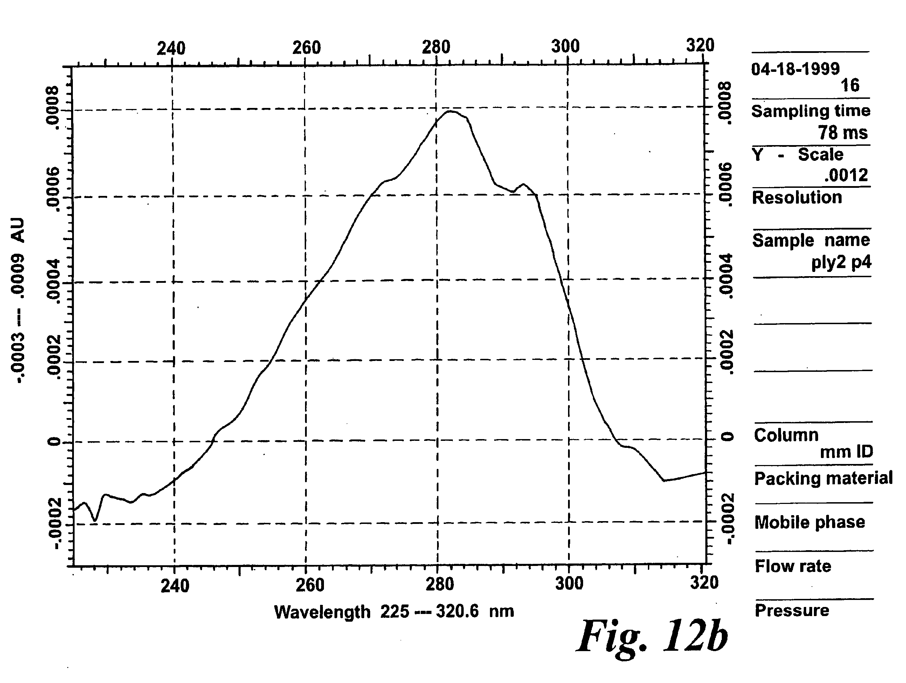

A source of airway epithelium is surgical specimens of nasal polyps. Incubation of chopped human nasal polyps with arachidonic acid (40 µM) for 5 minutes led to the formation of 14, 15-LTC₄ and a series of 8, 15-DHETE and 14, 15-DHETE metabolites (Figure 12a). The UV-spectrum of the material that co-eluted with synthetic 14, 15-LTC₄ showed the characteristic triene spectrum (Figure 12b).

### Biosynthesis ofEoxin C₄ (14, 15-LTC₄) by human dendritic cells

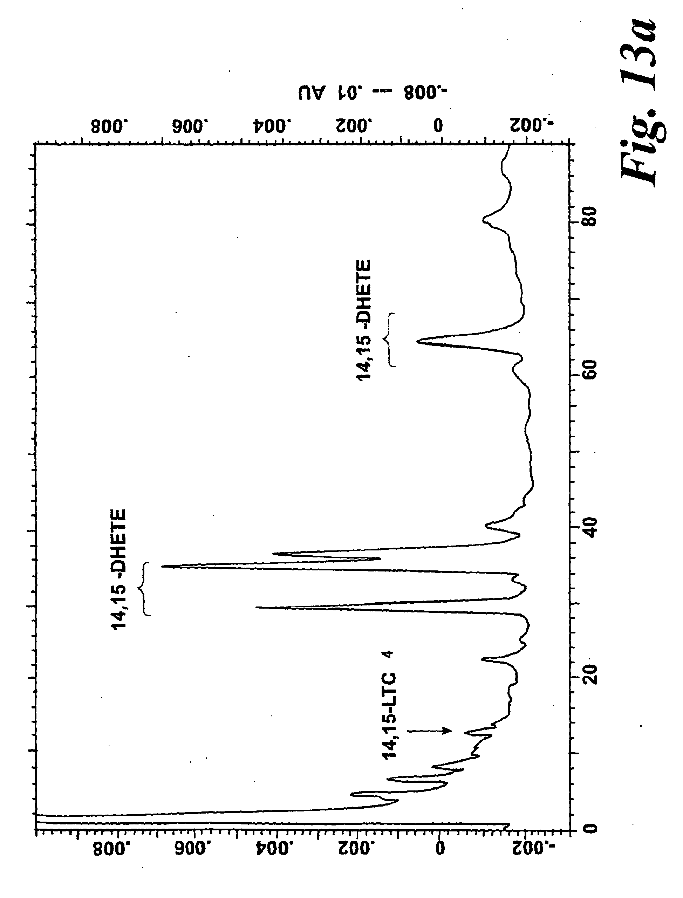

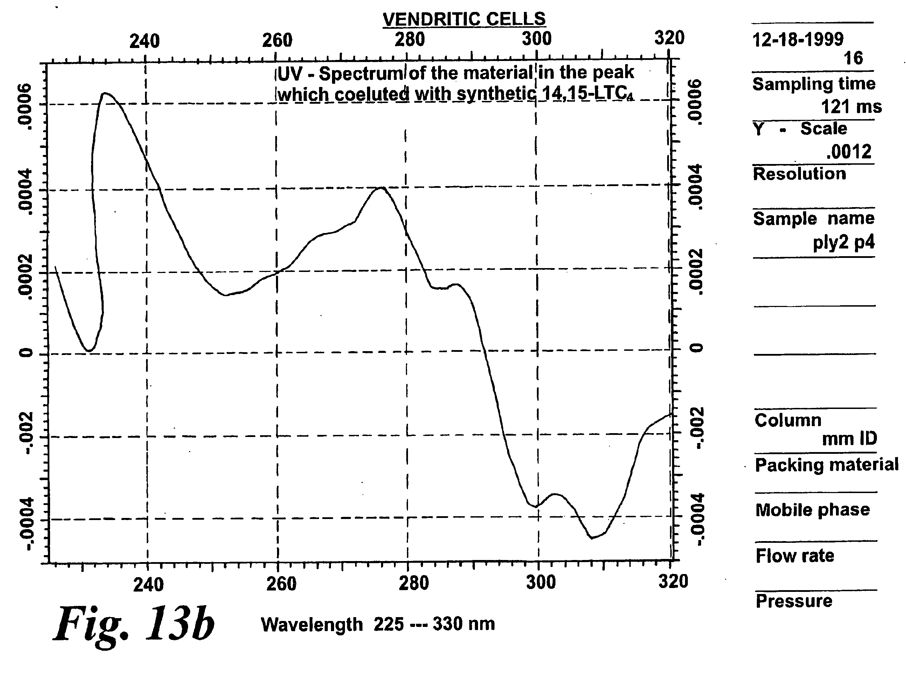

The dendritic cells were obtained by separation and cultivation of buffy coat cells (leukocytes) in the presence of growth factors and interleukin-4. The isolated cells showed characteristic function and morphology of dendritic cells. Incubation of these cells with arachidonic acid (40µM) for 5 minutes led to the formation of mainly 8, 15-DHETE and 14, 15-DHETE but also significant amounts of 14, 15-LTC₄ was formed (Figure 13a). The identification of 14, 15-LTC₄ was also based on UV-spectroscopy (Figure 13b).

### Example 3: Identification of cells expressing 15-LO

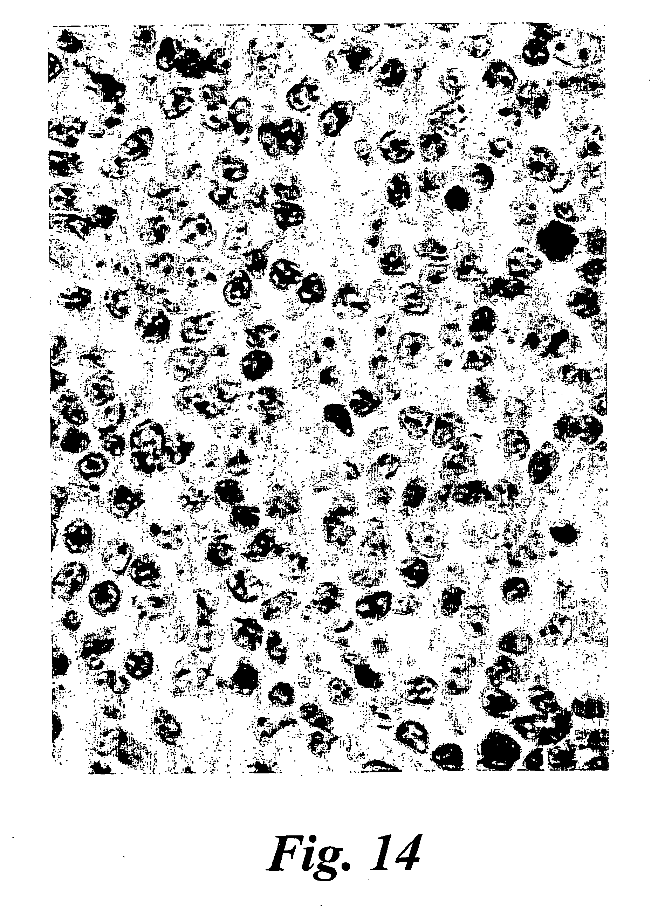

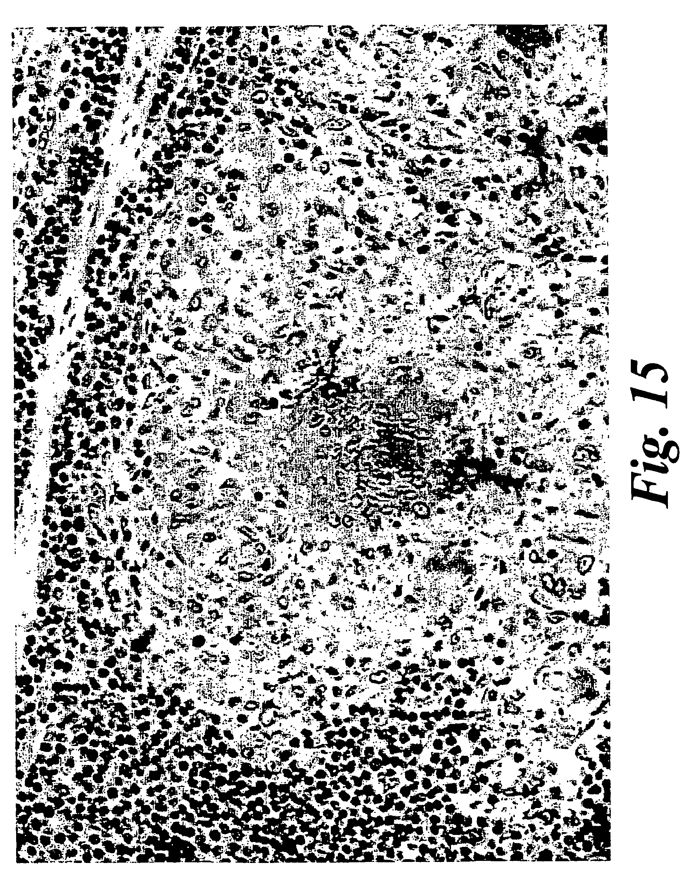

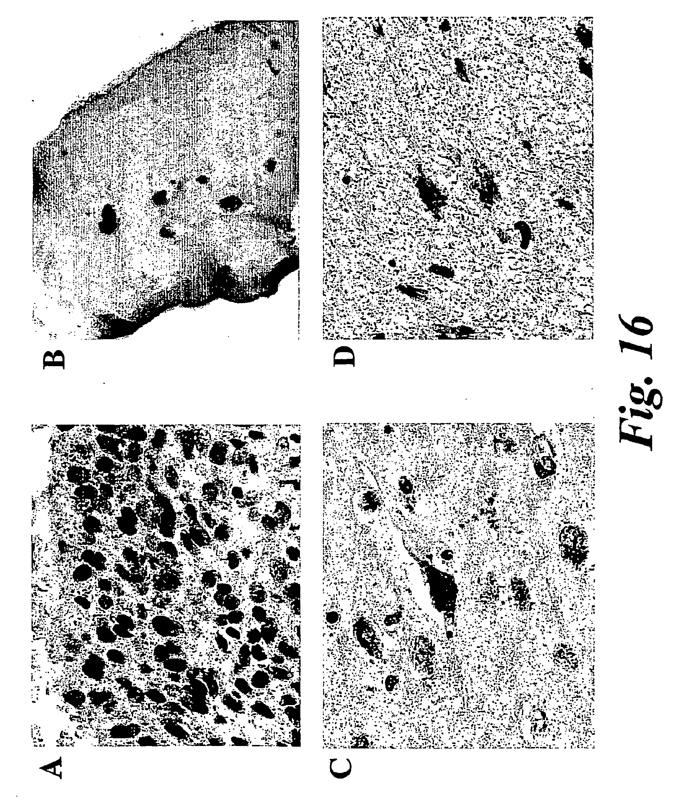

In addition to the earlier described 15-LO expression in airway respiratory epithelium, eosinophils, mast cells, and macrophages, the inventors have demonstrated 15-LO expression in several normal and pathological tissues (using antibodies raised against human 15-LO). 15-LO was found to be expressed in lymph nodes from patients with Hodgkin's lymphoma (HL) (Figure 14). In 13/15 cases of HL an expression of 15-LO was detected in Reed-Sternberg cells (Figure 14). There was no staining in any case of non-Hodgkin lymphoma. Biopsies from a patient with sarcoidosis demonstrated a strong staining of 15-LO in epitheloid cells (granulomas; Figure 15). The results suggest that all epitheloid cells express 15-LO. Epitheloid cells are found in granulomas from patients with Crohn's disease, TBC and several types of vasculitis. Other cell types, which were found to express 15-LO included synoviocytes (Figure 16a) and condrocytes (Figure 16b) from a rheumatoid arthritis patient, pyramidal cells (Figure 16c) from brain cortex of an Alzheimer patient, astrocytes (Figure 16d) from an astrocytoma specimen (but not normal tissue).

Taken together, the results indicate that 15-LO, and the metabolites formed through this pathway, are involved in the pathophysiology of these diseases.

### Example 4: Identification of the enzyme which converts Eoxin A₄ (14, 15-LTA₄) to Eoxin C₄ (14, 15-LTC₄) in L1236 cells.

### Methods

### Cell culturing

Cell culturing was performed as described in Example 1.

### Isolation of human eosinophils

Isolation of human eosinophils was performed as described in Example 2.

### RT-PCR analysis

Isolation of mRNA from eosinophils and subsequent RT-PCR analysis was performed according to standard procedures. Primers used for the PCR reaction were complementary to microsomal glutathione transferase I (MGSTI), microsomal glutathione transferase II (MGSTII), microsomal glutathione transferase III (MGSTIII) and β-actin (positive control).

### Incubation of cells

Isolated eosinophils (5-10 x 10⁶/sample) were resuspended in phosphate buffered saline (1ml/sample) and preincubated for 2 minutes in 37°C prior to stimulation with Eox A₄ (10 µM), LTA₄ (10 µM) or both for 5 minutes in 37°C. Alternatively, for inhibitor studies, eosinophils were preincubated for 2 minutes in 37°C in presence of BW70C (1 µM) or MK886 (10 µM) prior to stimulation with ionophore A23187 (1 µM) and arachidonic acid (40 µM) for 5 minutes in 37°C. The reactions were terminated by addition of three volumes of methanol. Metabolites were detected and quantified utilising Waters 996 photodiode array spectrophotometer connected to Waters 1996 HPLC system.

### Enzymatic studies

To explore the possible involvement of glutathione transferases in the conversion of EoxA₄ to EoxC₄, recombinant enzymes (1 µg) were incubated with EoxA₄ (100 µM) and glutathione (5mM) for 5 minutes in 37°C. Reactions were terminated by addition of 1 volume of stop solution (AcN: MeOH: HAc, 50:50:1 by volume) and EoxC₄ was detected and quantified utilising Waters 996 photo diode array spectrophotometer.connected to Waters 1996 HPLC system.

### Results

### Isolation, purification and characterisation of the enzyme which converts EoxA₄ to EoxC₄ in the Hodgkin cell line L1236

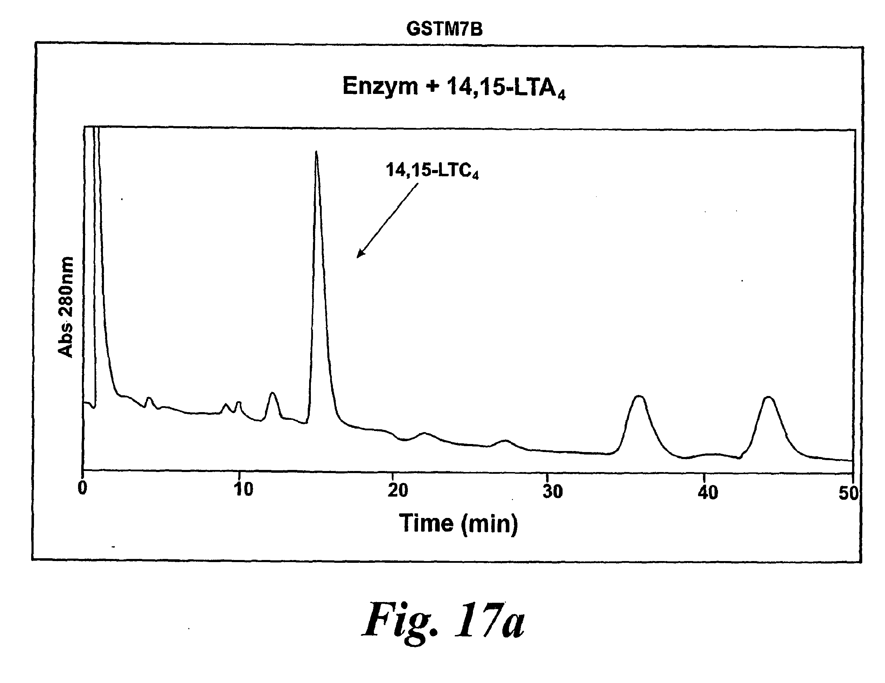

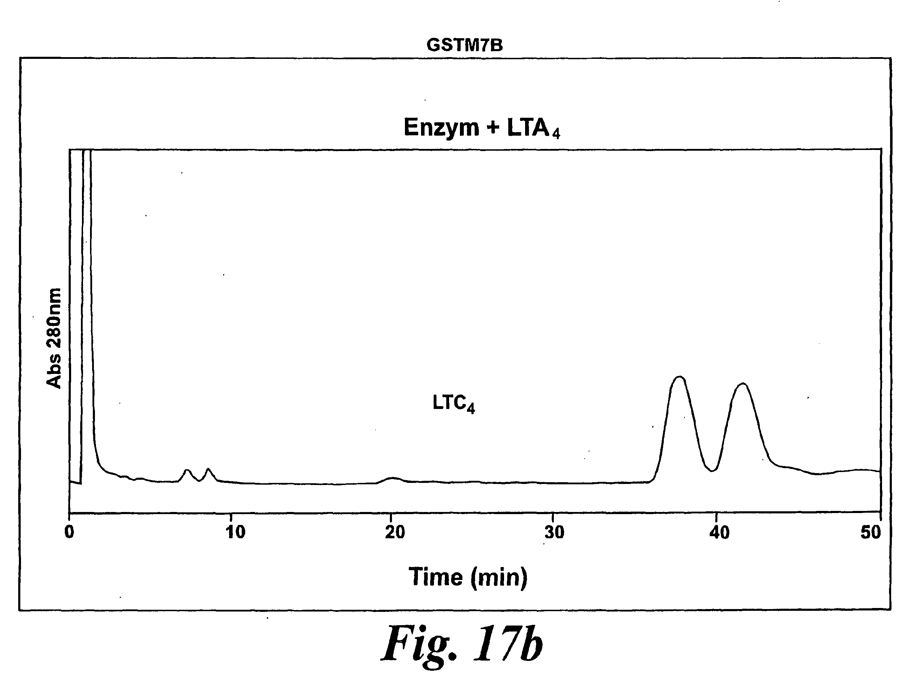

L1236 cells were subcellular fractionated according to standard procedures, and the cytosolic fraction, containing enzymatic activity, was applied to a Glutathione Sepharose column. The bound material was eluted by a glutathione buffer gradient. The eluted fractions were tested for enzymatic activity and run on a SDS-PAGE gel. One single band with an approximate Mw of 28 kD was obtained. Amino acid analysis (PAC), cDNA sequence analysis and BLAST searches revealed that the identity of the obtained band was the soluble glutathione transferase Mlb-lb (GSTM1b-1b). Recombinant GSTM1b-1b converted EoxA₄ to EoxC₄, but did not metabolise LTA₄ to LTC₄ under identical conditions (Figure 17A and B).

### Screening of the human soluble glutathione transferase family

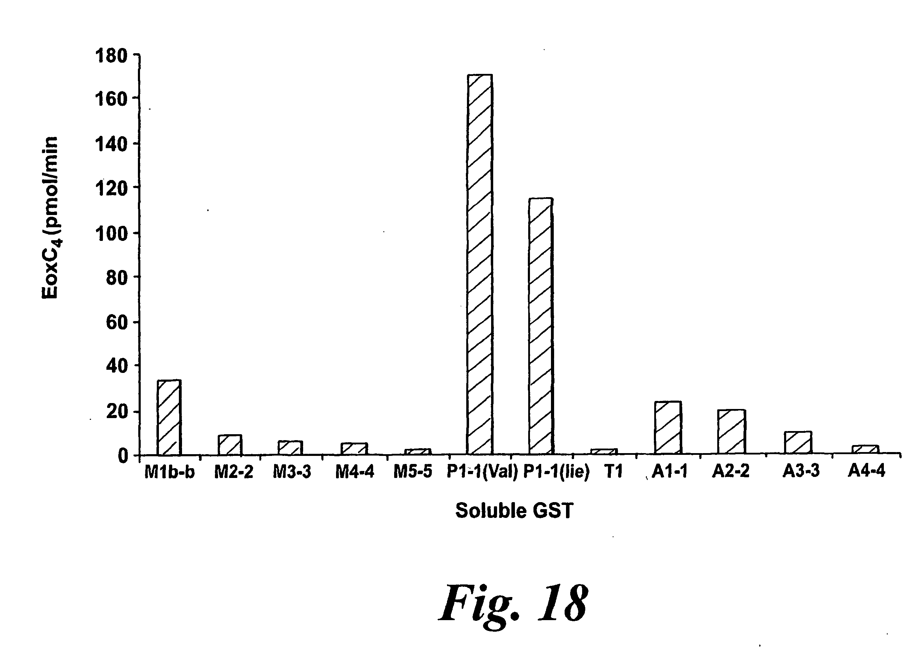

Since GSTM1b-1b is a member of the human soluble glutathione transferase family¹⁹, the capacity of soluble glutathione transferases to convert EoxA₄ to EoxC₄ was investigated. It was demonstrated that, in addition to GSTM1b-1b, also the two isoforms of GSTP1-1 (Val105 and Ile105) had a great capacity to convert EoxA₄ to EoxC₄ (Figure 18).

### Localisation of EoxC₄ synthase activity in eosinophils

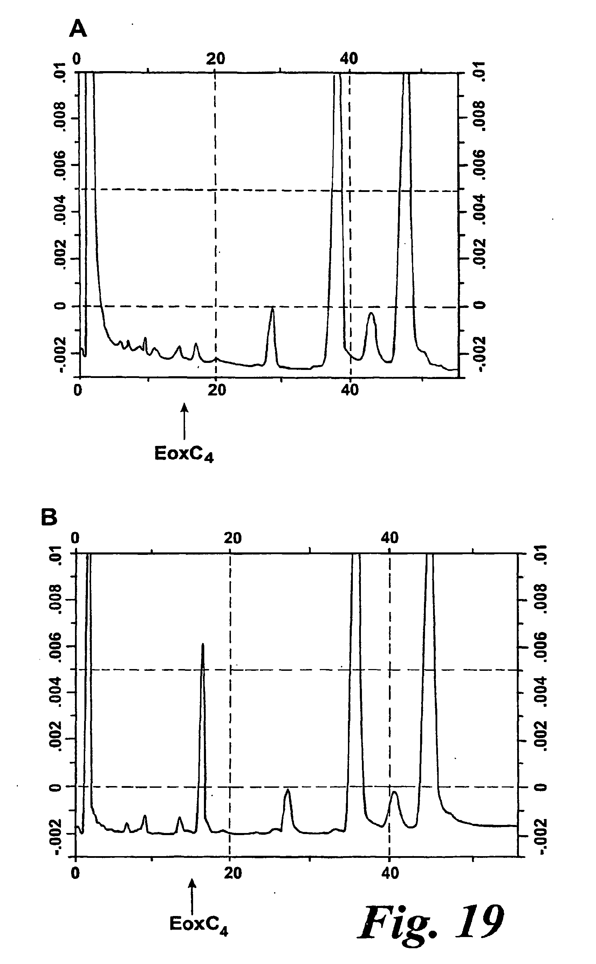

To investigate whether the soluble glutathione transferases are involved in EoxC₄ formation in eosinophils, a subcellular fractionation of isolated human eosinophils was prepared and incubated with EoxA₄. In contrast to L1236, the EoxC₄ synthase activity was almost totally located to the membrane fraction in eosinophils (Figure 19).

### Characterisation of "enzyme X" in eosinophils

Since the EoxC₄ synthase activity in eosinophils resided in the membrane fraction, the inventors investigated the involvement of membrane associated glutathione transferase in this respect.

Members of the MAPEG (Membrane Associated Proteins in Eicosanoid and Glutathione metabolism) superfamily²¹ are LTC₄ synthase, FLAP (5-lipoxygenase activating protein), PGE synthase, MGSTI (microsomal glutathione transferase I), MGSTII and MGSTBI (3). PCR analysis revealed that eosinophils posses nRNA coding for MGSTII and MGSTIII, whereas no mRNA coding for MGSTI was observed. From earlier studies it was known that eosinophils possess FLAP and LTC₄ synthase.

Incubation of the MAPEG family members with EoxA₄ and glutathione showed that only LTC₄ synthase has the capacity to convert EoxA₄ to EoxC₄ (data not shown).

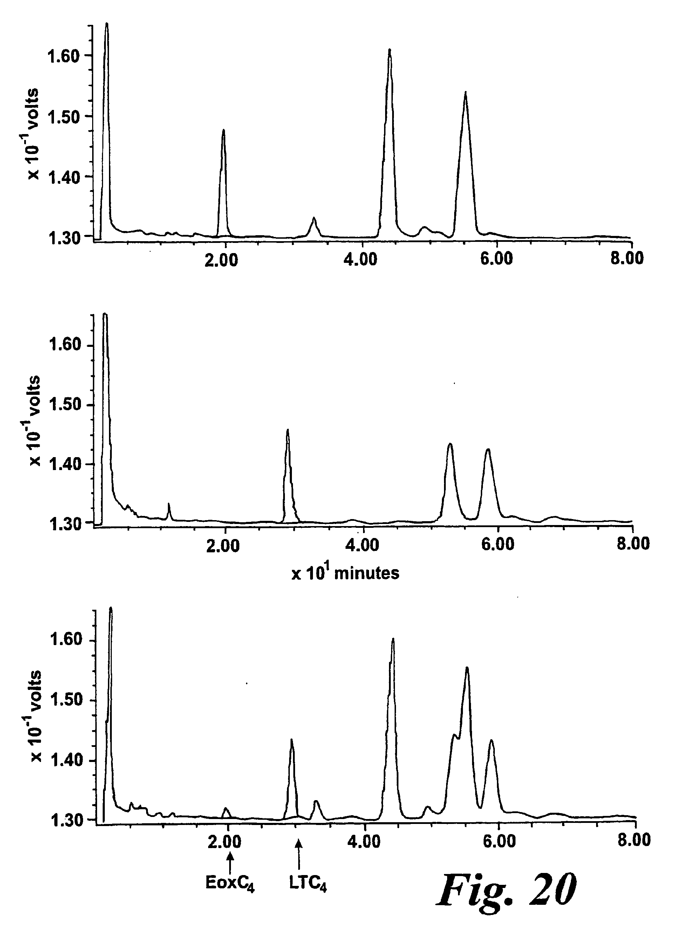

LTC₄ synthase is traditionally known to be the key enzyme in the biosynthesis of the glutathione containing leukotriene (LT)C₄ from LTA₄. LTA₄ is synthesised via the 5-lipoxygenase pathway. Eosinophils possess both 5-lipoxygenase and 15-lipoxygenase activities, and the result that LTC₄ synthase is the only MAPEG family member capable of converting EoxA₄ to EoxC₄ suggests that LTC₄ synthase could have dual activities: LTC₄ and EoxC₄ synthesis. To test the hypothesis that LTC₄ synthase is also the key enzyme in EoxC₄ formation, isolated eosinophils where incubated with EoxA₄ (10 µM), LTA₄ (10 µM), or both for 5 minutes at 37°C (Figure 20).

Incubation of eosinophils with EoxA₄ resulted in biosynthesis of EoxC₄, whereas incubation of eosinophils with LTA₄ gave rise to LTC₄. When the cells were incubated with both substrates, LTC₄ and EoxC₄ were produced in a 9:1 ratio, approximately. This is in line with previously reported affinities of LTC₄ synthase for these substrates²⁰.

To further support the findings that LTC₄ synthase also acts as EoxC₄ synthase in eosinophils, the inventors performed inhibitory studies.

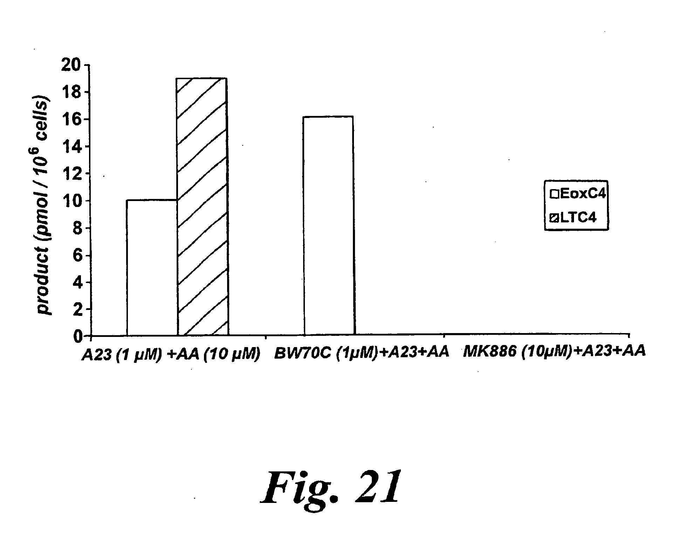

BW70C is a 5-lipoxygenase inhibitor and MK886 is a FLAP inhibitor, which also block LTC₄ synthase when used in higher (10 µM) concentrations (Figure 21). Preincubation in the presence of BW70C totally abolished the LTC₄ peak and preincubation in presence of MK886 also blocked EoxC₄ synthesis. These results further support that LTC₄ synthase is the key enzyme in EoxC₄ formation in eosinophils.

### Example 5: Assay for the detection of lipidhydroperoxides formed by human 15-lipoxygenase (15-LO)

The assay described herein takes advantage of the ability of lipidhydroperoxides to oxidize diphenyl-1-pyrenylphosphine (DPPP) to a phosphine oxide (see scheme below). Diphenyl-1-pyrenylphosphine is a non-fluorescent compound until oxidized. Oxidation of DPPP yields a fluorescent compound, which is not sensitive to temperature and is stable for at least 60 minutes.

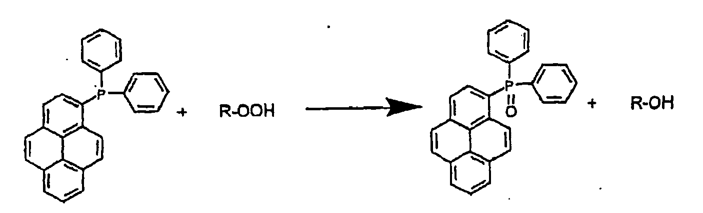

### Materials

Diphenyl-1-pyrenylphosphine (DPPP) (D-7894) is purchased from Molecular Probes. Cholic acid is purchased from Sigma, linoleic acid from Biomol and PBS from Gibco Life Technologies. Fluorescence is measured using a dual-scanning microplate spectrofluorometer, Spectramax Gemini, from Molecular Devices. The Spectramax Gemini spectrofluorometer uses a Xenon lamp and two holographic diffraction grating monochromators. Microplates with up to 384 wells can be used with the spectroflurometer. Fluorescent detection is achieved from the top of the well, through the entire depth of the sample in the well.

All reagents are provided in a ready-to-use concentration, except for the fluorophore (DPPP) and the substrate, linoleic acid. DPPP is provided as powder and it should be stored at -20°C and well protected from light. Linoleic acid is supplied as a 100 mM stock solution in ethanol. Enzyme preparations are provided as frozen aliquots.

The enzyme is expressed using baculovirus and is partially purified from Spodoptera Frugiperdia cells (Sf9) and delivered in Tris-HCl (pH 8.0), 75 mM NaCl and 5% glycerol. The purified enzyme is stable for at least up to 5 months at -70°C. Up to three freeze and thaw cycles do not seem to affect the enzyme activity. However, freeze and thaw cycles of the enzyme should be avoided, if not absolutely necessary. The enzyme is thawed and diluted in PBS just prior to the experiment. Diluted enzyme is to be used within the same day. DPPP (powder) is dissolved in ethanol and 2-butanol (1:1 ratio) to a final concentration of 0.25 mM. This solution is mixed by vortexing and subsequently sonicated in an ultrasonic waterbath at +4°C for 10 seconds to completely dissolve DPPP. This solution should be made fresh on the same day as the experiment is performed. The solution should be shaded from light.

### Diphenyl-1-pyrenylphosphine method for lipihydroperoxide detection

The assay is performed in 96-well all-back plates at room temperature (20-22°C). each well contains 45 µl consisting of Dulbecco's phosphate buffered saline (PBS) supplemented with 500 µM cholic acid and 15-LO enzyme (pre-mixed). Inhibitor (1 µl) or vehicle (DMSO) is added 5-15 minutes prior to the addition of substrate, 5 µl 1 mM linoleic acid. The plate is then incubated for 20 minutes at room temperature. The enzymatic reaction is terminated by the addition of 50 µl methanol. The plate can now be stored at room temperature for up to 60 minutes without significant loss of lipidhydroperoxides. 50 µl of 25 µm DPPP-solution is added and the plate is incubated at room temperature. After 30-60 minutes, the fluorescence can be read using an excitation wavelength of 355 nm and an emission wavelength of 380 nm.

Due to a low auto-oxidation of DPPP in aqueous solutions, one of the blanks should include buffer, linoleic acid, methanol and DPPP in the same ratios as for the enzymatic reactions.

### Example 6: Demonstration of the biological effects of Eoxin C₄, Eoxin D₄ and Eoxin E₄ (14,15-LTC₄,14-15-LTD₄, 14-15-LTE₄)

### Example 6a: Assessment of endothelial cell barrier function

### Materials and methods

### Endothelial cell assay

Bovine aorta endothelial cells (BAEC) and human umbilical vein endothelial cells (HUVEC) were isolated and cultured with calf serum or human serum. BAEC or HUVEC in the first to fifth passages were detached by brief (2 min) trypsin-EDTA treatment (0.25% trypsin/0.01% EDTA) and replated onto 3.0 µm pore size polycarbonate filters (Tissue Culture inserts, 10 mm; NUNC, Roskilde, Denmark) or, for the purpose of visual inspection, 0.2 µm pore size Anopore inorganic membrane (transparent when wet). In order to promote cellular differentiation and enhance attachment of EC, the filters were pretreated with 50 µl Biomatrix I (167 µg/ml) and air-dried. EC were seeded at a density of 2 x 10⁵ cells per filter and incubated in culture medium at 37°C in a humidified atmosphere of 5% CO₂ in air. The EC were grown to confluent monolayers, as assessed by daily microscopic observation, in transparent 0.2 µm pore size filters and measurement of the electrical resistance across the monolayer. Under these experimental conditions maximal resistance is obtained at day 7 postseeding, at which day the EC monolayers were then used in the experiments.

### Measurement ofEC barrier function

Rapid changes in endothelial cell (EC) barrier function in response to various arachidonic acid metabolites were analysed through continuous registration of transendothelial electrical resistance (TEER) with electrodes on each side of EC monolayer¹⁸. For measurement of TEER, the filter insert with ECs was transferred to a resistance measurement chamber (Endohm-12, World Precision Instruments, Sarasota, FL, USA) modified so as to assure exact positioning of the electrodes in relation to each other and to the filter insert during repeated measurements. The chamber (low compartment) and the filter insert (upper compartment) were filled with 2 ml and 400 µl culture medium, respectively. All measurements were made at 37°C with the equipment placed in a cell culture incubator. Direct readings of the electrical resistance were given by an ohmmeter (Evom, World Precision Instruments, Sarasota, FL, US) connected to the electrodes of the resistance chamber. The electrical resistance of individual EC monolayers was obtained by subtracting the resistance of the corresponding naked filter coated with Biomatrix as measured prior to seeding of the EC. Thus, TEER values represent the resistance of the EC monolayer solely and depend on the development of tight junctions between adjoining endothelial cells.

### Results

### Biological effects of 14,15-cysteinyl-contauting leukotrienes (Eoxins)

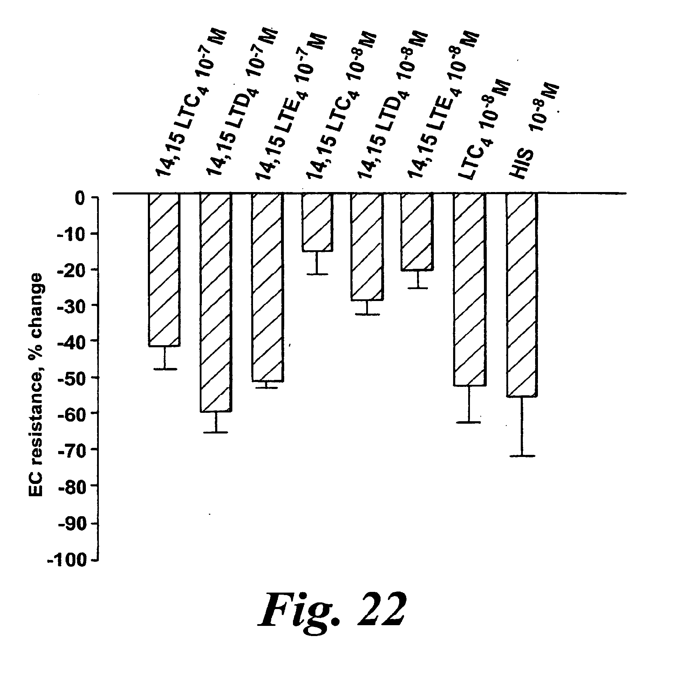

Increase of vascular permeability is a marker of inflammation and it is well known that one of the biological effects of cysteinyl-containing leukotrienes is to cause specific plasma leakage in the same way as histamine and bradykinin. In contrast to the 5-lipoxygenase derived cysteinyl-containing leukotrienes, EoxC₄ and its metabolites appear not to have contractile effects on guinea pig pulmonary parenchymal strip or ileum. To explore the ability of EoxC₄ and its metabolites EoxD₄ and EoxE₄ to cause plasma leakage, a model system for registration of rapid changes in endothelial cell barrier function was set up. The inventors found that all three 14,15-cysteinyl-containing leukotrienes increased vascular permeability. Concentrations of 10⁻⁷ and 10⁻⁸ M of the compounds induced significant change in TEER. In comparison, EoxC₄, D₄ and E₄ were about five to ten times less potent than the most potent 5-lipoxygenase derived leukotrienes but more than a hundred times as potent as histamine in this respect (Figure 22).

### Example 6b : Effects of Eoxins on the oxidative metabolism of leukocytes.

The oxidative metabolism by the neutrophil and eosinophil can be induced by various agents, e.g. phorbol myristate acetate (PMA), platelet-activating factor (PAF), serum treated zymosan (STZ), immunoglobulins or leukotrienes (LTB₄), but also by cytokines, e.g. IL-5 and GM-CSF. Priming refers to a process that leads to an increased responsiveness of the cell to stimulation, without activating the cell. The mechanisms leading to the state of priming of the cells are still unclear, although the hemopoietic cytokines IL-3, IL-5 and GM-CSF have been shown to prime the eosinophil and neutrophil *in vitro*. However, only IL-5 has been shown to be a selective activator of the human blood eosinophils, while IL-3 and GM-CSF can act on both eosinophils and neutrophils

### Materials and methods

Neutrophil and eosinophil granulocytes were purified from peripheral whole blood using a Percoll gradient and the magnetic cell separation system (MACS). Granulocytes were primed for 30 minutes at 37°C with either IL-5 (10.5 ng/mL) or GM-CSF (10.5 ng/mL) and subsequently stimulated with IL-5 (100 ng/mL), GM-CSF (100 ng/mL), LTB₄ (10⁻⁷M) or Eoxin (10⁻⁸ - 10⁻⁶M). Oxidative metabolism was measured by a lucigenin-enhanced chemiluminescence (CL) assay.

The test compound used were EoxC₄ (BLX-925), EoxD₄ (BLX- 926), EoxE₄ (BLX-927).

### Results

### Eoxin-induced oxidative metabolism by non-primed neutrophils

The purified neutrophils were stimulated with two known activators of the oxidative metabolism; the cytokine GM-CSF and LTB₄, for comparison with the stimulatory effect of the different Eoxins on neutrophils. Both activators induced the oxygen free radical production by the non-primed neutrophils, while none of the Eoxins induced the oxygen free radical production by non-primed blood neutrophils.

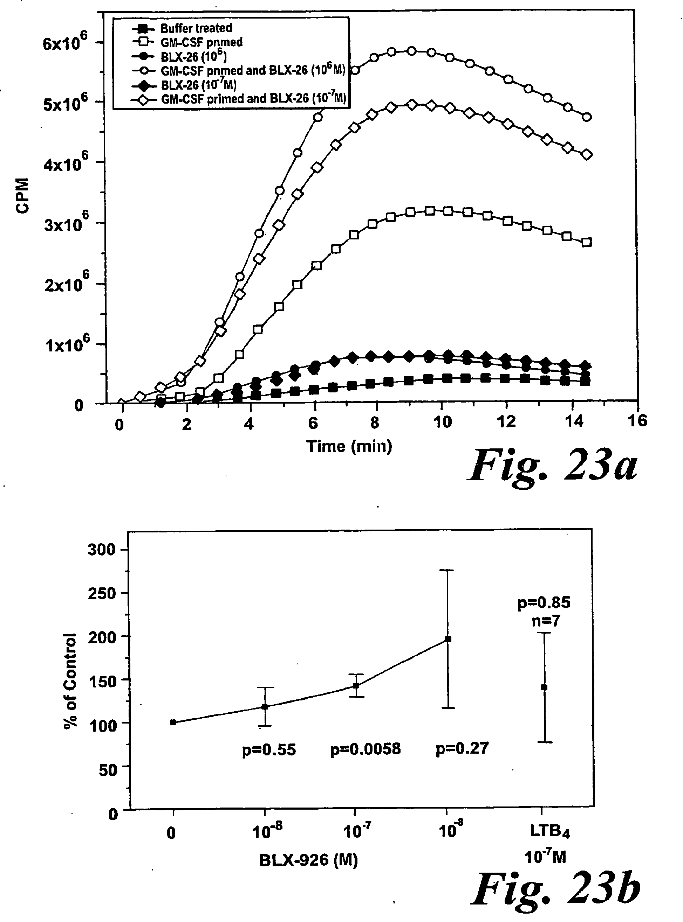

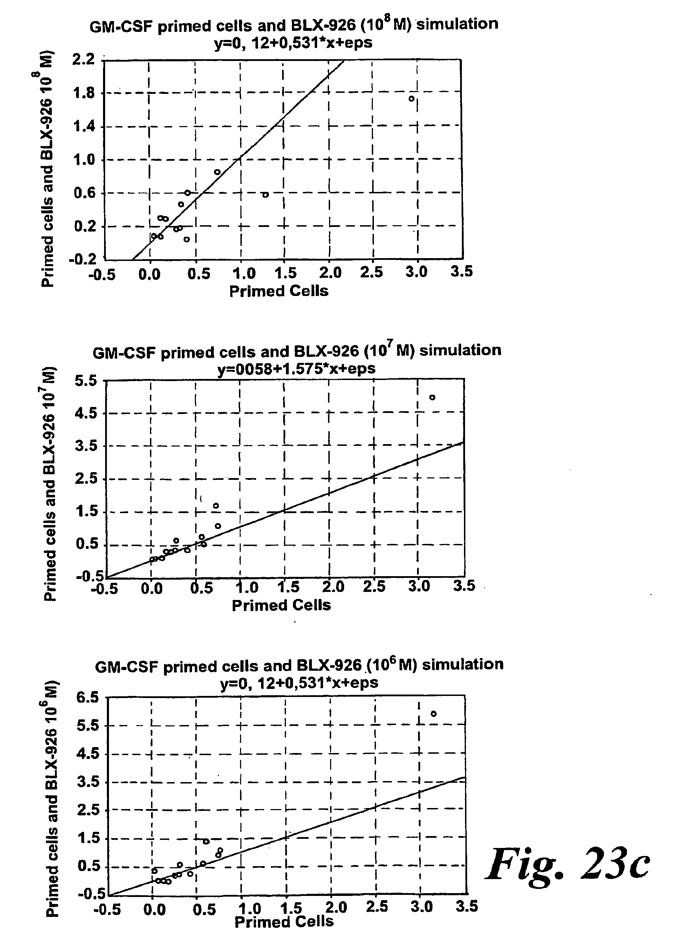

The kinetic curves (Figure 23a), illustrating one representative experiment, showed an enhanced radical production after stimulation of the cytokine-primed cells as compared to the non-primed cells. This enhanced radical production showed a dose-response pattern as seen in Figure 23b, presenting all results of the different donors as mean-values. Since we experienced a strong individual dose-responsiveness to EoxD₄ (BLX-926) between the different blood donors, all individual data were plotted and illustrated in a Scatter plot (Figure 23c). By doing this, it became clear that EoxD₄ (BLX-926) induced the radical production by primed cells in a dose-dependent manner, with different optimal doses for the different donors. 7-10 subjects of 13 were responders to EoxD₄ (BLX-926).

A similar pattern was also seen for EoxC₄ (BLX-925). This compound induced the radical production by GM-CSF primed neutrophils in a dose-dependent manner (7-8 subjects of 13 were responders to EoxC₄; data not shown).

### Eoxin induced oxidative Metabolism by non primed eosinophils

All three Eoxins induced a small production of oxygen free radicals by non-primed eosinophils. However, the produced amount of oxygen free radicals is rather low as compared to other known activators of the oxidative metabolism by eosinophils, in this case the eosinophil specific cytokine IL-5 (Figure 24a).

### Eoxin induced oxidative metabolism by IL-5 primed eosinophils

EoxE₄ (named BLX-27 in the showed a broad dose-related induction of the radical production by the IL-5 primed eosinophils, illustrated in Figure 24, indicating that this Eoxin might be the best inducer of the oxidative metabolism by the eosinophil among those tested. 3 of 6 subjects were responders to EoxE₄.

### Conclusion

EoxC₄ (BLX-925) and EoxD₄ (BLX-926) induced mainly the oxygen free radical production by GM-CSF primed neutrophils, while EoxE₄ (BLX-927) induced mainly the oxygen free radical production by IL-5 primed eosinophils.

### Example 7: Effect of 15-LO inhibition on the formation of Eoxin C₄

The Hodgkin cell line L1236 was stimulated for 5 min with arachidonic acid (40 µM). L1236 cells efficiently converted arachidonic acid to EoxC₄, EoxD₄ and a series of 8,15-DHETE and 14,15-DHETE metabolites.

### Materials and Methods

### Cell Incubation

The Hodgkin cell line L1236 (10 x 10⁶ cells/sample) was suspended in 1 ml PBS-buffer and pre-warmed in 2 minutes at 37 °C prior to addition of test substance (1, 10 or 50 µM). After pre-incubation with the test substance for 5 minutes, arachidonic acid (40 µM) was added. Incubation was stopped after 2 minutes with addition of one volume of methanol. Each test substance concentration was incubated in duplicate.

### Solid Phase Extraction

Cells and cell debris were removed by centrifugation (2600 rpm, 6 min) from the samples. The supernatant was diluted with water and then transferred to a washed and equilibrated extraction cartridge, Oasis HLB 1 cc 10 mg (Waters, Sweden). The metabolites were eluted with methanol and diluted with water before injected to the HPLC system.

### HPLC analysis

RP-HPLC was performed on a Nova Pak C₁₈ column (2.1 x 150 mm, Waters AB, Sweden). The initial mobile phase consists of 100 % A (0.05 % (8.7 mM) acetic acid, with pH adjusted to 5.6 with ammonium) at a flow rate at 0.4 ml/min. A linear gradient was started after five minutes to 36 % B (60:40 acetonitrile: methanol) at 20 minutes. The mobile phase was than held isocratic at 64:36, A:B for another 20 minutes. Column effluent was monitored using diode array detection (PDA 996, Waters AB, Sweden) and UV spectra were acquired between 200-340 nm.

### Test compounds

Figure 25 shows the structure of various compounds that inhibited the formation of EoxC₄. Some are described in WO 2004/080999. 1-Phenylthiocarbamoyl-1*H-*pyrazole-3-carboxylic acid (2-chlorophenyl)-amide (Compound A) is described in Example 47. Pyrazole-1,3-dicarboxylic acid 1-(benzo[1,3]dioxol-5-ylamide) 3-(2-chloro-4-fluoropbenylamide (Compound B) is described in Example 64. 6-{[3-(2-Chloro-4-fluorophenylcarbamoyl)pyrazole-l-carbonyl]-amino} -hexanoic acid ethyl ester (Compound C) is described in Example 65. Pyrazole-1,3-dicarboxylic acid 3-(2-chloro-4-fluorophenylamide) 1-pentyl-amide (Compound D) is described in Example 66. Pyrazole-1,3-dicarboxylic acid 3-(2-chloro-4-fluorophenylamide) 1-batylamide (Compound E) is prepared analogously to methods described in WO 2004/080999 (see in particular Example 66, where butyl isocyanate is employed instead of pentyl isocyanate).

*N*'-(2-(Ethoxyimino)-1-phenylethylidene)benzohydrazide (Compound F) may be prepared as follows:

### (a) Benzoic acid (2-hydroxyimino-1-phenylethylidene)hydrazide

Benzoic hydrazide (1 to 1.2 equivalents) was dissolved in an alcoholic solvent (e.g. EtOH or MeOH). Two drops of acid (e.g.. concentrated sulfuric acid or glacial acetic acid) were added, followed by the dropwise addition of 2-isonitrosoacetophenone (1 equivalent). The reaction mixture was stirred (e.g. at elevated temperature (e.g: 50 - 85°C)), before collecting the solid formed by filtration and, optionally, recrystallising the crude material (e.g. from EtOH/water) to provide the sub-title compound.

### (b) N'-(2-(Ethoxyimino)-1-phenylethylidene)benzohydrazide

To a solution of benzoic acid (2-hydroxyimino-1-phenylethylidene)hydrazide (1 equivalent; see step (a) above) and KOH (1 to 1.2 equivalents) in dimethylsulfoxide was added ethyl bromide (at least 1 equivalent). The reaction was stirred at elevated temperature (e.g. 45°C) for a period of time (e.g. 6 days). Water was added and the mixture was extracted with dichloromethane. The combined organic phase was washed with NaOH (2 M) and water. The organic phase was dried (Na₂SO₄), evaporated *in vacuo* and, optionally, recrystallised (e.g. from EtOH/water) to provide the title compound
3-Chloro-*N*'-(2-(hydroxyimino)-1-(3-chlorophenyl)ethylidene)benzohydrazide (Compound G) may be prepared as follows:

### (a) 2-(3-Chlorophenyl)-2-oxoacetaldehyde oxime

Isoamyl nitrite (1.83 g, 15.6 mmol) was dissolved in ice-cold ethanolic sodium ethoxide (0.9 M, 18.3 mL, 16.5 mmol). 3-Chloroacetophenone (14.9 mmol) was added dropwise to the cooled solution, the reaction was allowed to reach room temperature and stirring was continued for 16 h. The solid formed during the course of the reaction was filtered off, washed with diethyl ether, dissolved in water and the aqueous solution acidified with glacial acetic acid. The resultant crystalline solid was filtered off and recrystallised from EtOH/water to provide the sub-title compound.

### (b) 3-Chloro-N'-(2-(hydroxyimino)-1-(3-chlorophenyl)ethylidene)benzohydrazide

3-Chlorobenzoic hydrazide (1 to 1.2 equivalents) was dissolved in EtOH and to it was added 2 drops of concentrated sulfuric acid and 2-(3-chlorophenyl)-2-oxoacetaldehyde oxime (1 equivalent; see step (a) above). The reaction was heated at 50°C overnight, filtered, washed with EtOH and recrystallised from EtOH/water to provide the title compound.

### Results

### Inhibition of EoxC₄ formation in the human cell line L1236

Eicosatetraynoic acid (ETYA) at a concentration of 10⁻⁴-4 M, completely blocked the formation of EoxC₄, EoxD₄ (Figure 26) and other 15-LO derived products. The formation of these products was also decreased in the presence of 10⁻⁵-5 M ETYA. These results show that the lipoxygenase inhibitor ETYA can block the formation of EoxC₄ and its metabolites.

### The effects of the compounds on the formation of EoxC₄ and 15-HETE

L1236 cells were pre-incubated with the indicated substance (1µM) for two min and subsequently incubated with arachidonic acid (12.5µM) for five min. Incubation and extraction was performed as described above.

All tested compounds (as described in Figure 25) inhibited the formation of EoxC₄ (Figure 27). Also, the inhibitors blocked the synthesis of EoxC₄ and 15-HETE to a similar degree (Figure 28).

### Example 8: Identification of proinflammatory arachidonic acid metabolites produced via the 15-lipoogenase-1 pathway in human eosinophils and mast cells

**Abbreviations:**

| | |
|---|---|
| CBMC | coord blood mast cells |
| EC | endothelial cells |
| EIA | enzyme immuno assay |
| HETE | hydroxyl eicosatetraenoic acid |
| HPLC | high pressure liquid chromatography |
| IL | interleukin |
| LO | lipoxygenase |
| LT | leukotriene |
| MS | mass spectrometry |
| PG | prostaglandin |

Human eosinophils contain abundant amounts of 15-lipoxygenase-1 (15-LO-1). The biological role, however, of 15-LO-1 in human is unclear. Incubation of eosinophils with arachidonic acid for five minutes led to formation of a major product with UV absorbance maximum at 282 nm and shorter retention time on RP-HPLC than leukotriene (LT) C₄. Analysis with positive ion electrospray tandem mass spectrometry identified the unknown eosinophil metabolite as 14,15-LTC₄. This compound could be metabolized to 14,15-LTD and 14,15-LTE₄ in eosinophils. Also cord blood derived mast cells and surgically removed nasal polyps produced 14,15-LTC₄. Incubation of eosinophils with arachidonic acid favoured the production of 14,15-leukotriene C₄ over LTC₄, whereas challenge with calcium ionophore led to exclusively formation of LTC₄. 14,15-cysteinyl-containing leukotrienes induced increased permeability of endothelial cell (EC) monolayer in vitro indicating that these metabolites can induce increased vascular permeability, a hallmark of inflammation. In this model system, 14,15-leukotrienes were almost as potent as LTC₄ and LTD₄ but 100 times more potent than histamine. Eosinophils produced 14,15-LTC₄ after challenge with LTC₄, prostaglandin D₂ or interleukin-5, demonstrating that 14,15-LTC₄ can be synthesized from the endogenous pool of arachidonic acid. Taken together, the present report describes the formation of proinflammatory 14,15-leukotrienes particularly in human eosinophils but also in human mast cells and nasal polyps. Since eosinophils are such an abundant source of these newly characterized metabolites, and to avoid confusion with 5-lipoxygenase derived leukotrienes, we suggest the name Eoxins C₄, D₄ and E₄ instead of 14,15-leukotriene C₄, D₄ and E₄, respectively.

### Introduction

The mammalian lipoxygenases belong to a family of structurally related lipid peroxidizing enzymes that are implicated in the pathogenesis of asthma and other inflammatory disorders. The 5-lipoxygenase (5-LO) is the key enzyme in leukotriene synthesis and this enzyme catalyzes the conversion of arachidonic acid to leukotriene (LT) A₄. This compound can be hydrolyzed enzymatically to LTB₄ or conjugated with glutathione to form LTC₄ which in turn can be metabolized to LTD₄ and LTE₄. Leukotriene C₄ and its metabolites are proinflammatory agents and potent bronchoconstrictors (1). In contrast to 5-LO, much less is known about the biological function of human 15-lipoxygenase (15-LO-1). Human airway epithelial cells, eosinophils and subsets of mast cells and dendritic cells constitutively express high amounts of 15-LO-1 (2-6) . A second form of the human 15-LO also exist, which is named 15-LO-2 (7). The amino acid sequence similarity between this enzyme and 15-LO-1 is only 40 %(3). Interleukin (IL)-4 and/or IL-13 induce the expression of 15-LO-1 in cultivated epithelial cells, monocytes and mast cells (4, 8, 9) and thus these cytokines may act as important physiological/pathophysiological regulators of 15-LO-1 expression in vivo. 15-lipoxygenase-1 has been suggested to be involved in the pathogenesis of bronchial asthma. The amount of 15-LO-1 is significantly increased in the bronchial submucosa of patients with asthma or chronic bronchitis compared to control subjects (10, 11). 15-lipoxygenase-1 converts arachidonic acid to 15-hydroxy-eicosatetraenoic acid (15-HETE), which has been used as a marker for 15-LO activity in vivo as well as in various cells and tissues in vitro. Human tracheal epithelial cells, chopped human bronchi and human lung homogenate convert arachidonic acid mainly to 15-HETE. Lung specimens from asthmatic subjects produced more 15-HETE than specimens from non-asthmatic subjects (12-14) and 15-HETE was the predominant arachidonic acid metabolite in stimulated human lung tissue from atopic and non-atopic asthmatic subjects (15). Increased amounts of 15-HETE were found in bronchoalveolar lavage fluid (BALF) of antigen challenged atopic asthmatic patients (16), in sputum samples from asthmatic subjects (17) and in nasal secretions from healthy control subjects (18). Severe asthmatic patients with persistent airway eosinophilia have higher levels of 15-HETE in BALF compared to healthy subjects, or patients with asthma but without eosinophilia (14). The levels of 15-HETE was found to correlate with sub-basement thickness indicating that 15-LO might be associated with airway remodelling (14).

The most abundant arachidonic acid metabolite generated via the 15-LO pathway is 15(S)-HETE) which is formed from 15(S)-hydroperoxy-eicosatetraenoic acid (15(S)-HPETE). In addition to undergoing reduction to 15-HETE, 15-HPETE may also undergo dehydration to form 14,15-epoxy-eicosatetraenoic acid (14,15-LTA₄) (19, 20). Another 15-lipoxygenase product, 5'-oxo-15-hydroxy-6,8,11,13-eicosatetraenoic acid, has been reported to be a potent chemotactic agent for human eosinophils (21). However, there is also evidence that 15-LO products could have protective roles in inflammatory disorders since 15-LO-1 can be involved in the formation of lipoxins and resolvins (22). Taken together, the physiological and pathophysiological role of the 15-LO pathway in human is not clear.

In this report, we describe the formation and biological effects of 14,15-leukotriene C₄, D₄ and E₄, indicating a proinflammatory role for 15-LO-1 in humans. Since eosinophils are such an abundant source of these newly characterized metabolites, and to avoid confusion with 5-lipoxygenase derived leukotrienes, we suggest the name eoxin C₄, D₄ and E₄ for these novel metabolites instead of 14,15-leukotriene C₄, D₄ and E₄, respectively.

### Results

### Formation of novel 15-lipoxygenase products in isolated human eosinophils

Incubation of isolated human eosinophils with arachidonic acid for five minutes led to the formation of 15 (S)-HETE and 12(S)-HETE in a ratio of approximately 9:1 (data not shown), which is in agreement with the activity of 15-LO-1 (2). A number of peaks with UV absorbance maximum around 270 nm and retention times between 5-7 min were observed in the same chromatogram. In order to get better separation of these more polar metabolites, an acetonitrile based mobile HPLC phase was used. Five peaks containing a conjugated triene spectrum and UV absorbance maximum at 272 nm were identified (Fig. 29). The retention times of the material in these five peaks corresponded to the retention times for the two double oxygenation products 8(S),15(S)-DHETE and 8(R),15(S)-DHETE, the two 8(S),15(S)-LTB₄ and 8(R),15(S)-LTB₄ ,derived from 14,15-oxido-leukotriene A4 (19, 20) and 14(R),15(S)-DHETE. The pattern of products formed was similar to what has been reported for human airway epithelial cells incubated with arachidonic acid (5). Small amounts of leukotriene (LT) C₄, with the characteristic UV spectrum of a conjugated triene but with maximum at 282 nm, was also observed. In addition to these well known arachidonic acid derived metabolites, a more polar major metabolite (Metabolite 1) was observed in the chromatogram, possessing a conjugated triene spectrum, with UV absorbance maximum at 282 nm (insert, fig.29).

### Identification of Metabolite 1 by LC-MS/MS

To further analyse the structure of Metabolite 1, samples of human eosinophils incubated with exogenous arachidonic acid were subjected to analyses by LC-MS/MS using a positive ion electrospray tandem mass spectrometry. The obtained ion scan spectrum contained several fragments also present in the spectrum of LTC₄ (Fig 30 A). This finding together with the known high 15-LO activity in eosinophils, indicated that Metabolite 1 could be 14(R)-glutathionyl-15(S)-hydroxy-5,8,10,12(Z,Z,E,E)-eicosatetraenoic acid (14,15-LTC₄). To confirm this hypothesis, synthetic 14,15-LTC₄ was prepared (Biomol Inc, USA) and the collision induced dissociation spectrum obtained for Metabolite 1 was compared with the spectrum obtained for the synthetic 14,15-LTC₄ (fig. 30A). The sample was analyzed with a product ion scan at 626.2 m/z, equivalent to the protonated 14,15-LTC4_{.} The chromatogram contains a peak at the same retention time as standard 14,15-LTC₄, and when comparing the mass spectra it is identical to synthetic 14,15-LTC₄.

### Metabolism of 14,15-LTC4 in human eosinophils

In order to analyze if eosinophils can metabolize 14,15-LTC₄ to 14,15-LTD₄ and 14,15-LTE₄, eosinophils were incubated with synthetic 14,15-LTC₄ or 14 (R)-cysteinyl-glycyl-15(S)-hydroxy-5,8,10,12(Z,Z,E,E)-eicosatetraenoic acid (14,15-LTD₄) for 60 min at 37 °C. The metabolites formed were analyzed with product ion scan at 497.2 m/z for 14,15-LTD₄ and 440.2 m/z for 14,15-LTE₄. The retention time and mass spectrum of a product formed (Metabolite 2) from 14,15-LTC₄ were identical to synthetic 14,15-LTD₄ (fig. 30B), and a product formed (Metabolite 3) after incubation with 14,15-LTD₄ had identical retention time and mass spectrum as synthetic 14 (R)-cysteinyl-15(S)-hydroxy-5,8,10,12(Z,Z,E,E)-eicosatetraenoic (14,15-LTE₄) (fig. 30C). There was no non-enzymatic metabolism of 14,15-LTC₄ and 14,15-LTD₄ during 60 min incubation at 37 °C (data not shown).

Basically, the product ion mass spectra of 14,15-leukotrienes and corresponding 5-lipoxygenase derived cysteinyl-leukotrienes are almost identical. However, 14,15-leukotrienes form a fragment at 205 m/z, whereas the corresponding fragment in 5-lipoxygenase derived cysteinyl-leukotrienes is at 189 m/z (fig. 31). These collision induced dissociation products are probably formed by a cleavage next to the carbon where the cysteinyl group is positioned, which is at carbon 14 for 14,15-leukotrienes and carbon 6 for leukotrienes.

### Comparison between the formation of 14,15-LTC₄ and LTC₄ in eosinophils

Human eosinophils contain abundant amounts of both 15-lipoxygenase and 5-lipoxygenase. In order to examine under which conditions the 15-lipoxygenase and 5-lipoxygenase pathways are most active, eosinophils were incubated with either arachidonic acid or calcium ionophore A23187, or both. The cells were incubated for five minutes at 37 °C and the amounts of 14,15-LTC₄ and LTC₄ were analyzed with RP-HPLC. There is almost no metabolism of 14,15-LTC₄ or LTC₄ in eosinophils after 5 min of incubation (data not shown). Therefore, measurement of these metabolites is considered to reflect the total formation of 15-lipoxygenase derived cysteinyl-containing 14,15-leukotrienes and 5-lipoxygenase derived cysteinyl-containing leukotriens, respectively. Incubation of eosinophils with exogenous arachidonic acid led to the formation of preferentially 14,15-LTC₄ but LTC₄ was also formed in some incubations (table 1). Activation of the cells with calcium ionophore A23187 exclusively induced formation of LTC₄, while challenge of eosinophils with both ionophore A23187 and arachidonic acid led to the formation of both 14,15-LTC₄ and LTC₄. Although similar amounts of these metabolites were produced in some incubation, the levels of LTC₄ were much higher in certain samples. Taken together, incubations of the cells with arachidonic acid favour the formation of 14,15-LTC₄ and calcium activation of the cells triggers the 5-LO and strongly favour the formation of 5-LO derived leukotrienes.

**Table 1: Biosynthesis of 14,15-LTC₄ and LTC₄ in eosinophils. Eosinophils were isolated from healthy donors or from patients. The cells were suspended in phosphate buffered saline (1 ml) and incubated with arachidonic acid (10 µM), ionophore A23187 (1 µM) or both, for five minutes at 37 °C and the supernatants were thereafter subjected to reverse phase HPLC analysis. The relative amounts of 14,15-LTC₄ and LTC₄ were calculated as pmol / 10⁷ eosinophils.**

| | Arachidonic acid | | A23187 | | Arachidonic acid + A23187 | |
|---|---|---|---|---|---|---|
| Donor | 14,15-LTC₄ | LTC₄ | 14,15-LTC₄ | LTC₄ | 14,15-LTC₄ | LTC₄ |
| | pmol/10⁷ | pmol/10⁷ | pmol/10⁷ | pmol/10⁷ | pmol/10⁷ | pmol/10⁷ |
| 1 | 40 | 0 | 0 | 50 | 30 | 14 |
| 2 | 32 | 0 | 0 | 40 | 16 | 20 |
| 3 | 58 | 34 | 0 | 198 | 20 | 322 |
| 4 | 74 | 12 | 0 | 494 | 34 | 420 |
| 5 | 34 | 20 | 0 | 82 | 26 | 34 |
| 6 | 12 | 0 | 0 | 108 | 4 | 18 |
| 7 | 26 | 6 | 0 | 64 | 14 | 16 |
| 8 | 46 | 10 | 0 | 326 | 18 | 444 |
| 9 | 16 | 0 | 0 | 170 | 14 | 24 |
| 10 | 22 | 38 | 0 | 30 | 18 | 36 |
| 11 | 48 | 22 | 0 | 264 | 12 | 385 |

### Biological effects of 14,15-leukotrienes

Increase in vascular permeability leading to plasma leakage is a hallmark of inflammation and it is well known that the cysteinyl-containing leukotrienes may contribute to this effect in a similar way as histamine and bradykinin (23). To explore the ability of 14,15-LTC₄ and its metabolites 14,15-LTD₄ and 14,15-LTE₄ to induce alterations in vascular permeability, an established in vitro permeability assay based on assessment of changes in electrical resistance across human endothelial cell (EC) monolayers was used. Maximum stimulus-induced change in EC resistance in this model reaches down to approximately -70 %. Dose-response curve show that maximum effect of histamine was obtained at 10⁻⁵ M in this model system (data not shown). All 14,15-leukotrienes were capable of inducing maximum increase in EC monolayer permeability (fig. 32A). Although being somewhat less potent than LTC₄ and LTD₄ the 14,15-leukotrienes were about hundred times more potent than histamine in this respect (fig. 32A). 14,15-LTD₄ induced maximal increase of EC permeability at 10⁻⁷M but significant increase in EC permeability was also observed at 10⁻⁸M (fig. 32B). The kinetics of changes in EC permeability in response to 14,15-leukotrienes and LTD₄ resemble the pattern observed for directly-acting agonists such as histamine and LTC₄ (fig. 32B) (24). LTB₄ on the other hand requires the presence of granulocytes to affect EC barrier function and no effect of LTB4 was observed in this model system.

### Receptor mediated activation of 14,15-LTC₄ biosynthesis in eosinophils

A possible receptor mediated activation of 14,15-LTC₄ formation was investigated by using the allergen induced mast cell mediators LTC₄ and prostaglandin (PG) D₂ as well as IL-5, which is a potent eosinophil activator. Isolated eosinophils (10x10⁶/sample) were incubated for 5 min, at 37 °C with LTC₄ (1 µM), PGD₂ (1 µM) or IL-5 (10 ng/ml). Leukotriene C₄, PGD₂ and IL-5 all induced release of 14,15-LTC₄ from eosinophils after 5 min of incubation (table 2). These results demonstrate formation of 14,15-LTC₄ from the endogenous pool of arachidonic acid in eosinophils via receptor-mediated mechanisms.

**Table 2: Receptor mediated activation of 14,15-LTC4 biosynthesis in eosinophils. Human eosinophils isolated from healthy volunteers or from patients diagnosed with eosinophilia were suspended in 1 ml of PBS and incubated for 5 min at 37 °C in the presence of LTC₄ (1 µM), PGD₂ (1 µM) or IL-5 (10 ng/ml). Control, LTC₄, and PGD₂, n=4. IL,-5, n=2. The amount of 14,15-LTC₄ was analyzed by EIA.**

| | n | 14,15-LTC₄ |
|---|---|---|
| | | (pg / 10⁷ cells |
| Control | 4 | 11.9 ± 8.1 |
| LTC₄ | 4 | 56.2 ± 42.5 |
| PGD₂ | 4 | 43.8 ± 16.9 |
| IL-5 | 2 | 131 ± 4.9 |

### Biosynthesis of 14,15-LTC₄ and 14,15-LTD₄ in cord blood derived mast cells (CBMC^{MNC})

Mast cells play a key role in the pathophysiology of asthma. Recently, we reported that interleukin-4 (IL-4) induced the expression of 15-LO-1 in human cord blood derived mast cells in vitro and that mast cells express 15-LO-1 in vivo (4). Therefore, it was of interest to investigate if mast cells could produce 14,15-leukotrienes. IL-4 primed mast cells were incubated with arachidonic acid (10 µM) for 5 min. Supernatants were collected and analyzed using an EIA against 14,15-LTC₄. Under these conditions, human mast cells released 0.8 +/- 0.6 pmol/10⁶ cells, (n=3), indicating the capacity to synthesise and release immunoreactive 14,15-LTC4 after arachidonic acid stimulation.

The identity of 14,15-LTC₄ was further verified with LC-MS/MS single reaction monitoring, SRM. Thus, mast cells incubated with arachidonic acid gave rise to a metabolite with the same retention time and with a typical fragment pattern for the 14,15-LTC₄ SRM transition. (data not shown). In addition, mast cell samples were analyzed with a specific 14,15-LTD₄ SRM method to investigate whether the 14,15-LTC4 was further metabolised by the cells. Indeed, a metabolite with fragmentation pattern and retention time identical to synthetic 14,15-LTD₄ was demonstrated. Taken together, mast cells possess the capacity to produce both 14,15-LTC₄ and 14,15-LTD₄.

### Spontaneous release of 14.15-LTC₄ by human nasal polyps

Nasal polyposis is a multifactorial disease consisting of tissue infiltration by inflammatory cells such as eosinophils (25). To investigate if 14,15-LTC₄ could be synthesized by human nasal polyps, intact surgically removed nasal polyps were washed and incubated in PBS, containing protease inhibitor (Complete MINI^{®}) and indomethacin, at 37 °C for 5 or 30 min. Subsequently, the incubation medium was analyzed using an ELA against 14,15-LTC₄. The results demonstrate that nasal polyps spontaneously release 14,15-LTC₄ (10.1 ± 0.007 and 7.18 ± 1.18 pmol 14,15-LTC₄ / g polyp tissue (n=2) after 5 and 30 min incubation, respectively). The decreasing levels of 14,15-LTC₄ over time could be due to further metabolism to 14,15-LTD₄ and 14,15-LTE₄ which is not detected by the antibodies raised against 14,15-LTC4 (cross reactivity less than 1 % against 14,15-LTD4 and 14,15-LTE4 , see material and methods).

### Eoxins - a suggested novel name for 14,15-leukotrienes

The name leukotriene is traditionally linked to 5-lipoxygenase products. The working name for the 15-lipoxygenases metabolites formed via the 14,15-epoxide in arachidonic acid has been 14,15-leukotriene, although it is not formed via the 5-lipoxygenase pathway. Since eosinophils are a rich source for these novel 15-lipoxygenase products described in this report, and to avoid confusion with 5-LO derived products, we suggest the name eoxin (Eox) C₄, EoxD₄ and EoxE₄ instead of 14,15-LTC₄, 14,15-LTD₄ and 14,15-LTE₄, respectively (fig. 33).

### Discussion

Human airway epithelial cells and eosinophils constitutively express high amounts of 15-LO-1 (3, 5, 6). Also subsets of human mast cells, macrophages and dendritic cells express 15-LO-1 (26 + Gulliksson). Despite much work in this research field, the biological role of this enzyme is unclear except the enzyme's function during erythropoeisis (2, 3). In the present report we describe that human eosinophils in particular, but also cord blood derived mast cells and nasal polyps, have the ability to convert arachidonic acid via the 15-LO-1 pathway to metabolites with a conjugated triene spectrum and UV absorbance maximum at 282 nm (fig. 29). The structures of these metabolites formed in eosinophils and mast cells were determined by positive ion tandem mass spectrometry and RP-HPLC/UV-spectroscopy and were found to be identical to synthetic 14,15-LTC₄, 14,15-LTD₄ and 14,15-LTE₄ (figs. 30 and 31). Eosinophils mainly produced 14,15-LTC₄, and this metabolite could be further metabolised to 14,15-LTD₄ and 14,15-LTE₄. as demonstrated by incubation of these cells with synthetic 14,15-LTC₄ and 14,15-LTD₄, respectively. Although, synthetic 14,15-LTC4 has been studied in various biological systems (see, for example, Drazen et al (1981) Proc Natl Acad Sci 78, 3195-3198) and 14,15-LTA4 has been earlier found to be formed in epithelial cells and eosinophils (see, for example ref 32), the biosynthesis of cysteinyl-containing 14,15-leukotrienes have never been demonstrated to occur from arachidonic acid in human cells and tissues.

Incubation of eosinophils with exogenous arachidonic acid favoured the formation of 14,15-LTC₄ over LTC₄ (table 1), while increasing intracellular calcium levels with a calcium ionophore instead triggered 5-LO and led only to the production of LTC₄. Thus, the regulation of 5-LO and 15-LO leukotriene production differs and which metabolite that will be the major metabolite excreted will depend on the stimulus. In this respect the 14,15-leukotriene pathway resembles the prostaglandin pathway, occurring readily when free arachidonic acid is available and being independent of calcium fluxes.

In contrast to the 5-lipoxygenase derived cysteinyl-containing leukotrienes, 14,15-LTC₄ and its metabolites appear not to have contractile effects on guinea pig pulmonary parenchymal strip or ileum (27). However, we here demonstrate that all three 14,15-leukotrienes increased vascular leakage, a marker of inflammation, in a model system with confluent human endothelial cells. The 14,15-leukotrienes induced increased EC permeability at 10⁻⁷ and 10⁻⁸ M and were almost as potent as cysteinyl-containing leukotrienes and more than 100 times more potent than histamine in this respect (fig. 32). In this study, we used an in vitro model system for analysis of mediator-induced perturbation of endothelial cell barrier function. Continuous measurement of TEER permits detection of rapid changes in EC permeability and has previously been shown to be a sensitive measure of the barrier capacity of cultured endothelial cell monolayers (24, 28). The permeability-increasing activity of inflammatory mediators like histamine and the cysteinyl leukotrienes is known since long. These mediators exert their effect on EC barrier function through triggering intracellular Ca²⁺ mobilization and cytoskeletal rearrangement (29) leading to paracellular gap formation and enhanced macromolecular permeability. Likely, the 14,15-leukotrienes stimulate increase in vascular permeability in a similar way.

Receptor-mediated activation of human eosinophils with LTC₄, PGD₂ and IL-5 also triggered the production and release of 14,15-leukotrienes (table 2). Thus, 14,15-leukotrienes can be produced from the endogenous pool of arachidonic acid in eosinophils. LTC₄ and PGD₂ are both arachidonic acid derived compounds, known to be synthesized and released by mast cells upon antigen challenge, suggesting that activated mast cells, in addition to produce 14,15-leukotrienes itself, can stimulate the biosynthesis of these products in eosinophils. In addition to LTC₄ and PGD₂, IL-5 was able to activate the endogenous biosynthesis of 14,15-LTC₄. IL-5 is a cytokine critically involved in regulating several aspects of eosinophils, including their production, activation and tissue recruitment.

The cause of nasal polyp formation is unknown, but arachidonic acid metabolites seem to be important in the pathogenesis, especially in patients with aspirin hypersensitivity rhinosinusitis/asthma symptoms. The principal hallmark of nasal polyposis is considered to be accumulation of activated eosinophils, which account for up to 28% of the inflammatory cells within the tissue (30). Therefore, it was of interest to investigate whether human nasal polyps incubated in vitro could produce and release 14,15-leukotrienes. Indeed, 14,15-LTC₄ was spontaneously released into the incubation medium by the nasal polyps. It is likely that activated eosinophils are the major source of 14,15-LTC₄ in nasal polyps. However, it cannot be excluded that the airway epithelial cells could play a role since these cells are known to contain high amounts of 15-LO (see refs 2-6). Since eosinophils are a rich source of these novel metabolites, we suggest the name eoxin instead of 14,15-leukotriene to avoid confusion with compounds produced via the 5-LO pathway. Thus, the names 14,1 5-leukotriene A₄, C₄, D₄ and E₄ are replaced with eoxin (Eox) A₄, EoxC₄, EoxD₄ and EoxE₄, respectively (fig. 33). Eoxins have never been reported to be produced from arachidonic acid in human cells. Human basophils, however, has earlier been found to convert exogenous 14,15-leukotriene A₄ to 14,15-leukotriene C₄ (33).

Taken together, this report describes the formation of novel proinflammatory arachidonic acid metabolites produced by eosinophils and mast cells, as well as the identification of various physiological triggers for the biosynthesis of these metabolites, Thus, it is possible that activated mast cells and Th2 cells in inflammatory airways excrete LTC4, PGD2 and IL-5, which could induce nearby eosinophils to produce and release eoxins, leading to plasma leakage. Furthermore, in addition to allergic inflammation, it can be speculated that also in non-allergic inflammation, where free arachidonic acid is present in high amounts, eoxin production could occur contributing to the inflammatory process.

Many reports have demonstrated increased amounts and activity of 15-LO-1 in the airway tissues of patients with asthma compared to control subjects. Healthy smokers, but in particular smokers with chronic bronchitis, display an increased expression of 15-LO-1 in bronchial biopsies. In this report we show that the 15-LO-1 pathway can generate the pro-inflammatory 14,15-leukotrienes (eoxins) in eosinophils and mast cells, indicating that inhibition of 15-LO-1 could be an attractive target for treatment of asthma and rhinitis in human.

### Material and methods

Vacutainer^{®} blood collection tubes were obtained from Becton Dickinson (Rutherford, NJ). Paramagnetic microbeads conjugated to monoclonal mouse antihuman CD16 antibodies, magnetic stands and LS⁺ magnetic separation columns were from Miltenyi Biotec (Bergisch Gladbach, Germany). Recombinant human (rh) IL-4 was bought from In Vitro Sweden AB (Sweden). 14,15-LTC₄ EIA kit was purchased from Cayman Chemical (Ann Arbor, MI). Ionophore A23187 was purchased from Calbiochem-Boehring (La Jolla, CA). Indomethacin was purchased from Sigma-Aldrich. All solvents were of HPLC grade. Arachidonic acid was bought from Nu-Chek prep. Synthetic 14,15-epoxy-5,8,10,12 (Z,Z,E,E)-eicosatraenoic acid (14,15-LTA₄), 14(R)-glutathionyl-15(S)-hydroxy-5,8,10,12 (Z,Z,E,E)-eicosatetraenoic acid (14,15-LTC₄), 14(R)-cysteinyl-glycyl-15(S)-hydroxy-5,8,10,12 (Z,Z,E,E)-eicosatetraenoic acid (14,15-LTD₄), 14(R)-cysteinyl-15(S)-hydroxy-5,8,10,12 (Z,Z,E,E)-eicosatetiaenoic acid (14,15-LTE₄), 8(R),15(S)-dihydroxy-5,9,11,13 (Z,E,E,E)-eicosatetraenoic acid (8(R),15(S)-LTB₄), 8(S),1S(S)-dihydroxy-5,9,11,13 (Z,E,Z,E)-eicosatetraenoic acid (8(S),15(S)-DHETE), 8(S),15(S)-dihydroxy-5,9,11,13 (Z,E,E,E)-eicosatetraenoic acid (8(S),15(S)-LTB₄), 8(R),15(S)-dihydroxy-5,9,11,13 (Z,E,Z,E)-eicosatetraenoic acid (8(R),15(S)-DHETE), 14(R),15(S)-dihydroxy-5,8,10,12 (Z,Z,E,E)-eicosatetraenoic acid (14(R),15(S)-DHETE or 14(R),15(S)-LTB₄), 1S(S)-hydroxy-5,8,11,13 (Z,Z,Z,E)-eicosatetraenoic acid (15-HETE), LTC₄, LTD₄ and LTE₄ were from Biomol Inc (Plymouth Meeting, USA).

### Isolation of human eosinophils

Venous blood (100 ml) was drawn from healthy volunteers or from patients with hypereosinophilia, including hypereosinophilic syndrome (HES), chronic lymphocytic leukemia (CLL) and certain inflammatory disorders. An initial centrifugation at 400 x g for 15 min was performed. The upper phase was discarded and the lower phase was collected and subjected to Dextran^{®} sedimentation for 30 min at 20 °C. After sedimentation, the upper phase containing white blood cells was centrifuged once at 400 x g for 10 min. The pellet was suspended in Lysis buffer (100 mM NH₄Cl, 10 mM TRIS-HCl, pH 7.4) and incubated for 30 min in room temperature to get rid of contaminating erythrocytes. Following incubation, density gradient centrifugation, using Lymphoprep^{®}, was performed. The polymorphonuclear (PMN) fraction, containing neutrophils and eosinophils, was collected and subjected to Magnetic Cell Sorting (MACS). Eosinophils were isolated by negative selection using CD16 antibodies conjugated to magnetic MicroBeads^{®}. Purity was confirmed by morphological examination and by staining with May Grunewald-Giemsa solution. Purity was always found to be greater than 95%.

### Preparation of cord blood derived mast cells (CBMC^{MNC}) and isolation of polyps

Cord blood derived mast cells (CBMC^{MNC}) were established as described (4). Polyps were surgically removed and kept in PBS for a maximum of 1 h at 4 °C. Polyps were separated from surrounding tissue and washed in PBS w/o Ca²⁺ and Mg²⁺. The weight of washed wet polyps was 0.7-1 g.

### Cell incubations

### Eosinophils

Isolated eosinophils (1-20 x 10⁶ / sample) were resuspended in phosphate buffered saline (1 ml / sample) and pre-incubated for 2 minutes in 37 °C (in presence or absence of indomethacin, 1 µM) prior to stimulation with ionophore A23187 (1 µM) / arachidonic acid (1 or 10 µM) / A23187 plus arachidonic acid / PGD₂ (1 µM) / LTC₄ (1 µM) / IL-5 (10 ng/ml) / 14,15-LTC₄ (10 µM) or 14,15-LTD₄ (10 µM) for indicated time periods at 37 °C. The incubations were terminated by addition of one volume of methanol (for analysis by RP-HPLC). For subsequent analysis by enzyme immunoassay (EIA), reactions were terminated by rapid cool off, 0 °C, before centrifugation at 1200 x g, 5 min. Supernatants were transferred to cryo tubes and stored in -70°C until analyzed.

### Mast cells

Prior to incubation, CBMC cells were primed for four days with IL-4 (10 ng/ml). After mechanical removal and washing in PBS, cells were resuspended in PBS and pre-incubated with or without indomethacin (1 µM) for 2 minutes in 37 °C. The CBMC cells were subsequently incubated in 37 °C with arachidonic acid for indicated time periods. Incubations were terminated either by addition of one volume of methanol (LC-MS analysis) or by rapid cool off, 0 °C, and subsequently analyzed by EIA.

### Human Nasal Polyps

Human washed intact polyps were transferred into clean test tubes containing PBS, protease inhibitor (Complete MINT^{®}) and indomethacin (1 µM) in a total volume of one mL Samples were incubated at 37 °C for 5 min and 30 min, respectively. Reactions were terminated by rapid cool off, 0 °C and subsequent centrifugation at 1200 x g, 5 min. Supernatants were transferred to cryo tubes and stored in -70°C until analyzed by EIA.

### Analysis of 15-lipoxygenase products

### Reverse Phase - High Pressure Liquid Chromatography-UV (RP-HPLC-UV)

In order to get rid of cellular debris and proteins, centrifugation at 1200 x g for 5 min. was performed. Supernatants were evaporated under reduced pressure and the residues resolved in methanol and transferred to a test tube. Samples were thereafter evaporated to dryness under a stream of nitrogen, and finally resuspended in 200 µl mobile phase (acetonitril:methanol:H₂0:acetic acid, 29:19:72:0.8, pH adjusted to 5.6 with NH₃ for analysis of leukotrienes and 14,15-leukotrienes, and methanol:water:trifluoroacetic acid 70:30:0.007 for analysis of monohydroxy acids). The extracted samples were subjected to analysis in a reverse phase HPLC system as described. Briefly, samples were applied to a octadecyl reverse phase column (Chromabond ¹⁸C, Macherey-Nagel, Duren, Germany) and elution rate was 1.2 ml/minute. Eluted compounds were detected and quantified utilizing Waters 996 photo diode array spectrophotometer connected to Waters 1996 HPLC system.

### Liquid Chromatography-Mass Spectrometry analysis (LC-MS/MS)

After termination of incubations, samples were centrifuged (1400 x g, 6 min) and the supernatants were diluted with water to contain maximum 25 % methanol and transferred to a washed and equilibrated extraction cartridge, Oasis HLB 1 cc 10 mg (Waters AB, Sweden). The columns were washed with water and eluted with 200µl methanol to retrieve the metabolites.

Liquid chromatography - mass spectrometry was performed on a Surveyor MS coupled to a TSQ Quantum Ultra triple quadrupole mass spectrometer (Thermo Electron Corporation, Sweden). One volume of water was added to samples prior to injection. Reverse phase LC was performed using a Zorbax Eclipse Plus 3.5µM C18 column 2.1 x 50 mm (ChromTech, Sweden) with flow rate constantly held at 400 µl/min. Mobile phase A consisted of 2 % acetonitrile in water and 0.1% acetic acid and mobile phase B consisted of 80 % acetonitrile in water and 0.1% acetic acid. All water used was of MilliQ grade. Starting isocratically for 1.5 min at 90 % A was followed by a 6.5 min linear gradient reaching 100 % of B. The system was washed at 100 % B for 4 minutes and subsequently equilibrated at 90. % A for 4 minutes. The 14,15-leukottienes were chromatographically separated from leukotrienes by approximately 0.5 min. The mass spectrometer was operated using electrospray atmospheric pressure ionization source in positive mode. The spray voltage was set to 4500 V, capillary temperature was 375 °C and sheath and auxillary gas was optimal at 40 respectively 5 (arbitrary units). Skimmer offset was at 10 V and tube lens 121 V.

### LC-MS/MS for eosinophil metabolites

LC-MS/MS was utilized to perform product ion scans of protonated 14,15-LTC₄, 14,15-LTD₄ and 14,15-LTE₄. 14,15-LTC₄ at 626.2 m/z was fragmented at a collision energy of 22 eV, 14,15-LTD₄ at 497.2 and 14,15-LTE₄ at 440.2 m/z both at 18 eV.

### LC-MS/MS for mast cell metabolites

14,15-LTC₄ and 14,15-LTD₄ was detected by single reaction monitoring, SRM. The transition utilized in the SRM scan was 626 m/z→279 m/z for 14,15-LTC₄ and 497 m/z→205 m/z for 14,15-LTD₄, both at a collision energy of 20 eV. The transition chosen for 14,15-LTC₄ was also present for LTC₄, but these two compounds were chromatographically separated by approximately 0.5 minutes. The 497 m/z→205 m/z transition was specific for 14,15-LTD₄, and therefore not applicable for LTD₄.

### Characterization of an enzyme immunoassay (EIA) against 14,15-LTC₄

An enzyme immunoassay (EIA) against 14,15-LTC₄ was developed by Cayman Chemical (Ann Arbor, MI) in order to quantify small amounts of 14,15-LTC₄. This method was approximately thousand times more sensitive than RP-HPLC in quantifying 14,15-LTC₄. The EIA cross reactivities were determined by the manufacturer. The cross reactivity to 14,15-LTD₄ and 14,15-LTE₄ was 0.9 % and 0.04 %, respectively. For Leukotriene C₄, leukotriene D₄, leukotriene E₄, arachidonic acid, 12-HETE, 15-HETE, 5(S),6(S)-diHETE, 5(S),15(S)-diHETE, 8(S),15(S)-diHETE and 14,15-diHETE cross reactivity was less than 0.01%. Supernants were diluted in EIA buffer and analyses were performed according to the manufacturer's protocol. The optical density (OD) from the EIA determinations was used to calculate % BB₀ according to the relationship: % BB₀ = (sample OD - NSB OD) / (zero standard OD - NSB OD) x 100

A standard curve ranging from 7.8-1000 pg/ml (0.012-1.6 pmol/ml) 14,15-LTC₄ was generated and used to determine concentrations of 14,15-LTC₄ in samples.

### Assessment of endothelial cell barrier function

Human umbilical vein endothelial cells (HUVEC) were isolated and cultured as previously described (24). The cells were seeded onto 3 µm pore size polycarbonate filters (Tissue Culture inserts, 10 mm; NUNC, Roskilde, Denmark) at a density of 2 x 10⁵ cells and grown to confluent monolayers. The capacity of arachdonic acid metabolites to induce changes in barrier function of endothelial cell (EC) monolayers was analyzed through continuous registration of transendothelial electrical resistance (TEER) (24). For measurement of TEER, the filter insert with ECs was transferred to a resistance measurement chamber (Endohm-12, World Precision Instruments, Sarasota, FL, USA) modified so as to assure exact positioning of the electrodes in relation to each other and to the filter insert during repeated measurements. The chamber (lower compartment) and the filter insert (upper compartment) were filled with 2 ml and 400 µl culture medium, respectively. All measurements were made at 37 °C with the equipment placed in a cell culture incubator. The electrical resistance of individual EC monolayers was obtained by subtracting the resistance of the corresponding naked filter coated with Biomatrix as measured prior to seeding of the EC. Thus, TEER values represent the resistance of the EC monolayer solely.

### References for Example 8

- 1.: Claesson, H. E. & Dahlen, S. E. (1999) J Intern Med 245, 205-27.
- 2.: Kuhn, H., Walther, M. & Kuban, R. J. (2002) Prostaglandins Other Lipid Mediat 68-69, 263-90.
- 3.: Kuhn, H. & O'Donnell, V. B. (2006) ProgLipid Res 45, 334-56.
- 4.: Gulliksson, M., Brunnström, A., Johannesson, M., Backman, L., Nilsson, G., Harvima, I. T., Dahlén, B., Kumlin, M. and Claesson, H-E. (2007) Biochem Biopys Acta in press.
- 5.: Hunter, J. A., Finkbeiner, W. E., Nadel, J. A., Goetzl, E. J. & Holtzman, M. J. (1985) Proc Natl Acad Sci USA 82, 4633-7.
- 6.: Turk, J., Maas, R. L., Brash, A. R., Roberts, L. J., 2nd & Oates, J. A. (1982) J Biol Chem 257, 7068-76.
- 7.: Brash, A. R., Boeglin, W. E. & Chang, M. S. (1997) Proc Natl Acad Sci U S A 94, 6148-52.
- 8.: Conrad, D. J., Kuhn, H., Mulkins, M., Highland, E. & Sigal, E. (1992) Proc Natl Acad Sci U S A 89, 217-21.
- 9.: Brinckmann, R., Topp, M. S., Zalan, I., Heydeck, D., Ludwig, P., Kuhn, H., Berdel, W. E. & Habenicht, J. R. (1996) Biochem J 318 ( Pt 1), 305- 12.
- 10.: Shannon, V. R., Chanez, P., Bousquet, J. & Holtzman, M. J. (1993) Am Rev Respir Dis 147, 1024-8.
- 11.: Bradding, P., Redington, A. E., Djukanovic, R., Conrad, D. J. & Holgate, S. T. (1995) Am J Respir Crit Care Med 151, 1201-4.
- 12.: Hamberg, M., Hedqvist, P. & Radegran, K. (1980) Acta Physiol Scand 110, 219-21.
- 13.: Kumlin, M., Ohlson, E., Bjorck, T., Hamberg, M., Granstrom, E., Dahlen, B., Zetterstrom, O. & Dahlen, S. E. (1991) Adv Prostaglandin Thromboxane Leukot Res 21A, 441-4.
- 14.: Chu, H. W., Balzar, S., Westcott, J. Y., Trudeau, J. B., Sun, Y., Conrad, D. J. & Wenzel, S. E. (2002) Clin Exp Allergy 32, 1558-65.
- 15.: Dahlen, S. E., Hansson, G., Hedqvist, P., Bjorck, T., Granstrom, E. & Dahlen, B. (1983) Proc Natl Acad Sci USA 80, 1712-6.
- 16.: Murray, J. J., Tonnel, A. B., Brash, A. R., Roberts, L. J., 2nd, Gosset, P., Workman, R., Capron, A. & Oates, J. A. (1985) Trans Assoc Am Physicians 98, 275-80.
- 17.: Profita, M., Sala, A., Riccobono, L., Paterno, A., Mirabella, A., Bonanno, A., Guerrera, D., Pace, E., Bonsignore, G., Bousquet, J. & Vignola, A. M. (2000) JAllergy Clin Immunol 105, 711-6.
- 18.: Ramis, I., Rosello-Catafau, J., Bulbena, O., Picado, C. & Gelpi, E. (1989) J Chromatogr 496, 416-22.
- 19.: Jubiz, W., Radmark, O., Lindgren, J. A., Malmsten, C. & Samuelsson, B. (1981) Biochem Biophys Res Commun 99, 976-86.
- 20.: Maas, R. L. & Brash, A. R. (1983) Proc Natl Acad Sci USA 80, 2884-8.
- 21.: Schwenk, U., Morita, E., Engel, R. & Schroder, J. M. (1992) J Biol Chem 267, 12482-8.
- 22.: Serhan, C. N. & Savill, J. (2005) Nat Immunol 6, 1191-7.
- 23.: Raud, J., Lindbom, L. & Hedqvist, P. (1992) Acta Physiol Scand 146, 545- 6.
- 24.: Gautam, N., Hedqvist, P. & Lindbom, L. (1998) Br J Pharmacol 125, 1109-14.
- 25.: Bachert, C., Gevaert, P., Holtappels, G. & van Cauwenberge, P. (2002) Curr Allergy Asthma Rep 2, 481-7.
- 26.: Spanbroek, R., Hildner, M., Kohler, A., Muller, A., Zintl, F., Kuhn, H., Radmark, O., Samuelsson, B. & Habenicht, A. J. (2001) Proc Natl Acad Sci U S A 98, 5152-7.
- 27.: Lewis, R. A., Drazen, J. M., Austen, K. F., Toda, M., Brion, F., Marfat, A. & Corey, E. J. (1981) Proc Natl Acad Sci USA 78, 4579-83.
- 28.: Gautam, N., Herwald, H., Hedqvist, P. & Lindbom, L. (2000) J Exp Med 191, 1829-39.
- 29.: Huang, A. J., Manning, J. E., Bandak, T. M., Ratau, M. C., Hanser, K. R. & Silverstein, S. C. (1993) J Cell Biol 120, 1371-80.
- 30.: Ferguson, B. J. (2000) Laryngoscope 110, 799-813.
- 31.: Holtzman, M. J., Hansbrough, J. R., Rosen, G. D. & Turk, J. (1988) Biochim Biophys Acta 963, 401-13.
- 32.: Turk, J., Rand, T. H., Maas, R. L., Lawson, J. A., Brash, A. R., Roberts, L. J., 2nd, Colley, D. G. & Oates, J. A. (1983) Biochim Biophys Acta 750, 78- 90.
- 33.: Sok, D. E., Han, C. O., Shieh, W. R., Zhou, B. N. & Sih, C. J. (1982) Biochem Biophys Res Commun 104, 1363-70.

References (other than for Example 8)
1. Funk CD. The molecular biology of mammalian lipoxygenases and the quest for eicosanoid functions using lipoxygenase-deficient mice [Review]. Biochimica et Biophysica Acta - Lipids & Lipid Metabolism 1996; 1304(1):65-84.
2. Claesson HE, Dahlen SE. Asthma and leukotrienes: antileukotrienes as novel anti-asthmatic drugs [Review]. Journal of Internal Medicine 1999; 245(3):205-227.
3. Rapoport SM and Schewe T. The maturational breakdown of mitochondria in reticulocytes. Biochim. Biophys. Acta 1986; 864:471-495.
4. Vanleyen K, Duvoisin RM, Engelhardt H, Wiedmann M. A function for lipoxygenase in programmed organelle degradation. Nature 1998; 395(6700):392-395.
5. Ford-Hutchinson AW. Arachidonate 15-lipoxygenase; characteristics and potential biological significance. Eicosanoids 1991; 4:65-74.
6. Conrad DJ, Kuhn H, Mulkins M, et al. Specific inflammatory cytokines regulate the expression of human monocyte 15-Lipoxygenase. Proc Natl Acad Sci USA 1992; 89(1):217-221.
7. Holtzman MJ, Hansbrough, R. J., Rosen, G. D. and Turk, J. Uptake, release and novel species-dependent oxygenation of arachidonic acid in human and animal airway epithelial cells. Biochim. Biophys. Acta 1988; 963:401-413.
8. Rapoport SM, Schewe,T, Wiesner, R., Halangk, W., Ludwig, P., Janicke-Hohne, M., Tannert, C., Hiebsch, C. and Klat, D. The lipoxygenase of reticulocytes. Purification, characterisation and biological dynamics of the lipoxygenase: its identity with the respiratory inhibitors of the reticulocyte. Eur. J. Biochem 1979; 96:545-561.
9. Sigal E, Grunberger, D., Highland, E., Gross, C., Dixon, R. A., Craik, C. S. Expression of cloned human reticulocyte 15-lipoxygenase and immunological evidence that 15-lipoxygenase of different cell types are related. J. Biol. Chem. 1990;265:5113-5120.
10. Brash AR, Boeglin WE, Chang MS. Discovery of a second 15s-lipoxygenase in humans. Proc Natl Acad Sci USA 1997; 94(12):6148-6152.
11. Maas RL, Brash, A. R. and Oates, J. A. A second pathway of leukotriene biosynthesis in porcine leukocytes. Proc. Natl. Acad. Sci. 1981; 78:5523-5527.
12. Lundberg U, Malmsten C, Samuelsson B. Transformation of 15-hydroperoxy-5,9,11,13-eicosatetraenoic acid into novel leukotrienes. FEBS Lett 1981; 126:127-132.
13. Serhan CN, Haeggström, J. Z. and Leslie, C. C. Lipid mediator networks in cell signaling: update and impact och cytokines. The Faseb journal 1996; 10:1147-1158.
14. Schwenk U, Morita E, Engel R, Schroder JM. Identification of 5-oxo-15-hydroxy-6,8,11,13-eicosatetraenoic acid as a novel and potent human eosinophil chemotactic eicosanoid. J Biol Chem 1992; 267(18):12482-12488.
15. Wolf W, Kapp U, Bohlen H, Komacker M, Schoch C, Stahl B, Mucke S, von Kalle C, Fonatsch C, Schaefer H-E, Hansmann M-L and Diehl V: Peripheral blood mononuclear cells of a patient with advanced Hodgkin's lymphoma give rise to permanently growing Hodgkin-Reed Sternberg cells. Blood, 1996; Vol 87 (8) 3418-3428.
16. Griffiths W, Yang Y, Sjövall J and Lindgren JÅ: Electrospray tandem mass spectrometry of cysteinyl leukotrienes. Rapid Commun. Mass Spectrom. 1996; 10:1054-10709.
17. Hunter JA, Finkbeiner WE, Nadel EJ, et al. Predominant generation of 15-lipoxygenase metabolites of arachidonic acid by epithelial cells from human trachea. Proc. Natl. Acad. Sci. USA 1985; 82:4633-4637.
18. Gautam N, Hedqvist P and Lindbom L (1998). Kinetics of leukocyte-induced changes in endothelial barrier function. Br J Pharmacol 125: 1109-1114.
19. Mannervik B and Danielsson UH (1988). Glutathione transferases: structure and catalytic activity. CRC Crit Rev Biochem 3, 283-337.
20. Izumi Takashi et al (1988). Solubilization and partial purification of leukotriene C4 synthase from guinea-pig lung: a microsomal enzyme with high specificity towards 5,6-epoxide leukotriene A4. Biochem Biophys Acta 959, 305-315.
21. Jakobsson P-J, Morgenstern R, Mancini J, Ford-Hutchinson A and Persson B (2000). Membrane-associated proteins in eicosanoid and glutathione metabolism. Am J Respir Crit Care Med 161, 20-24.
22. Gillmor, S.A., Villasenor, A., Fletterick, R., Sigal, E. And Browner, M.F., 1997, The structure of mammalian 15-lipoxygenase reveals similarity to the lipases and the determinants of substrate specificty Nature Structural Biology, vol 4, issue 12, 1003-1009.

## Claims

1. A method for identifying a compound for modulating the formation of 14,15-LTC₄ (Eoxin C₄; EoxC₄), 14,15-LTD₄ (Eoxin D₄; EoxD₄), or 14,15-LTE₄ (Eoxin E₄; EoxE₄) in a biological system other than a human being or human embryonic cells, the method composing testing the compound for an effect on 15 lipoxygenase (15-LO) activity or Glutathione S transferase M1-1 (GSTM1-1), Glutathione S transferase P1-1 (GSTP1-1) and/or leukotriene C₄ synthase activity.

2. A method for identifying a compound with an inflammation-modulating effect or a bone loss-modulating effect, the method comprising testing the compound for an effect on formation of EoxC₄, EoxD₄ or EoxE₄ in a biological system other than a human being or human embryonic cells, and/or
testing the compound for an effect on activity of EoxC₄, EoxD₄ or EoxE₄ in a biological system other than a human being or human embryonic cells wherein the activity of the compound is tested by testing for an inhibitory effect on Eoxin-induced permeabilisation of endothelial cells in an *in vitro* model of inflammation; or for an inhibitory effect on Eoxin-induced oxygen free radical production by leukocytes.

3. The method of claim 2 wherein the method is for identifying a compound with an anti-inflammatory effect or a bone loss-reducing effect and the method comprises testing the compound for an inhibitory effect on formation and/or activity of EoxC₄, EoxD₄ or EoxE₄ in a biological system other than a human being or human embryonic cells.

4. The method of claim 1 further comprising the step of testing the compound for an effect on formation of EoxC₄. EoxD₄ or EoxE₄ in a biological system other than a human being or human embryonic cells, or
testing the compound for an effect on activity of EoxC₄, EoxD₄ or EoxE₄ in a biological system other than a human being or human embryonic cells wherein the activity of the compound is tested by testing for an inhibitory effect on Eoxin-induced permeabilisation of endothelial cells in an *in vitro* model of inflammation; or for an inhibitory effect on Eoxin-induced oxygen free radical production by leukocytes.

5. The method of any one of claims 2 to 4, wherein the biological system comprises 15-LO, glutathione S transferase (for example GSTM1-1 or GSTP1-1) and/or leukotriene C4 synthase activity.

6. The method of any one of claims 1 to 5 wherein the compound is tested in a system comprising a cell fraction or isolated enzymes other than from human embryonic cells, a system other than a human being comprising a cell other than a human embryonic cell, or a system comprising a cell culture other than human embryonic cells.

7. The method of claim 6 wherein the cell is a L1236 cell or a primary eosinophilic leukocyte or a mast cell.

8. The method of claim 6 wherein the cell is stimulated or incubated with arachidonic acid.

9. The method of claim 6 wherein the compound is tested in a system other than a human being or human embryonic cells comprising a tissue or an organ, or wherein the compound is tested in a system comprising a non-human animal.

10. A method according to Claim 6, wherein the compound is tested for an inhibitory effect on Eoxin-induced permeabilisation of endothelial cells in an *in vitro* model of inflammation; or for an inhibitory effect on Eoxin-induced oxygen free radical production by leukocytes.

11. The method of any one of the preceding claims wherein the compound is further tested in a system other than a human being or human embryonic cells for an anti-inflammatory effect in an inflammatory disease or in a disease involving inflammatory processes.

12. The method of claim 11 wherein the inflammatory disease or disease involving inflammatory processes is chosen from asthma, chronic obstructive pulmonary disease (COPD), pulmonary fibrosis, allergic disorders, rhinitis, inflammatory bowel disease, ulcers, inflammatory pain, fever, atherosclerosis, coronary artery disease, vasculitis, pancreatitis, arthritis, osteoarthritis, rheumatoid arthritis, conjunctivitis, iritis, scleritis, uveitis, wound healing, dermatitis, eczema, psoriasis, stroke, diabetes, autoimmune diseases, Alzheimer's disease, multiple sclerosis, sarcoidosis, atherosclerosis, hypertension, Hodgkin's lymphoma and other malignancies.

13. The method of any one of claims 1 to 10 wherein the compound is further tested in a system other than a human being or human embryonic cells for a bone loss-reducing effect in
osteoporosis, osteoarthritis, multiple myeloma, Paget's disease or periodontal disease.

14. Use of a L1236 cell or cell culture, or other cell other than a human being or human embryonic cells expressing 15-lipoxygenase and at least one of Glutathione S transferase M1-1 (GSTM1-1), Glutathione S transferase P1-1 (GSTP1-1) and leukotriene C₄ synthase, in a method for identifying a compound with an inflammation-modulating effect or a bone loss-modulating effect, by identifying a compound for modulating the formation of EoxC₄. EoxD₄, or EoxE₄ in a biological system.

15. Eox C₄, Eox D₄ or Eox E₄ for use in treating a patient in need of promotion of an inflammatory response.

16. Eox C₄, Eox D₄ or Eox E₄ for use in medicine.

## Patentansprüche

1. Verfahren zur Identifizierung einer Verbindung zur Modulierung der Bildung von 14,15-LTC₄ (Eoxin C₄; EoxC₄), 14,15-LTD₄ (Eoxin D₄; EoxD₄) oder 14,15-LTE₄ (Eoxin E₄; EoxE₄) in einem biologischen System außer einem menschlichen Wesen oder humanen embryonalen Zellen, wobei das Verfahren das Testen der Verbindung auf eine Wirkung auf 15-Lipoxygenase (15-LO)-Aktivität oder Glutathion-S-Transferase M1-1 (GSTM1-1)-Aktivität, Glutathion-S-Transferase P1-1 (GSTP1-1)-Aktivität und/oder Leukotrien-C₄-Synthase-Aktivität umfasst.

2. Verfahren zur Identifizierung einer Verbindung mit einer entzündungsmodulierenden Wirkung oder einer Knochenabbau modulierenden Wirkung, wobei das Verfahren das Testen der Verbindung auf eine Wirkung auf die Bildung von EoxC₄, EoxD₄ oder EoxE₄ in einem biologischen System außer einem menschlichen Wesen oder humanen embryonalen Zellen und/oder
das Testen der Verbindung auf eine Wirkung auf die Aktivität von EoxC₄, EoxD₄ oder EoxE₄ in einem biologischen System außer einem menschlichen Wesen oder humanen embryonalen Zellen umfasst, wobei die Aktivität der Verbindung durch Testen auf eine Hemmwirkung auf die Eoxin-induzierte Permeabilisierung von Endothelzellen in einem in-vitro-Model einer Entzündung oder auf eine Hemmwirkung auf die Eoxin-induzierte Bildung von freien Radikalen von Sauerstoff durch Leukozyten getestet wird.

3. Verfahren nach Anspruch 2, wobei das Verfahren zur Identifizierung einer Verbindung mit einer entzündungshemmenden Wirkung oder Knochenabbau verringernden Wirkung dient und das Verfahren das Testen der Verbindung auf eine Hemmwirkung auf die Bildung und/oder Aktivität von EoxC₄, EoxD₄ oder EoxE₄ in einem biologischen System außer einem menschlichen Wesen oder humanen embryonalen Zellen umfasst.

4. Verfahren nach Anspruch 1, das ferner die Stufe des Testens der Verbindung auf eine Wirkung auf die Bildung von EoxC₄, EoxD₄ oder EoxE₄ in einem biologischen System außer einem menschlichen Wesen oder humanen embryonalen Zellen oder
des Testens der Verbindung auf eine Wirkung auf die Aktivität von EoxC₄, EoxD₄ oder EoxE₄ in einem biologischen System außer einem menschlichen Wesen oder humanen embryonalen Zellen umfasst, wobei die Aktivität der Verbindung durch Testen auf eine Hemmwirkung auf die Eoxin-induzierte Permeabilisierung von Endothelzellen in einem in-vitro-Model einer Entzündung oder auf eine Hemmwirkung auf die Eoxin-induzierte Bildung von freien Radikalen von Sauerstoff durch Leukozyten getestet wird.

5. Verfahren nach einem der Ansprüche 2 bis 4, wobei das biologische System 15-LO-, Glutathion-S-Transferase (beispielsweise GSTM1-1 oder GSTP1-1)- und/oder Leukotrien-C₄-Synthase-Aktivität umfasst.

6. Verfahren nach einem der Ansprüche 1 bis 5, wobei die Verbindung in einem System, das eine Zellfraktion oder isolierte Enzyme außer solchen von humanen embryonalen Zellen umfasst, einem System außer einem menschlichen Wesen, das eine Zelle außer humanen embryonalen Zelle umfasst, oder einem System, das eine Zellkultur außer humanen embryonalen Zellen umfasst, getestet wird.

7. Verfahren nach Anspruch 6, wobei die Zelle eine L1236-Zelle oder ein primärer eosinophiler Leukozyt oder eine Mastzelle ist.

8. Verfahren nach Anspruch 6, wobei die Zelle mit Arachidonsäure stimuliert oder inkubiert wird.

9. Verfahren nach Anspruch 6, wobei die Verbindung in einem System außer einem menschlichen Wesen oder humanen embryonalen Zellen, das ein Gewebe oder ein Organ umfasst, getestet wird oder wobei die Verbindung in einem System, das ein nichthumanes tierisches Lebewesen umfasst, getestet wird.

10. Verfahren nach Anspruch 6, wobei die Verbindung auf eine Hemmwirkung auf die Eoxin-induzierte Permeabilisierung von Endothelzellen in einem in-vitro-Model einer Entzündung oder auf eine Hemmwirkung auf die Eoxin-induzierte Bildung von freien Radikalen von Sauerstoff durch Leukozyten getestet wird.

11. Verfahren nach einem der vorhergehenden Ansprüche, wobei die Verbindung ferner in einem System außer einem menschlichen Wesen oder humanen embryonalen Zellen auf entzündungshemmende Wirkung, bei einer entzündlichen Erkrankung oder bei einer entzündliche Prozesse umfassenden Erkrankung getestet wird.

12. Verfahren nach Anspruch 11, wobei die entzündliche Erkrankung oder entzündliche Prozesse umfassende Erkrankung aus Asthma, chronisch-obstruktiver Lungenerkrankung (COPD), Lungenfibrose, Allergieerkrankungen, Rhinitis, entzündlicher Darmerkrankung, Ulzera, Entzündungsschmerzen, Fieber, Atherosklerose, koronarer Herzerkrankung, Vaskulitis, Pankreatitis, Arthritis, Osteoarthritis, rheumatoider Arthritis, Konjunktivitis, Iritis, Skleritis, Uveitis, Wundheilung, Dermatitis, Ekzemen, Psoriasis, Schlaganfall, Diabetes, Autoimmunerkrankungen, Alzheimer-Krankheit, multipler Sklerose, Sarkoidose, Atherosklerose, Hypertonie, Hodgkin-Lymphom und anderen bösartigen Erkrankungen ausgewählt ist.

13. Verfahren nach einem der Ansprüche 1 bis 10 wobei die Verbindung ferner in einem System außer einem menschlichen Wesen oder humanen embryonalen Zellen auf eine Knochenabbau verringernde Wirkung bei Osteoporose, Osteoarthritis, multiplem Myelom, Morbus Paget oder einer Periodontiumerkrankung getestet wird.

14. Verwendung einer L1236-Zelle oder Zellkultur oder einer anderen Zelle als einem menschlichen Wesen oder humanen embryonalen Zellen, die 15-Lipoxygenase und mindestens eine Komponente von Glutathion-S-Transferase M1-1 (GSTM1-1), Glutathion-S-Transferase P1-1 (GSTP1-1) und Leukotrien-C₄-Synthase exprimieren, in einem Verfahren zur Identifizierung einer Verbindung mit einer entzündungsmodulierenden Wirkung oder Knochenabbau modulierenden Wirkung durch Identifizieren einer Verbindung zur Modulierung der Bildung von EoxC₄, EoxD₄ oder EoxE₄ in einem biologischen System.

15. Eox C₄, Eox D₄ oder Eox E₄ zur Verwendung bei der Behandlung eines Patienten, der eine Förderung einer entzündlichen Reaktion benötigt.

16. Eox C₄, Eox D₄ oder Eox E₄ zur Verwendung in der Medizin.

## Revendications

1. Procédé pour identifier un composé pour moduler la formation de 14,15-LTC₄ (Eoxine C₄ ; EoxC₄), de 14,15-LTD₄ (Eoxine D₄ ; EoxD₄) ou de 14,15-LTE₄ (Eoxine E₄ ; EoxE₄) dans un système biologique autre qu'un être humain ou des cellules embryonnaires humaines, le procédé comprenant le test du composé quant à un effet sur l'activité de la 15 lipoxygénase (15-LO) ou l'activité de la glutathion S transférase M1-1 (GSTM1-1), de la glutathion S transférase P1-1 (GSTP1-1) et/ou de la leucotriène C₄ synthase.

2. Procédé pour identifier un composé ayant un effet modulateur de l'inflammation ou un effet modulateur de la perte osseuse, le procédé comprenant le test du composé quant à un effet sur la formation d'EoxC₄, d'EoxD₄ ou d'EoxE₄ dans un système biologique autre qu'un être humain ou des cellules embryonnaires humaines, et/ou
le test du composé quant à un effet sur l'activité d'EoxC₄, d'EoxD₄ ou d'EoxE₄ dans un système biologique autre qu'un être humain ou des cellules embryonnaires humaines, dans lequel l'activité du composé est testée en recherchant un effet inhibiteur sur la perméabilisation de cellules endothéliales induite par une éoxine dans un modèle d'inflammation *in vitro* ; ou un effet inhibiteur sur la production, par des leucocytes, de radicaux libres oxygénés induite par une éoxine.

3. Procédé selon la revendication 2, lequel procédé est destiné à identifier un composé ayant un effet anti-inflammatoire ou un effet réducteur de la perte osseuse et le procédé comprend le test du composé quant à un effet inhibiteur sur la formation et/ou l'activité d'EoxC₄, d'EoxD₄ ou d'EoxE₄ dans un système biologique autre qu'un être humain ou des cellules embryonnaires humaines.

4. Procédé selon la revendication 1, comprenant en outre l'étape consistant à tester le composé quant à un effet sur la formation d'EoxC₄, d'EoxD₄ ou d'EoxE₄ dans un système biologique autre qu'un être humain ou des cellules embryonnaires humaines, ou
à tester le composé quant à un effet sur l'activité d'EoxC₄, d'EoxD₄ ou d'EoxE₄ dans un système biologique autre qu'un être humain ou des cellules embryonnaires humaines, dans lequel l'activité du composé est testée en recherchant un effet inhibiteur sur la perméabilisation de cellules endothéliales induite par une éoxine dans un modèle d'inflammation *in vitro* ; ou un effet inhibiteur sur la production, par des leucocytes, de radicaux libres oxygénés induite par une éoxine.

5. Procédé selon l'une quelconque des revendications 2 à 4, dans lequel le système biologique comprend une activité de la 15-LO, glutathion S d'une transférase (par exemple GSTM1-1 ou GSTP1-1) et/ou de la leucotriène C4 synthase.

6. Procédé selon l'une quelconque des revendications 1 à 5, dans lequel le composé est testé dans un système comprenant une fraction cellulaire ou des enzymes isolées autre(s) que des cellules embryonnaires humaines, un système autre qu'un être humain comprenant une cellule autre qu'une cellule embryonnaire humaine ou un système comprenant une culture cellulaire autre que des cellules embryonnaires humaines.

7. Procédé selon la revendication 6, dans lequel la cellule est une cellule L1236 ou un leucocyte éosinophile primaire ou un mastocyte.

8. Procédé selon la revendication 6, dans lequel la cellule est stimulée ou incubée avec de l'acide arachidonique.

9. Procédé selon la revendication 6, dans lequel le composé est testé dans un système autre qu'un être humain ou des cellules embryonnaires humaines comprenant un tissu ou un organe, ou dans lequel le composé est testé dans un système comprenant un animal non humain.

10. Procédé selon la revendication 6, dans lequel le composé est testé quant à un effet inhibiteur sur la perméabilisation de cellules endothéliales induite par une éoxine dans un modèle d'inflammation *in vitro* ; ou un effet inhibiteur sur la production, par des leucocytes, de radicaux libres oxygénés induite par une éoxine.

11. Procédé selon l'une quelconque des revendications précédentes, dans lequel le composé est en outre testé dans un système autre qu'un être humain ou des cellules embryonnaires humaines quant à un effet anti-inflammatoire dans une maladie inflammatoire ou dans une maladie faisant intervenir des processus inflammatoires.

12. Procédé selon la revendication 11, dans lequel la maladie inflammatoire ou la maladie faisant intervenir des processus inflammatoires est choisie parmi un asthme, une bronchopneumopathie obstructive chronique (BPCO), une fibrose pulmonaire, des troubles allergiques, une rhinite, une maladie intestinale inflammatoire, des ulcères, une douleur inflammatoire, une fièvre, une athérosclérose, une coronaropathie, une vasculite, une pancréatite, une arthrite, une arthrose, une polyarthrite rhumatoïde, une conjonctivite, une iritis, une sclérite, une uvéite, une cicatrisation de blessure, une dermatite, un eczéma, un psoriasis, une attaque, un diabète, des maladies auto-immunes, une maladie d'Alzheimer, une sclérose en plaques, une sarcoïdose, une athérosclérose, une hypertension, un lymphome de Hodgkin et d'autres malignités.

13. Procédé selon l'une quelconque des revendications 1 à 10, dans lequel le composé est en outre testé dans un système autre qu'un être humain ou des cellules embryonnaires humaines quant à un effet réducteur de la perte osseuse dans une ostéoporose, une arthrose, un myélome multiple, une maladie de Paget ou une maladie parodontale.

14. Utilisation d'une cellule ou culture cellulaire L1236, ou d'une autre cellule autre qu'un être humain ou des cellules embryonnaires humaines exprimant de la 15-lipoxygénase et au moins l'une parmi la glutathion S transférase M1-1 (GSTM1-1), la glutathion S transférase P1-1 (GSTP1-1) et la leucotriène C₄ synthase, dans un procédé pour identifier un composé ayant un effet modulateur d'inflammation ou un effet modulateur de la perte osseuse, en identifiant un composé pour moduler la formation d'EoxC₄, d'EoxD₄ ou d'EoxE₄ dans un système biologique.

15. EoxC₄, EoxD₄ ou EoxE₄ pour une utilisation dans le traitement d'un patient ayant besoin d'une activation d'une réponse inflammatoire.

16. EoxC₄, EoxD₄ ou EoxE₄ pour une utilisation en médecine.
